# EUROPEAN PATENT SPECIFICATION

(11) **EP 3 386 973 B1**
(45) Date of publication and mention of the grant of the patent: **12.08.2020**
(21) Application number: 16810320.8
(22) Date of filing: 08.12.2016
(51) Int. Cl.: C07D 401/14, C07D 413/14, C07D 401/06, C07D 403/06, C07D 413/06, C07D 417/06, C07D 491/10, C07D 498/04, A01N 43/653

(54) **PESTICIDALLY ACTIVE 1,2,4-TRIAZOLE DERIVATIVES**
PESTIZIDWIRKSAME 1,2,4-TRIAZOLDERIVATE
DÉRIVÉS DE 1,2,4-TRIAZOLE ACTIF SUR LE PLAN PESTICIDE

(30) Priority: 11.12.2015 EP 15199652
(43) Date of publication of application: 17.10.2018
(73) Proprietor: Syngenta Participations AG, 4058 Basel (CH)
(72) Inventor: EDMUNDS, Andrew, 4332 Stein (CH); SCHAETZER, Jürgen, Harry, 4332 Stein (CH); BIGOT, Aurelien, 4332 Stein (CH); RENDLER, Sebastian, 4332 Stein (CH); JEANGUENAT, André, 4332 Stein (CH)
(74) Representative: SYNGENTA IP
(86) International application number: PCT/EP2016/080312
(87) International publication number: WO 2017/097927

(56) References cited:
- WO-A1-2008/063912
- WO-A1-2013/173218
- WO-A2-2008/017932
- PAULVANNAN K ET AL: "An Improved Synthesis of 1,2,4-Triazoles using Ag2CO3", TETRAHEDRON, ELSEVIER SCIENCE PUBLISHERS, AMSTERDAM, NL, vol. 56, no. 41, 6 October 2000 (2000-10-06), pages 8071-8076, XP004217678, ISSN: 0040-4020, DOI: 10.1016/S0040-4020(00)00726-2

## Description

The present invention relates to 1,2,4-triazole derivatives, to processes for preparing them, to intermediates for preparing them, to pesticidal, in particular insecticidal, acaricidal, molluscicidal and nematicidal compositions comprising those derivatives and to methods of using them to combat and control pests such as insect, acarine, mollusc and nematode pests.

It has now surprisingly been found that certain 1,2,4-triazole derivatives have highly potent insecticidal properties. Other compounds in this area are known from WO2013/063282, WO2013/173218, WO2015/077436, WO2015/091645 and WO2015/022293.

The present invention relates to a compound of formula (I) 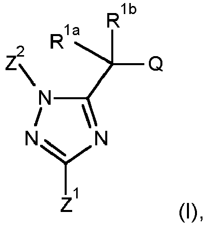 wherein
Q is 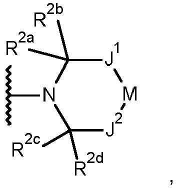 wherein
M is
(ii) -C(R³⁹)(A)-;
J¹ is -C(R^{3a}R^{3b})-;
J² is -C(R^{3e}R³)-;
R^{2a}, R^{2b}, R^{2c} and R^{2d} are H;
R^{3a}, R^{3b} R^{3e} and R^{3f} are H;
R^{3g} is selected from H and C₁-C₆-alkyl;
A is selected from H, halogen, cyano, C₁-C₆-alkyl, C₁-C₆-haloalkyl, -C(=O)OR⁶, -C(=O)NR⁷R⁸,-NR⁷C(=O)R⁸, -NR⁹R¹⁰, OR¹² and -S(=O)₂R¹¹; or -(C₀-C₃-alkyl)-phenyl, a -(C₀-C₃-alkyl)-5-6 membered heteroaromatic ring or a -(C₀-C₃-alkyl)-7-11 membered bicyclic ring system, each ring or ring system is unsubstituted or substituted with 1 to 3 R⁴⁹;
R⁴⁹ are independently selected from halogen, cyano, nitro, -C(=O)R⁵, -C(=S)R⁵, -C(=O)OR⁶,-C(=O)NR⁷R⁸, -NR⁷C(=O)R⁸, -C(=S)NR⁷R⁸, -NR⁹R¹⁰, -S(=O)₂NR¹R¹⁰, -SR¹¹, -S(=O)R¹¹, -S(=O)₂R¹¹, OR^{12a}, C₁-C₆-alkyl, C₂₋C₆-alkenyl, C₂₋C₆-alkynyl, C₃₋C₇-cycloalkyl, C₃₋C₇-cycloalkyl- C₁₋C₆-alkyl and C₃₋C₇-cycloalkenyl, which C₁-C₆-alkyl, C₂₋C₆-alkenyl, C₂-C₆-alkynyl, C₃₋C₇-cycloalkyl, C₃-C₇-cycloalkyl-C₁₋C₆-alkyl and C₃₋C₇-cycloalkenyl are unsubstituted or substituted with 1-4 substituents independently selected from halogen, cyano, nitro, C₁-C₆-alkyl, C₁-C₆-haloalkyl, -SR^{11a}, -S(=O)R^{11a} and -S(=O)₂R^{11a}; R⁵ is selected from H, C₁-C₆-alkyl, C₂₋C₆-alkenyl, C₃₋C₆-alkynyl, C₃₋C₇-cycloalkyl, C₃₋C₇-cycloalkyl-C₁₋C₆-alkyl and C₃₋C₇-cycloalkenyl, which C₁-C₆-alkyl, C₂₋C₆-alkenyl, C₃₋C₆-alkynyl, C₃₋C₇-cycloalkyl, C₃₋C₇₋cycloalkyl- C₁₋C₆-alkyl and C₃₋C₇-cycloalkenyl are unsubstituted or substituted with 1-4 substituents independently selected from halogen, cyano, nitro, OR^{12a}, C₁-C₆-alkyl, C₁-C₆-haloalkyl, -SR^{11a}, - S(=O)R^{11a} and -S(=O)zR^{11a};
R⁶ is selected from C₁-C₆-alkyl, C₂₋C₆-alkenyl, C₃₋C₆-alkynyl, C₃₋C₇-cycloalkyl, C₃₋C₇-cycloalkyl-C₁₋C₆-alkyl and C₃₋C₇-cycloalkenyl, which C₁-C₆-alkyl, C₂₋C₆-alkenyl, C₃₋C₆-alkynyl, C₃₋C₇-cycloalkyl, C₃₋C₇-cycloalkyl- C₁₋C₆-alkyl and C₃₋C₇-cycloalkenyl are unsubstituted or substituted with 1-4 substituents independently selected from halogen, cyano, nitro, OR^{12a}, C₁-C₆-alkyl, C₁-C₆-haloalkyl, -SR^{11a},-S(=O)R^{11a} and -S(=O)₂R^{11a};
R⁷ and R⁸ are independently selected from H, C₁-C₆-alkyl, C₂₋C₆-alkenyl, C₃-C₆-alkynyl, C₃₋C₇-cycloalkyl, C₃₋C₇₋cycloalkyl-C₁₋C₆-alkyl and C₃₋C₇-cycloalkenyl, which C₁-C₆-alkyl, C₂₋C₆-alkenyl, C₃₋C₆-alkynyl, C₃₋C₇-cycloalkyl, C₃₋C₇₋cycloalkyl-C₁₋C₆-alkyl and C₃₋C₇-cycloalkenyl are unsubstituted or substituted with 1-4 substituents independently selected from halogen, cyano, nitro, OR^{12a}, C₁-C₆-alkyl, C₁₋C₆₋haloalkyl, -SR^{11a}, -S(=O)R^{11a} and -S(=O)₂R^{11a};
R⁹ and R¹⁰ are independently selected from H, -C(=O)R⁵, -C(=S)R⁵, -C(=O)OR⁶, -C(=O)NR⁷R⁸,-NR⁷C(=O)R⁸, C₁-C₆-alkyl, C₂₋C₆-alkenyl, C₃₋C₆-alkynyl, C₃₋C₇-cycloalkyl, C₃₋C₇₋cycloalkyl-C₁₋C₆-alkyl and C₃₋C₇₋cycloalkenyl; which C₁-C₆-alkyl, C₂₋C₆-alkenyl, C₃₋C₆-alkynyl, C₃₋C₇-cycloalkyl, C₃₋C₇-cycloalkyl-C₁₋C₆-alkyl and C₃₋C₇-cycloalkenyl are unsubstituted or substituted with 1-4 substituents independently selected from halogen, cyano, nitro, OR^{12a}, C₁-C₆-alkyl, C₁-C₆-haloalkyl, -SR^{11a},-S(=O)R^{11a} and -S(=O)₂R^{11a};
R¹¹ is selected from C₁-C₆-alkyl, C₂₋C₆-alkenyl, C₃₋C₆-alkynyl, C₃₋C₇-cycloalkyl, C₃₋C₇₋cycloalkyl-C₁₋C₆-alkyl and C₃₋C₇-cycloalkenyl, which C₁-C₆-alkyl, C₂₋C₆-alkenyl, C₃₋C₆-alkynyl, C₃₋C₇-cycloalkyl, C₃₋C₇-cycloalkyl- C₁₋C₆-alkyl and C₃₋C₇-cycloalkenyl are unsubstituted or substituted with 1-4 substituents independently selected from halogen, cyano, nitro, OR^{12a}, C₁-C₆-alkyl, C₁-C₆-haloalkyl, -SR^{11a},-S(=O)R^{11a} and -S(=O)zR^{11a};
R^{11a}, R^{12a} and R¹⁴ are independently selected from H, C₁₋C₆-alkyl and C₁₋C₆-haloalkyl;
R¹² is selected from H, C₁-C₆-alkyl, C₂₋C₆-alkenyl, C₃₋C₆-alkynyl, C₃₋C₇-cycloalkyl, C₃₋C₇-cycloalkyl-C₁₋C₆-alkyl and C₃₋C₇-cycloalkenyl, which C₁-C₆-alkyl, C₂₋C₆-alkenyl, C₃₋C₆-alkynyl, C₃₋C₇-cycloalkyl, C₃₋C₇₋cycloalkyl-C₁₋C₆-alkyl and C₃₋C₇-cycloalkenyl are unsubstituted or substituted with 1-4 substituents independently selected from halogen, cyano, nitro, OR^{12a}, C₁-C₆-alkyl, C₁-C₆-haloalkyl, -SR^{11a},-S(=O)R^{11a} and -S(=O)zR^{11a};
   or an agrochemically acceptable salt or N-oxide thereof.

In an embodiment 1 of the invention, the invention provides
a compound of formula (Ia) 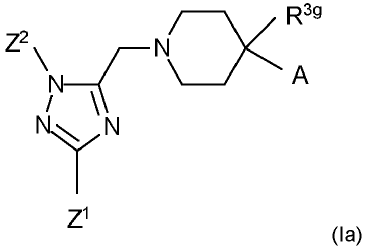 wherein Z¹, Z², A and R³⁹ are as defined in the following table:

| Example No. | Z¹ | Z² | A | R^{3g} |
|---|---|---|---|---|
| 1.1 | 4-chlorophenyl | 2-fluorophenyl | pyrimidin-2-yl | H |
| 1.2 | 5-chloropyridin-2-yl | 2-fluorophenyl | CF₃ | H |
| 1.3 | 5-chloropyridin-2-yl | 2-fluorophenyl | 1,3,4-oxadiazol-2-yl | H |
| 1.4 | 5-chloropyridin-2-yl | 2-fluorophenyl | pyrimidin-2-yl | H |
| 1.5 | 4-chlorophenyl | 2-fluorophenyl | CF₃ | H |
| 1.6 | 4-chlorophenyl | 2-fluorophenyl | 1,3,4-oxadiazol-2-yl | H |
| 1.7 | 5-chloropyridin-2-yl | 2-fluorophenyl | CN | H |
| 1.8 | 4-chlorophenyl | 2-fluorophenyl | CN | H |
| 1.9 | 4-chlorophenyl | 2-fluorophenyl | CO₂CH₃ | H |
| 1.10 | 4-chlorophenyl | 2-fluorophenyl | CH=N-OCH₃ | H |
| 1.11 | 4-chlorophenyl | 2-fluorophenyl | 3yridine-2-yl | H |
| 1.12 | 4-chlorophenyl | 2-fluorophenyl | piperazin-1-yl | H |
| 1.13 | 4-chlorophenyl | 2-fluorophenyl | 5-chloro-pyrimidin-2-yl | H |
| 1.14 | 4-chlorophenyl | 2-fluorophenyl | triazol-2-yl | H |
| 1.15 | 4-chlorophenyl | 2-fluorophenyl | triazol-1-yl | H |
| 1.16 | 4-chlorophenyl | 2,6-difluorophenyl | pyrimidin-2-yl | H |
| 1.17 | 4-chlorophenyl | 2-fluorophenyl | 3-(4-fluorophenyl)-1,2,4-oxadiazol-5-yl | H |
| 1.18 | 4-chlorophenyl | 4-fluorophenyl | pyrimidin-2-yl | H |
| 1.19 | 4-chlorophenyl | 2-fluorophenyl | 4-bromothiazol-2-yl | H |
| 1.20 | 3,4-dichlorophenyl | 2-fluorophenyl | pyrimidin-2-yl | H |
| 1.21 | 4-(trifluoromethyl)-phenyl | 2,6-difluorophenyl | pyrimidin-2-yl | H |
| 1.22 | 4-chlorophenyl | 2-fluorophenyl | tetrazol-1-yl | H |
| 1.23 | 4-chlorophenyl | 2,6-dichlorophenyl | pyrimidin-2-yl | H |
| 1.24 | 4-chlorophenyl | 2-fluorophenyl | 5-chloro-1 H-benzimidazol-2-yl | H |
| 1.25 | 4-chlorophenyl | 2-fluorophenyl | tetrazol-2-yl | H |
| 1.26 | 4-(trifluoromethyl)-phenyl | 2-fluorophenyl | pyrimidin-2-yl | H |
| 1.27 | 4-chlorophenyl | 2-fluorophenyl | 5-(trifluoromethyl)-pyrimidin-2-yl | H |
| 1.28 | 4-chlorophenyl | 3,5-(bis-trifluoromethyl)phenyl | pyrimidin-2-yl | H |
| 1.29 | 4-fluorophenyl | 4-fluorophenyl | pyrimidin-2-yl | H |
| 1.30 | 4-chlorophenyl | 2,4-difluorophenyl | pyrimidin-2-yl | H |
| 1.31 | 4-chlorophenyl | 2-fluorophenyl | CH₃ | H |
| 1.32 | 4-chlorophenyl | 2-fluorophenyl | 4-(trifluoromethyl)-pyrimidin-2-yl | H |
| 1.33 | 3-chloro-4-fluorophenyl | 2-fluorophenyl | pyrimidin-2-yl | H |
| 1.34 | 4-(trifluoromethyl)-phenyl | 4-(trifluoromethyl)-phenyl | pyrimidin-2-yl | H |
| 1.35 | 4-chlorophenyl | 2-fluorophenyl | CH₃ | CH₃ |
| 1.36 | 2-methylphenyl | 2-fluorophenyl | pyrimidin-2-yl | H |
| 1.37 | 3-methylphenyl | 2-fluorophenyl | pyrimidin-2-yl | H |
| 1.38 | 3-(trifluoromethyl)-phenyl | 2-fluorophenyl | pyrimidin-2-yl | H |
| 1.39 | 4-chlorophenyl | 2-fluorophenyl I | 4-(trifluoromethyl)-pyrazol-1-yl | H |
| 1.40 | 4-chlorophenyl | 2-fluorophenyl | 5-(2-thienyl)-1 H-pyrazol-3-yl | H |
| 1.41 | 4-chlorophenyl | 2-fluorophenyl | 5-(4-chlorophenyl)-1H-pyrazol-3-yl | H |
| 1.42 | 4-chlorophenyl | 2-fluorophenyl | (4-chlorophenyl)methyl | H |
| 1.43 | 4-chlorophenyl | 2-fluorophenyl | phenyl | H |
| 1.44 | 4-chlorophenyl | 2-fluorophenyl | CO₂-tert-C₄H₉ | H |
| 1.45 | 4-chlorophenyl | 2-fluorophenyl | (phenyl)methyl | H |
| 1.46 | 4-chlorophenyl | 2-fluorophenyl | CH₃ | H |
| 1.47 | 4-chlorophenyl | 2-fluorophenyl | CONH₂ | H |
| 1.48 | 4-chlorophenyl | 2-fluorophenyl | 4-(fluorophenyl)-methanone | H |
| 1.49 | 4-chlorophenyl | 2,3,5,6-tetrafluoro-phenyl | pyrimidin-2-yl | H |
| 1.50 | 4-chlorophenyl | 3,4-dichlorophenyl | pyrimidin-2-yl | H |
| 1.51 | 4-chlorophenyl | 3-methylphenyl | pyrimidin-2-yl | H |
| 1.52 | 4-chlorophenyl | 4-methylphenyl | pyrimidin-2-yl | H |
| 1.53 | 4-chlorophenyl | 3,5-dichlorophenyl | pyrimidin-2-yl | H |
| 1.54 | 4-chlorophenyl | 2,4-difluorophenyl | pyrimidin-2-yl | H |
| 1.55 | 4-chlorophenyl | 2,3-dichlorophenyl | pyrimidin-2-yl | H |
| 1.56 | 4-chlorophenyl | (phenyl)methyl | pyrimidin-2-yl | H |
| 1.57 | 4-chlorophenyl | 3-bromophenyl | pyrimidin-2-yl | H |
| 1.58 | 4-chlorophenyl | cyclobutyl | pyrimidin-2-yl | H |
| 1.59 | 4-chlorophenyl | 6-chloropyridine-6-yl | pyrimidin-2-yl | H |
| 1.60 | 4-chlorophenyl | isopropyl | pyrimidin-2-yl | H |
| 1.61 | 4-chlorophenyl | phenyl | pyrimidin-2-yl | H |
| 1.62 | 4-chlorophenyl | 2-methylphenyl | pyrimidin-2-yl | H |
| 1.63 | 4-chlorophenyl | 4-(trifluoromethoxy)-phenyl | pyrimidin-2-yl | H |
| 1.64 | 4-chlorophenyl | 3-chloro-4-methylphenyl | pyrimidin-2-yl | H |
| 1.65 | 4-chlorophenyl | 2,4-dichlorophenyl | pyrimidin-2-yl | H |
| 1.66 | 4-chlorophenyl | 4-chlorophenyl | pyrimidin-2-yl | H |
| 1.67 | 4-chlorophenyl | 2-chloro-4-(trifluoromethyl)-phenyl | pyrimidin-2-yl | H |
| 1.68 | 4-chlorophenyl | 3-chloro-5-(trifluoromethyl)-pyridin-2-yl | pyrimidin-2-yl | H |
| 1.69 | 4-chlorophenyl | 3-chlorophenyl | pyrimidin-2-yl | H |
| 1.70 | 4-chlorophenyl | 2-methoxyphenyl | pyrimidin-2-yl | H |
| 1.71 | 4-chlorophenyl | 4-sulfonamido phenyl | pyrimidin-2-yl | H |
| 1.72 | 4-chlorophenyl | 6-chloropyridine-2-yl | pyrimidin-2-yl | H |
| 1.73 | 4-chlorophenyl | 3,4-dimethylphenyl | pyrimidin-2-yl | H |
| 1.74 | 4-chlorophenyl | 2,5-dimethylphenyl | pyrimidin-2-yl | H |
| 1.75 | 4-chlorophenyl | 3-methoxyphenyl | pyrimidin-2-yl | H |
| 1.76 | 4-chlorophenyl | 4-bromophenyl | pyrimidin-2-yl | H |
| 1.77 | 4-chlorophenyl | 2,4,6-trichlorophenyl | pyrimidin-2-yl | H |
| 1.78 | 4-chlorophenyl | 5yridine-2-yl | pyrimidin-2-yl | H |
| 1.79 | 4-chlorophenyl | 4-methoxyphenyl | pyrimidin-2-yl | H |
| 1.80 | 4-chlorophenyl | 3-fluorophenyl | pyrimidin-2-yl | H |
| 1.81 | 4-chlorophenyl | 3-(trifluoromethyl)-phenyl | pyrimidin-2-yl | H |
| 1.82 | 4-chlorophenyl | 2,3-dimethylphenyl | pyrimidin-2-yl | H |
| 1.83 | 4-chlorophenyl | 2-chlorophenyl | pyrimidin-2-yl | H |
| 1.84 | 4-chlorophenyl | 3-chloro-2-methylphenyl | pyrimidin-2-yl | H |
| 1.85 | 4-chlorophenyl | 4-chloro-2-methylphenyl | pyrimidin-2-yl | H |
| 1.86 | 4-chlorophenyl | 2,5-dichlorophenyl | pyrimidin-2-yl | H |
| 1.87 | 4-chlorophenyl | 2,4-dimethylphenyl | pyrimidin-2-yl | H |
| 1.88 | 4-chlorophenyl | 4-(trifluoromethyl)-phenyl | pyrimidin-2-yl | H |
| 1.89 | 4-chlorophenyl | 3-chloro-5yridine-2-yl | pyrimidin-2-yl | H |
| 1.90 | 4-chlorophenyl | 3,5-dichloro-5yridine-2-yl | pyrimidin-2-yl | H |
| 1.91 | (4-chloro-phenyl)methyl | 2-fluorophenyl | pyrimidin-2-yl | H |
| 1.92 | 3-fluoro-4-methylphenyl | 2-fluorophenyl | pyrimidin-2-yl | H |
| 1.93 | cyclopropyl | 2-fluorophenyl | pyrimidin-2-yl | H |
| 1.94 | phenyl | 2-fluorophenyl | pyrimidin-2-yl | H |
| 1.95 | 4-methylphenyl | 2-fluorophenyl | pyrimidin-2-yl | H |
| 1.96 | thiophen-2-yl | 2-fluorophenyl | pyrimidin-2-yl | H |
| 1.97 | 5-(trifluoromethyl)-pyridin-2-yl | 2-fluorophenyl | pyrimidin-2-yl | H |
| 1.98 | 6yridine-3-yl | 2-fluorophenyl | pyrimidin-2-yl | H |
| 1.99 | ethyl | 2-fluorophenyl | pyrimidin-2-yl | H |
| 1.100 | isopropyl | 2-fluorophenyl | pyrimidin-2-yl | H |
| 1.101 | 4-methoxyphenyl | 2-fluorophenyl | pyrimidin-2-yl | H |
| 1.102 | 6yridine-2-yl | 2-fluorophenyl | pyrimidin-2-yl | H |
| 1.103 | 2-methyl-propyl | 2-fluorophenyl | pyrimidin-2-yl | H |
| 1.104 | cyclohexyl | 2-fluorophenyl | pyrimidin-2-yl | H |
| 1.105 | 2-fluorophenyl | 2-fluorophenyl | pyrimidin-2-yl | H |
| 1.106 | 2-ethyl-propyl | 2-fluorophenyl | pyrimidin-2-yl | H |
| 1.107 | 6-methyl-pyridin-2-yl | 2-fluorophenyl | pyrimidin-2-yl | H |
| 1.108 | 4-chlorophenyl | 2-fluorophenyl | 4-carbonyl-piperidin-4-one | H |
| 1.109 | 4-chlorophenyl | 2-fluorophenyl | 4-(trifluoromethyl)-1 H-imdazol-2-yl | H |
| 1.110 | 4-chlorophenyl | 2-fluorophenyl | 2-methyl-pyrimidin-4-yl | H |
| 1.111 | 4-chlorophenyl | 2-fluorophenyl | N-methyl-N-methoxy-carboxamide | H |
| 1.112 | 4-chlorophenyl | 2-fluorophenyl | 1 H-imidazol-2-yl | H |
| 1.113 | 4-chlorophenyl | 2-fluorophenyl | 1,2,4-oxadiazol-3-yl | H |
| 1.114 | 4-chlorophenyl | 2-fluorophenyl | N-cyclopropyl-carboxamide | H |
| 1.115 | 4-chlorophenyl | 2-fluorophenyl | thiazol-2-yl | H |
| 1.116 | 4-chlorophenyl | 2-fluorophenyl | pyrimidin-4-yl | H |
| 1.117 | 4-chlorophenyl | 2-fluorophenyl | 1-methylimidazol-2-yl | H |
| 1.118 | 4-chlorophenyl | 2-fluorophenyl | 2-methyl-1,3,4-oxadiazol-5-yl | H |
| 1.119 | 4-chlorophenyl | 2-fluorophenyl | thiazol-4-yl | H |
| 1.120 | 4-chlorophenyl | 2-fluorophenyl | isoxazol-3-yl | H |
| 1.121 | 4-chlorophenyl | 2-fluorophenyl | 1,2,4-oxadiazol-5-yl | H |
| 1.122 | 4-chlorophenyl | 2-fluorophenyl | 5-fluoro-pyridin-2-yl | H |
| 1.123 | 4-chlorophenyl | 2-fluorophenyl | (4-pyridyl)-1,3,4-oxadiazol-2-yl | H |
| 1.124 | 4-chlorophenyl | 2-fluorophenyl | isoxazol-2-yl | H |
| 1.125 | 4-chlorophenyl | 2-fluorophenyl | 4-methylpyrimidin-2-yl | H |
| 1.126 | 4-chlorophenyl | 2-fluorophenyl | 3-chloro-pyridin-2-yl | H |
| 1.127 | 4-chloro-3-fluorophenyl | 2-fluorophenyl | pyrimidin-2-yl | H |

or an agrochemically acceptable salt or N-oxide thereof.

The term "compound(s) of the invention" or a "compound of the invention" as used herein means a compound as defined in embodiment 1.

### Definitions:

The term "Alkyl" as used herein- in isolation or as part of a chemical group - represents straight-chain or branched hydrocarbons, preferably with 1 to 6 carbon atoms, for example methyl, ethyl, n-propyl, isopropyl, n-butyl, isobutyl, s-butyl, t-butyl, pentyl, 1- methylbutyl, 2-methylbutyl, 3-methylbutyl, 1,2-dimethylpropyl, 1,1 -dimethylpropyl, 2,2- dimethylpropyl, 1 -ethylpropyl, hexyl, 1 -methylpentyl, 2-methylpentyl, 3-methylpentyl, 4- methylpentyl, 1,2-dimethylpropyl, 1,3-dimethylbutyl, 1,4-dimethylbutyl, 2,3-dimethylbutyl, 1,1- dimethylbutyl, 2,2-dimethylbutyl, 3,3-dimethylbutyl, 1,1,2-trimethylpropyl, 1,2,2-trimethylpropyl, 1- ethylbutyl and 2-ethylbutyl. Alkyl groups with 1 to 4 carbon atoms are preferred, for example methyl, ethyl, n-propyl, isopropyl, n-butyl, isobutyl, s-butyl or t-butyl.

The term "Alkenyl" - in isolation or as part of a chemical group - represents straight-chain or branched hydrocarbons, preferably with 2 to 6 carbon atoms and at least one double bond, for example vinyl, 2-propenyl, 2-butenyl, 3-butenyl, 1- methyl-2-propenyl, 2-methyl-2-propenyl, 2-pentenyl, 3-pentenyl, 4-pentenyl, 1-methyl-2-butenyl, 2- methyl-2-butenyl, 3-methyl-2-butenyl, 1-methyl-3-butenyl, 2-methyl-3-butenyl, 3-methyl-3-butenyl, 1,1 - dimethyl-2-propenyl, 1,2-dimethyl-2-propenyl, 1 -ethyl-2-propenyl, 2-hexenyl, 3-hexenyl, 4- hexenyl, 5-hexenyl, 1 -methyl-2-pentenyl, 2-methyl-2-pentenyl, 3-methyl-2-pentenyl, 4-methyl-2- pentenyl, 3-methyl-3-pentenyl, 4-methyl-3-pentenyl, 1 -methyl-4-pentenyl, 2-methyl-4-pentenyl, 3- methyl-4-pentenyl, 4-methyl-4-pentenyl, 1, 1 -dimethyl-2-butenyl, 1,1-dimethyl-3-butenyl, 1,2- dimethyl-2-butenyl, 1,2-dimethyl-3-butenyl, 1,3-dimethyl-2-butenyl, 2,2-dimethyl-3-butenyl, 2,3- dimethyl-2-butenyl, 2,3-dimethyl-3-butenyl, 1 -ethyl-2-butenyl, 1-ethyl-3-butenyl, 2-ethyl-2-butenyl, 2-ethyl-3-butenyl, 1, 1,2-trimethyl-2-propenyl, 1 -ethyl- 1 -methyl-2-propenyl und 1-ethyl-2-methyl-2-propenyl. Alkenyl groups with 2 to 4 carbon atoms are preferred, for example 2-propenyl, 2-butenyl or 1-methyl-2-propenyl.

The term "Alkynyl" - in isolation or as part of a chemical group - represents straight-chain or branched hydrocarbons, preferably with 2 bis 6 carbon atoms and at least one triple bond, for example 2-propinyl, 2-butinyl, 3-butinyl, 1-methyl-2- propinyl, 2-pentinyl, 3-pentinyl, 4-pentinyl, 1-methyl-3-butinyl, 2-methyl-3-butinyl, 1-methyl-2- butinyl, 1,1 -dimethyl-2-propinyl, 1 -ethyl-2-propinyl, 2-hexinyl, 3-hexinyl, 4-hexinyl, 5-hexinyl, 1- methyl-2-pentinyl, 1-methyl-3-pentinyl, 1 -methyl-4-pentinyl, 2-methyl-3-pentinyl, 2-methyl-4- pentinyl, 3 -methyl-4-pentinyl, 4-methyl-2-pentinyl, 1,1 -dimethyl-3 -butinyl, 1,2-dimethyl-3 -butinyl, 2,2- dimethyl-3-butinyl, 1-ethyl-3-butinyl, 2-ethyl-3-butinyl, 1-ethyl-1-methyl, 1,2-propinyl and 2,5-hexadiynyl. Alkinyls with 2 to 4 carbon atoms are preferred, for example ethinyl, 2-propinyl or 2-butinyl-2-propenyl.

The term "cycloalkyl" - in isolation or as part of a chemical group - represents saturated or partially unsaturated mono-, bi- or tricyclic hydrocarbons, preferably 3 to 10 carbon atoms, for example cyclopropyl, cyclobutyl, cyclopentyl, cyclohexyl, cycloheptyl, cyclooctyl, bicyclo[2.2.1]heptyl, bicyclo[2.2.2]octyl or adamantyl.
Cycloalkyls with 3, 4, 5, 6 or 7 carbon atoms are preferred, for example cyclopropyl or cyclobutyl.

The term "halogen" represents fluoro, chloro, bromo or iodo, particularly fluoro, chloro or bromo. The chemical groups which are substituted with halogen, for example halogenalkyl, halogencycloalkyl, halogenalkyloxy, halogenalkylsulfanyl, halogenalkylsulfinyl or halogenalkylsulfonyl are substituted one or up to the maximum number of substituents with halogen. If "alkyl", "alkenyl" or "alkinyl" are substituted with halogen, the halogen atoms can be the same or different and can be bound at the same carbon atom or different carbon atoms.

The term "alkoxy" represents straight or branched chain O-alkyl, preferably having 1 to 6 carbon atoms, for example methoxy, ethoxy, n-propoxy, isopropoxy, n-butoxy, isobutoxy, s-butoxy und t-butoxy. Alkoxy having 1 to 4 carbon atoms are preferred.

The term "cycloalkenyl" - in isolation or as part of a chemical group - represents saturated or partially unsaturated mono-, bi- or tricyclic hydrocarbons, preferably 3 to 10 carbon atoms, which cyclic hydrocarbons have at least one double bond, for example cyclopropenyl, cyclobutenyl, cyclopentenyl, cyclohexenyl, cycloheptenyl, cyclooctenyl.

The term "heteroaromatic monocyclic ring" represents a 5 to 7 membered aromatic ring which contains 1 to 3 heteroatoms selected from oxygen, nitrogen orsulphur. Examples of 5-membered heteroaryl rings in this instance include furanyl, pyrrolyl, oxazolyl, thiazolyl, imidazolyl, oxadiazolyl, thiadiazolyl, triazolyl, isothiazolyl, isoxazolyl, thiophenyl, or pyrazolyl. Examples of 6-membered heteroaryl rings include pyridinyl, pyridazinyl, pyrimidinyl, pyrazinyl, or triazinyl.

The term "5 to 6 membered heterocyclyl" or "5 to 6 membered heterocyclic ring" represents a 5 or 6 membered saturated or partically unsaturated ring which contains 1 to 3 heteroatoms independently selected from oxygen, nitrogen and sulphur. Suitable examples of such rings include pyrrolidinyl, piperidinyl, piperazinyl, morpholinyl, homomorpholinyl, thiomorpholinyl, tetrahydrofuranyl, tetrahydropyranyl, tetrahydrothiophenyl, pyrrolinyl or oxazolinyl.

The term "7 to 10-membered bicyclic ring system" represents a fused bicyclic ring system which contains 0 to 3 heteroatoms selected from oxygen, nitrogen or sulphur. The rings may be fully saturated, partially saturated or aromatic. Examples include but are not limited to indolinyl, indolyl, isoindolinyl, isoindolyl, indenyl, benzofuranyl, benzothiophenyl, indazolyl, benzimidazolyl, benzothiazolyl, benzoxazolyl, benzoxazinyl, benzopyranyl, benzothiopyranyl, quinolinyl, isoquinolinyl, chromenyl, cinnolinyl, phthalazinyl, quinazolinyl, quinoxalinyl, naphthyridinyl, furopyridinyl, naphthyl, dihydrobenzoxazinyl, dihydrochromenyl, dihydrobenzodioxinyl, tetrahydroquinolinyl, tetrahydroquinoxalinyl, tetrahydronaphthalenyl dihydrobenzofuranyl, dihydrobenzopyranyl, dihydrobenzothiopyranyl, dihydrobenzothiophenyl, dihydrodioxinopyridinyl, dihydroindenyl, dihydropyrrolopyridinyl, dihydropyrrolopyrimidinyl, dihydropyrrolopyrazinyl, dihydropyrrolopyridazinyl, pyrrolopyridinyl, pyrrolopyrazinyl, pyrrolopyridazinyl, pyrrolopyrimidinyl, furopyrimidinyl, furopyrazinyl, furopyridazinyl, thienopyridinyl, thienopyrazinyl, thienopyridazinyl, thienopyrimidinyl, pyrazolopyridinyl, pyrazolopyrazinyl, pyrazolopyridazinyl, pyrazolopyrimidinyl, imidazopyridinyl, imidazopyrazinyl, imidazopyridazinyl, imidazopyrimidinyl, thiazolopyridinyl, thiazolopyrazinyl, thiazolopyridazinyl, thiazolopyrimidinyl, oxazolopyridinyl, oxazolopyrazinyl, oxazolopyridazinyl, oxazolopyrimidinyl, pyridopyrazinyl, pyridopyridazinyl, pyridopyrimidinyl, pyridooxazinyl, pyrazinooxazinyl, pyridazinooxazinyl, pyrimidooxazinyl, dihydropyridooxazinyl, dihydropyrazinooxazinyl, dihydropyridazinooxazinyl, dihydropyrimidooxazinyl, dihydropyranopyridinyl, dihydropyranopyrazinyl, dihydropyranopyridazinyl, dihydropyrimidinyl, pyranopyridinyl, pyranopyrimidinyl, pyranopyrazinyl, pyranopyridazinyl, dihydrodioxinopyridinyl, dihydrodioxinopyrazinyl, dihydrodioxinopyridazinyl, dihydrodioxinopyrimidinyl, tetrahydronaphthyridinyl, tetrahydropyridopyridazinyl, tetrahydropyridopyrazinyl, tetrahydropyridopyrimidinyl, tetrahydropyrazinopyridazinyl, tetrahydropteridinyl, tetrahydropyrazinopyrazinyl, tetrahydroquinolinyl, tetrahydrocinnolinyl, tetrahydroquinazolinyl, tetrahydroquinoxalinyl, thiinopyridinyl, thiinopyrazinyl, thiinopyridazinyl, thiinopyrimidinyl, dihydrothiinopyridinyl, dihydrothiinopyrazinyl, dihydrothiinopyridazinyl, dihydrothiinopyrimidinyl, dihydrofuropyridinyl, dihydrofuropyrazinyl, dihydrofuropyridazinyl, dihydrofuropyrimidinyl, dihydrothienopyridinyl, dihydrothienopyrazinyl, dihydrothienopyridazinyl, dihydrothienopyrimidinyl, dihydrocyclopentapyridinyl, dihydrocyclopentapyrazinyl, dihydrocyclopentapyridazinyl, dihydrocyclopentapyrimidinyl, quinolinonyl, naphtyridinonyl, pyridopyrazinonyl, pyridopyridazinonyl and pyridopyrimidinonyl. The term "8-, 9- or 10-membered heteroaromatic bicyclic ring system" represents a subclass of "7 to 11-membered bicyclic ring systems" where the bicyclic ring system is aromatic and contains one or more heteroatoms independently selected from oxygen, nitrogen and sulphur.

A compound according to embodiment 1 which has at least one basic centre can form, for example, acid addition salts, for example with strong inorganic acids such as mineral acids, for example perchloric acid, sulfuric acid, nitric acid, nitrose acid, a phosphorus acid or a hydrohalic acid, with strong organic carboxylic acids, such as C₁-C₄alkanecarboxylic acids which are unsubstituted or substituted, for example by halogen, for example acetic acid, such as saturated or unsaturated dicarboxylic acids, for example oxalic acid, malonic acid, succinic acid, maleic acid, fumaric acid or phthalic acid, such as hydroxycarboxylic acids, for example ascorbic acid, lactic acid, malic acid, tartaric acid or citric acid, or such as benzoic acid, or with organic sulfonic acids, such as C₁-C₄alkane- or arylsulfonic acids which are unsubstituted or substituted, for example by halogen, for example methane- or p-toluenesulfonic acid. A compound according to embodiment 1 which have at least one acidic group can form, for example, salts with bases, for example mineral salts such as alkali metal or alkaline earth metal salts, for example sodium, potassium or magnesium salts, or salts with ammonia or an organic amine, such as morpholine, piperidine, pyrrolidine, a mono-, di- or tri-lower-alkylamine, for example ethyl-, diethyl-, triethyl- or dimethylpropylamine, or a mono-, di- or trihydroxy-lower-alkylamine, for example mono-, di- or triethanolamine.

Compounds according to embodiment 1 also include hydrates which may be formed during the salt formation.

The compounds according to embodiment 1 may be made by a variety of methods well known to a person skilled in the art or as shown in Schemes 1 to 7. Further instructions regarding the preparation can be found in WO2013/063282, WO2013/173218, WO2015/077436, WO2015/091645 and WO2015/022293. 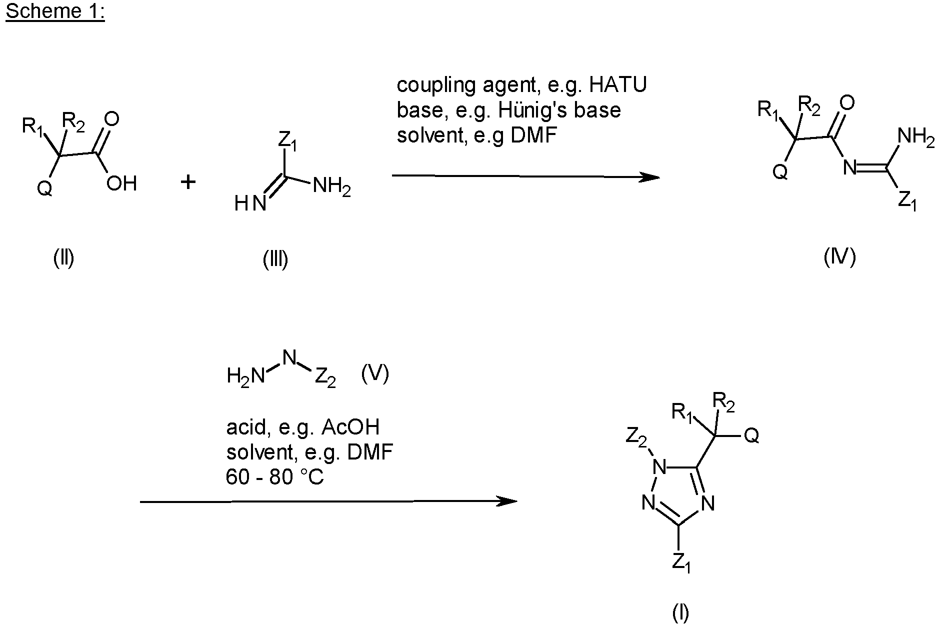 wherein Q, Z¹, Z², R¹ and R² are defined as above.

Reaction of carboxylic acids (II) with primary amidines (III) deliver the acyl amidines (IV) in the presence of a coupling agent, e.g. HATU (2-(7-aza-1H-benzotraizole-1-yl)-1,1,3,3-tetramethyluronium hexafluorophopshate) or EDC ((N-(3-dimethylaminopropyl)-N'-ethylcarbodiimide). The acylimidates (IV) are generated *in situ* and directly treated with hydrazines (V) in the presence of an acid, e.g. acetic acid, in a one-pot fashion. The reaction can be performed in a suitable solvent such as dipolar aprotic solvents, e.g. in DMF at a temperature range of 25 to 100°C, preferable at 70-80 °C (see e. g. J. Org. Chem. 2011,76, pages 1177-1179).

Intermediate amines of formula (II) may be obtained as shown in Scheme 2 by treatment of compounds of formula (VI) with compounds of formula (VII) or directly with compounds of formula (IX) wherein LG stands for a leaving group selected from iodine, bromine, chlorine, tosylate or mesylate and wherein Rx is C₁₋₄alkyl. Compounds (VI) and (VII) are commercially available or may be prepared according to known methods. 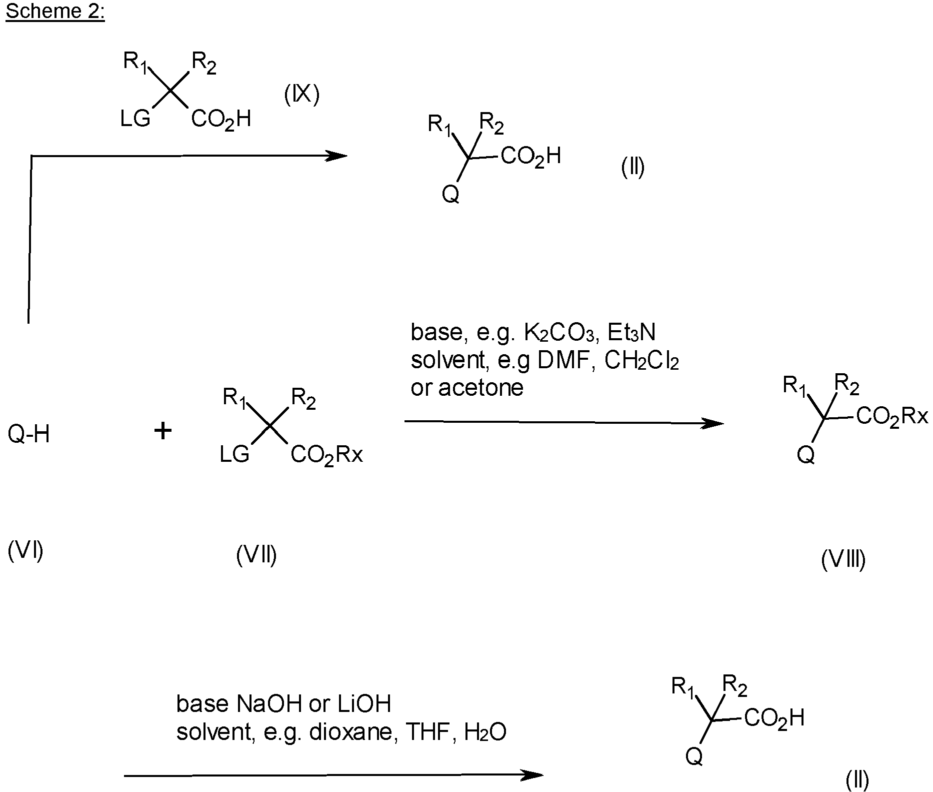 wherein Q, R¹ and R² are defined above.

Compounds of formula (I) wherein R1 and R2 is H may also be prepared by the method shown in Scheme 3. 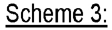 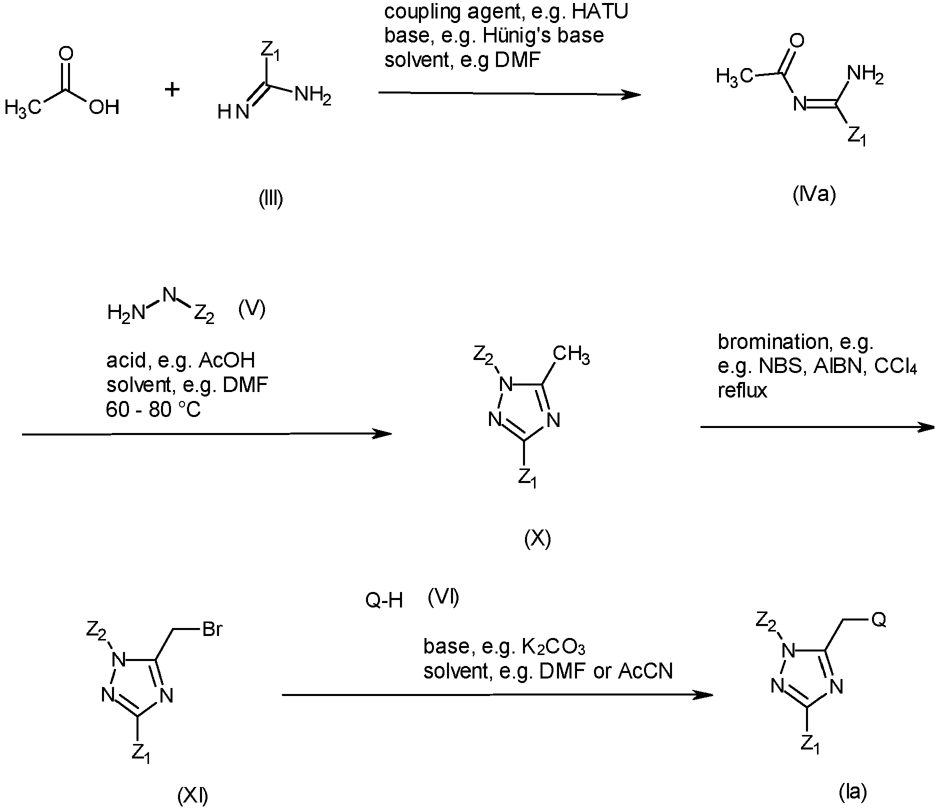 wherein Q, Z¹, Z², R¹ and R² are defined above.

In this method, acetic acid is reacted with appropriately substituted amidines (III) in a one-pot reaction described above to afford the intermediate (X) (see again J. Org. Chem. 2011,76, pages 1177-1179). Compound (X) is converted to the corresponding bromide of formula (XI) by known methods, e.g. treatment with N-Bromosuccinimide (NBS) in the presence of azoisobutyronitrile (AIBN) or benzoylperoxide (BPO) in a solvent under refluxing conditions, preferable in CCl4 (see for example WO2008/074991, page 47). Reaction of the intermediate bromide of formula (XI) with an amine of formula (VI) provides compounds of formula (la). Alkylation of the amine (VI) with compounds of formula (XI) can be achieved by using standard procedures as described for example in J. Med. Chem. 2008, 51, pages 7216-723

Compounds of formula (I) may also be synthesized according to Scheme 4. 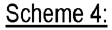 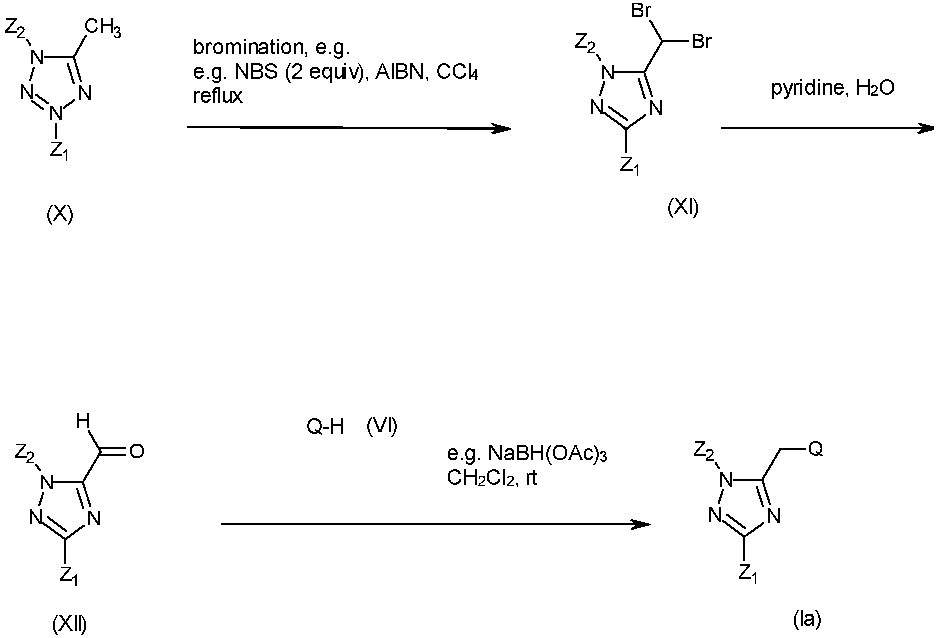 wherein Q, Z¹ and Z² are defined above.

Double bromination of compounds of formula (X) provides compounds of formula (XI). Subsequent treatment of compound (XI) with pyridine at refluxing conditions and hydrolysis with H₂O delivers carbonyl compounds of formula (XII), see e.g. Tetrahedron 2008, 64, pages 688-695. Finally, reductive amination of appropriately substituted carbonyl compounds of formula (XII) with reducing reagents such as sodium triacetoxyborohydride (see for e.g. WO2007/129195, page 55) delivers compounds of formula (Ia). The reaction is typically carried out in an inert solvent such as CH₂Cl₂ at room temperature.

Compounds of formula (I) wherein Z1, Z2, B1, B2, B3, B4 and M are definded as above can also be prepared by the method shown in Scheme 5. 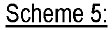 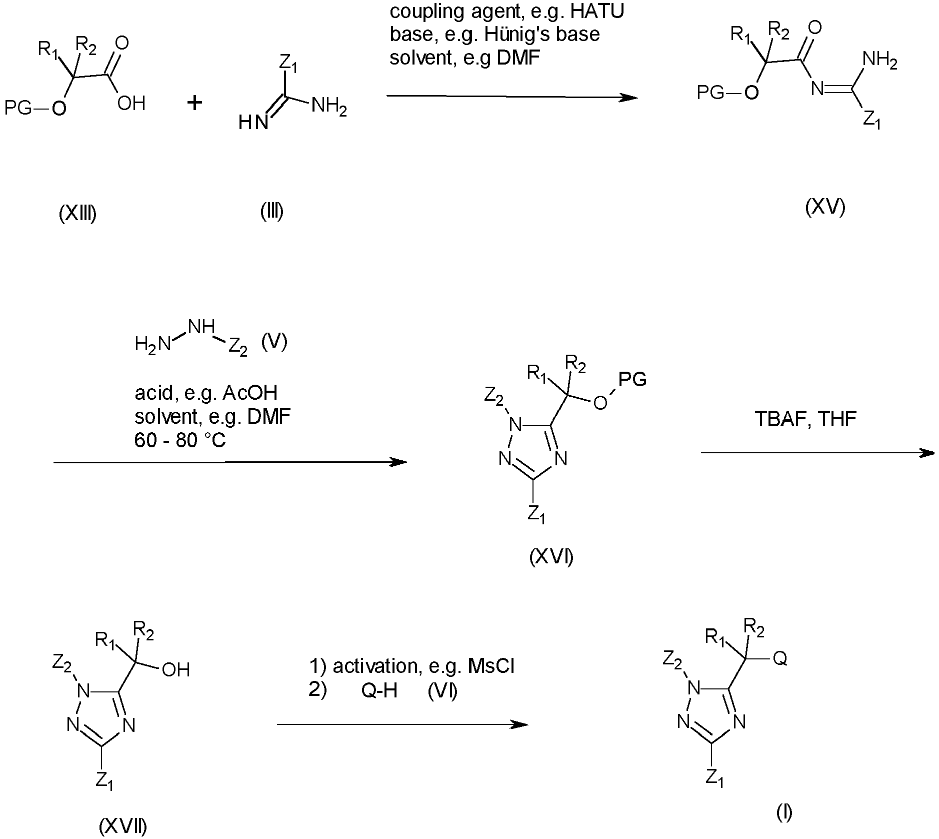 wherein Q, Z¹, Z², R¹ and R² are defined above.

Reaction of carboxylic acids (XII) equipped with a protecting group PG (e.g. tert-butyl-dimethylsilyl = TBS) with primary amidines (III) delivers the transient acyl amidines (XV) similar as already described in Scheme 1. Treatment with hydrazines (V) provides compounds of formula (XVI), which upon deprotection with tetrabutyl ammoniumfluoride (TBAF) under reaction conditions known in the art delivers compounds of formula (XVII). The alcohols are then transformed into the corresponding mesylates by treatment with mesylchloride (MsCl) in the presence of a base, e.g. triethyl amine or pyridine, preferable in CH₂Cl₂ as solvent. Final reaction with compounds of formula (VI) delivers in a similar manner the compounds of formula (I) as already shown in Scheme 3.

Compounds of formula (Ib) may be prepared starting from the respective hydroxypiperidine (XVIII) according to Scheme 6 (see e.g. WO2015/022293). 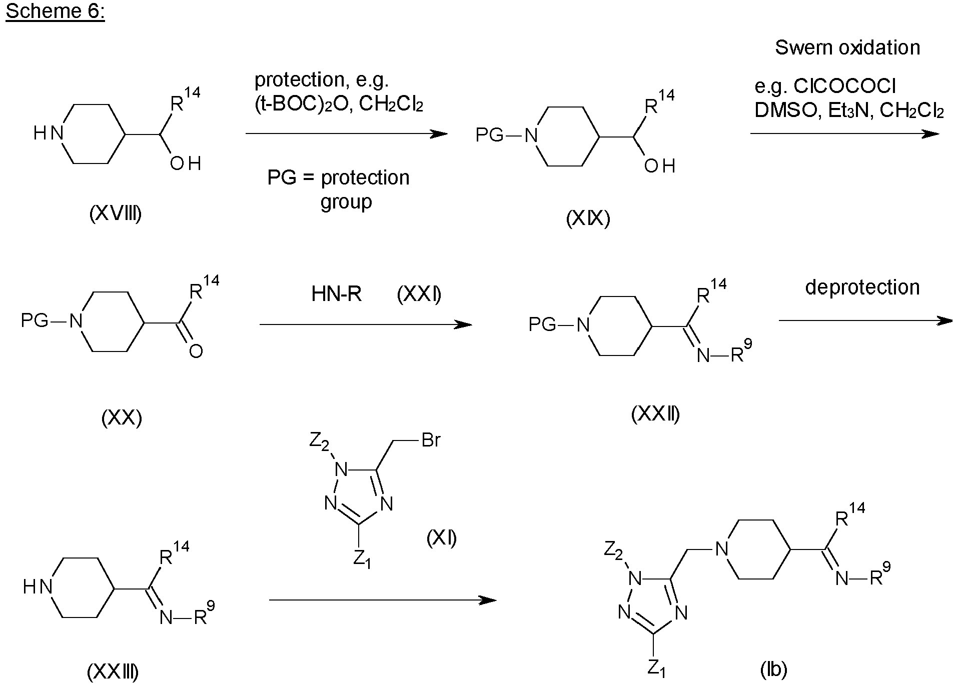 wherein Q, Z¹, Z², R⁹ and R¹⁴ are defined above, PG is a suitable protection group, e.g. tert-butyl carbamate.

Protection of intermediates (XVIII) with a suitable protection group (PG e.g. tert-butyl carbamate) leads to intermediates (XIX). Oxidation of the latter analogous to methods described for example in Bioorg. Med. Chem. Lett. 2001, 11, pages 491-494 gives compounds of formula (XX). Amination of compounds of formula (XX) as reported in WO2015/022293 is accomplished with amines of formula (XXI). Deprotection in the usual manner (see. e.g. T. W. Greene et al. "Protective Groups in Organic Synthesis, J. Wiley & Sons, 4th Edition, page 697) provides compounds of formula (XXII). Treatment of intermediates (XXII) with compounds of formula (XI) under similar conditions as already described in Scheme 3 gives final compounds (Ib).

Compounds of formula (XI) are reacted with pyridine derivatives of formula (XXIV) to form the pyridinium salts (XXV) as intermediates. The reaction is carried out in a suitable solvent, preferable in acetonitrile at a temperature between 25 - 100°C, preferably at 70- 80°C. The pyridinium salts (XXV) are then treated in the next step with NaBHa in ethanol at a temperature range from 0 to 25°C, preferably at 15°C to give final compounds (Ic), according to methods described in e.g. WO2005/0239826, page 22.

A compound according to embodiment 1 can be converted in a manner known per se into another compound according to embodiment 1 by replacing one or more substituents of the starting compound according to embodiment 1 in the customary manner by (an) other substituent(s) according to the invention.

Depending on the choice of the reaction conditions and starting materials which are suitable in each case, it is possible, for example, in one reaction step only to replace one substituent by another substituent according to the invention, or a plurality of substituents can be replaced by other substituents according to the invention in the same reaction step.

Salts of compounds of formula (I) may be prepared in a manner known per se. Thus, for example, acid addition salts of compounds according to embodiment 1 are obtained by treatment with a suitable acid or a suitable ion exchanger reagent and salts with bases are obtained by treatment with a suitable base or with a suitable ion exchanger reagent.

Salts of compounds according to embodiment 1 can be converted in the customary manner into the free compounds, acid addition salts, for example, by treatment with a suitable basic compound or with a suitable ion exchanger reagent and salts with bases, for example, by treatment with a suitable acid or with a suitable ion exchanger reagent.

Salts of compounds according to embodiment 1 can be converted in a manner known per se into other salts of compounds according to embodiment 1, acid addition salts, for example, into other acid addition salts, for example by treatment of a salt of inorganic acid such as hydrochloride with a suitable metal salt such as a sodium, barium or silver salt, of an acid, for example with silver acetate, in a suitable solvent in which an inorganic salt which forms, for example silver chloride, is insoluble and thus precipitates from the reaction mixture.

Depending on the procedure or the reaction conditions, the compounds according to embodiment 1 , which have salt-forming properties can be obtained in free form or in the form of salts.

The compounds according to embodiment 1 and, where appropriate, the tautomers thereof, in each case in free form or in salt form, can be present in the form of one of the stereoisomers which are possible or as a mixture of these, for example in the form of pure stereoisomers, such as antipodes and/or diastereomers, or as stereoisomer mixtures, such as enantiomer mixtures, for example racemates, diastereomer mixtures or racemate mixtures, depending on the number, absolute and relative configuration of asymmetric carbon atoms which occur in the molecule and/or depending on the configuration of non-aromatic double bonds which occur in the molecule; the invention relates to the pure stereoisomers and also to all stereoisomer mixtures which are possible and is to be understood in each case in this sense hereinabove and hereinbelow, even when stereochemical details are not mentioned specifically in each case.

Diastereomer mixtures or racemate mixtures of compounds according to embodiment 1 , in free form or in salt form, which can be obtained depending on which starting materials and procedures have been chosen can be separated in a known manner into the pure diasteromers or racemates on the basis of the physicochemical differences of the components, for example by fractional crystallization, distillation and/or chromatography.

Enantiomer mixtures, such as racemates, which can be obtained in a similar manner can be resolved into the optical antipodes by known methods, for example by recrystallization from an optically active solvent, by chromatography on chiral adsorbents, for example high-performance liquid chromatography (HPLC) on acetyl celulose, with the aid of suitable microorganisms, by cleavage with specific, immobilized enzymes, via the formation of inclusion compounds, for example using chiral crown ethers, where only one enantiomer is complexed, or by conversion into diastereomeric salts, for example by reacting a basic end-product racemate with an optically active acid, such as a carboxylic acid, for example camphor, tartaric or malic acid, or sulfonic acid, for example camphorsulfonic acid, and separating the diastereomer mixture which can be obtained in this manner, for example by fractional crystallization based on their differing solubilities, to give the diastereomers, from which the desired enantiomer can be set free by the action of suitable agents, for example basic agents.

Pure diastereomers or enantiomers can be obtained according to the invention not only by separating suitable stereoisomer mixtures, but also by generally known methods of diastereoselective or enantioselective synthesis, for example by carrying out the process according to the invention with starting materials of a suitable stereochemistry.
N-oxides may be prepared by reacting a compound according to embodiment 1 with a suitable oxidizing agent, for example the H₂O₂/urea adduct in the presence of an acid anhydride, e.g. trifluoroacetic anhydride. Such oxidations are known from the literature, for example from J. Med. Chem., 32 (12), 2561-73, 1989 or WO 00/15615.

It is advantageous to isolate or synthesize in each case the biologically more effective stereoisomer, for example enantiomer or diastereomer, or stereoisomer mixture, for example enantiomer mixture or diastereomer mixture, if the individual components have a different biological activity.
The compounds according to embodiment 1 and, where appropriate, the tautomers thereof, in each case in free form or in salt form, can, if appropriate, also be obtained in the form of hydrates and/or include other solvents, for example those which may have been used for the crystallization of compounds which are present in solid form.

The compounds of the invention can be distinguished from known compounds by virtue of greater efficacy at low application rates, which can be verified by the person skilled in the art using the experimental procedures outlined in the Examples, using lower application rates if necessary, for example 50 ppm, 12.5 ppm, 6 ppm, 3 ppm, 1.5 ppm or 0.8 ppm.

The compounds according to embodiment 1 are preventively and/or curatively valuable active ingredients in the field of pest control, even at low rates of application, which have a very favorable biocidal spectrum and are well tolerated by warm-blooded species, fish and plants. The active ingredients according to the invention act against all or individual developmental stages of normally sensitive, but also resistant, animal pests, such as insects or representatives of the order Acarina. The insecticidal or acaricidal activity of the active ingredients according to the invention can manifest itself directly, i. e. in destruction of the pests, which takes place either immediately or only after some time has elapsed, for example during ecdysis, or indirectly, for example in a reduced oviposition and/or hatching rate.

Examples of the above mentioned animal pests are:
from the order *Acarina,* for example,
Acalitus spp, Aculus spp, Acaricalus spp, Aceria spp, Acarus siro, Amblyomma spp., Argas spp., Boophilus spp., Brevipalpus spp., Bryobia spp, Calipitrimerus spp., Chorioptes spp., Dermanyssus gallinae, Dermatophagoides spp, Eotetranychus spp, Eriophyes spp., Hemitarsonemus spp, Hyalomma spp., Ixodes spp., Olygonychus spp, Ornithodoros spp., Polyphagotarsone latus, Panonychus spp., Phyllocoptruta oleivora, Phytonemus spp, Polyphagotarsonemus spp, Psoroptes spp., Rhipicephalus spp., Rhizoglyphus spp., Sarcoptes spp., Steneotarsonemus spp, Tarsonemus spp. and Tetranychus spp.;
from the order *Anoplura,* for example,
Haematopinus spp., Linognathus spp., Pediculus spp., Pemphigus spp. and Phylloxera spp.;
from the order *Coleoptera,* for example,
Agriotes spp., Amphimallon majale, Anomala orientalis, Anthonomus spp., Aphodius spp, Astylus atromaculatus, Ataenius spp, Atomaria linearis, Chaetocnema tibialis, Cerotoma spp, Conoderus spp, Cosmopolites spp., Cotinis nitida, Curculio spp., Cyclocephala spp, Dermestes spp., Diabrotica spp., Diloboderus abderus, Epilachna spp., Eremnus spp., Heteronychus arator, Hypothenemus hampei, Lagria vilosa, Leptinotarsa decemLineata, Lissorhoptrus spp., Liogenys spp, Maecolaspis spp, Maladera castanea, Megascelis spp, Melighetes aeneus, Melolontha spp., Myochrous armatus, Orycaephilus spp., Otiorhynchus spp., Phyllophaga spp, Phlyctinus spp., Popillia spp., Psylliodes spp., Rhyssomatus aubtilis, Rhizopertha spp., Scarabeidae, Sitophilus spp., Sitotroga spp., Somaticus spp, Sphenophorus spp, Sternechus subsignatus, Tenebrio spp., Tribolium spp. and Trogoderma spp.; from the order *Diptera,* for example,
Aedes spp., Anopheles spp, Antherigona soccata, Bactrocea oleae, Bibio hortulanus, Bradysia spp, Calliphora erythrocephala, Ceratitis spp., Chrysomyia spp., Culex spp., Cuterebra spp., Dacus spp., Delia spp, Drosophila melanogaster, Fannia spp., Gastrophilus spp., Geomyza tripunctata, Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis spp, Rivelia quadrifasciata, Scatella spp, Sciara spp., Stomoxys spp., Tabanus spp., Tannia spp. and Tipula spp.;
from the order *Hemiptera,* for example,
Acanthocoris scabrator, Acrosternum spp, Adelphocoris lineolatus, Amblypelta nitida, Bathycoelia thalassina, Blissus spp, Cimex spp., Clavigralla tomentosicollis, Creontiades spp, Distantiella theobroma, Dichelops furcatus, Dysdercus spp., Edessa spp, Euchistus spp., Eurydema pulchrum, Eurygaster spp., Halyomorpha halys, Horcias nobilellus, Leptocorisa spp., Lygus spp, Margarodes spp, Murgantia histrionic, Neomegalotomus spp, Nesidiocoris tenuis, Nezara spp., Nysius simulans, Oebalus insularis, Piesma spp., Piezodorus spp, Rhodnius spp., Sahlbergella singularis, Scaptocoris castanea, Scotinophara spp. , Thyanta spp , Triatoma spp., Vatiga illudens;
Acyrthosium pisum, Adalges spp, Agalliana ensigera, Agonoscena targionii, Aleurodicus spp, Aleurocanthus spp, Aleurolobus barodensis, Aleurothrixus floccosus, Aleyrodes brassicae, Amarasca biguttula, Amritodus atkinsoni, Aonidiella spp., Aphididae, Aphis spp., Aspidiotus spp., Aulacorthum solani, Bactericera cockerelli, Bemisia spp, Brachycaudus spp, Brevicoryne brassicae, Cacopsylla spp, Cavariella aegopodii Scop., Ceroplaster spp., Chrysomphalus aonidium, Chrysomphalus dictyospermi, Cicadella spp, Cofana spectra, Cryptomyzus spp, Cicadulina spp, Coccus hesperidum, Dalbulus maidis, Dialeurodes spp, Diaphorina citri, Diuraphis noxia, Dysaphis spp, Empoasca spp., Eriosoma larigerum, Erythroneura spp., Gascardia spp., Glycaspis brimblecombei, Hyadaphis pseudobrassicae, Hyalopterus spp, Hyperomyzus pallidus, Idioscopus clypealis, Jacobiasca lybica, Laodelphax spp., Lecanium corni, Lepidosaphes spp., Lopaphis erysimi, Lyogenys maidis, Macrosiphum spp., Mahanarva spp, Metcalfa pruinosa, Metopolophium dirhodum, Myndus crudus, Myzus spp., Neotoxoptera sp, Nephotettix spp., Nilaparvata spp., Nippolachnus piri Mats, Odonaspis ruthae, Oregma lanigera Zehnter, Parabemisia myricae, Paratrioza cockerelli, Parlatoria spp., Pemphigus spp., Peregrinus maidis, Perkinsiella spp, Phorodon humuli, Phylloxera spp, Planococcus spp., Pseudaulacaspis spp., Pseudococcus spp., Pseudatomoscelis seriatus, Psylla spp., Pulvinaria aethiopica, Quadraspidiotus spp., Quesada gigas, Recilia dorsalis, Rhopalosiphum spp., Saissetia spp., Scaphoideus spp., Schizaphis spp., Sitobion spp., Sogatella furcifera, Spissistilus festinus, Tarophagus Proserpina, Toxoptera spp, Trialeurodes spp, Tridiscus sporoboli, Trionymus spp, Trioza erytreae , Unaspis citri, Zygina flammigera, Zyginidia scutellaris, ;
from the order *Hymenoptera,* for example,
Acromyrmex, Arge spp, Atta spp., Cephus spp., Diprion spp., Diprionidae, Gilpinia polytoma, Hoplo-campa spp., Lasius spp., Monomorium pharaonis, Neodiprion spp., Pogonomyrmex spp, Slenopsis invicta, Solenopsis spp. and Vespa spp.;
from the order *Isoptera,* for example,
Coptotermes spp, Corniternes cumulans, Incisitermes spp, Macrotermes spp, Mastotermes spp, Microtermes spp, Reticulitermes spp.; Solenopsis geminate
from the order *Lepidoptera,* for example,
Acleris spp., Adoxophyes spp., Aegeria spp., Agrotis spp., Alabama argillaceae, Amylois spp., Anticarsia gemmatalis, Archips spp., Argyresthia spp, Argyrotaenia spp., Autographa spp., Bucculatrix thurberiella, Busseola fusca, Cadra cautella, Carposina nipponensis, Chilo spp., Choristoneura spp., Chrysoteuchia topiaria, Clysia ambiguella, Cnaphalocrocis spp., Cnephasia spp., Cochylis spp., Coleophora spp., Colias lesbia, Cosmophila flava, Crambus spp, Crocidolomia binotalis, Cryptophlebia leucotreta, Cydalima perspectalis, Cydia spp., Diaphania perspectalis, Diatraea spp., Diparopsis castanea, Earias spp., Eldana saccharina, Ephestia spp., Epinotia spp, Estigmene acrea, Etiella zinckinella, Eucosma spp., Eupoecilia ambiguella, Euproctis spp., Euxoa spp., Feltia jaculiferia, Grapholita spp., Hedya nubiferana, Heliothis spp., Hellula undalis, Herpetogramma spp, Hyphantria cunea, Keiferia lycopersicella, Lasmopalpus lignosellus, Leucoptera scitella, Lithocollethis spp., Lobesia botrana, Loxostege bifidalis, Lymantria spp., Lyonetia spp., Malacosoma spp., Mamestra brassicae, Manduca sexta, Mythimna spp, Noctua spp, Operophtera spp., Orniodes indica, Ostrinia nubilalis, Pammene spp., Pandemis spp., Panolis flammea, Papaipema nebris, Pectinophora gossypiela, Perileucoptera coffeella, Pseudaletia unipuncta, Phthorimaea operculella, Pieris rapae, Pieris spp., Plutella xylostella, Prays spp., Pseudoplusia spp, Rachiplusia nu, Richia albicosta, Scirpophaga spp., Sesamia spp., Sparganothis spp., Spodoptera spp., Sylepta derogate, Synanthedon spp., Thaumetopoea spp., Tortrix spp., Trichoplusia ni, Tuta absoluta, and Yponomeuta spp.;
from the order *Mallophaga,* for example,
Damalinea spp. and Trichodectes spp.;
from the order *Orthoptera,* for example,
Blatta spp., Blattella spp., Gryllotalpa spp., Leucophaea maderae, Locusta spp., Neocurtilla hexadactyla, Periplaneta spp. , Scapteriscus spp, and Schistocerca spp.;
from the order *Psocoptera,* for example,
Liposcelis spp.;
from the order *Siphonaptera,* for example,
Ceratophyllus spp., Ctenocephalides spp. and Xenopsylla cheopis;
from the order *Thysanoptera,* for example,
Calliothrips phaseoli, Frankliniella spp., Heliothrips spp, Hercinothrips spp., Parthenothrips spp, Scirtothrips aurantii, Sericothrips variabilis, Taeniothrips spp., Thrips spp;
from the order *Thysanura,* for example, Lepisma saccharina.

The active ingredients according to the invention can be used for controlling, i. e. containing or destroying, pests of the abovementioned type which occur in particular on plants, especially on useful plants and ornamentals in agriculture, in horticulture and in forests, or on organs, such as fruits, flowers, foliage, stalks, tubers or roots, of such plants, and in some cases even plant organs which are formed at a later point in time remain protected against these pests.

Suitable target crops are, in particular, cereals, such as wheat, barley, rye, oats, rice, maize or sorghum; beet, such as sugar or fodder beet; fruit, for example pomaceous fruit, stone fruit or soft fruit, such as apples, pears, plums, peaches, almonds, cherries or berries, for example strawberries, raspberries or blackberries; leguminous crops, such as beans, lentils, peas or soya; oil crops, such as oilseed rape, mustard, poppies, olives, sunflowers, coconut, castor, cocoa or ground nuts; cucurbits, such as pumpkins, cucumbers or melons; fibre plants, such as cotton, flax, hemp or jute; citrus fruit, such as oranges, lemons, grapefruit or tangerines; vegetables, such as spinach, lettuce, asparagus, cabbages, carrots, onions, tomatoes, potatoes or bell peppers; Lauraceae, such as avocado, Cinnamonium or camphor; and also tobacco, nuts, coffee, eggplants, sugarcane, tea, pepper, grapevines, hops, the plantain family, latex plants and ornamentals.

The active ingredients according to the invention are especially suitable for controlling Aphis craccivora, Diabrotica balteata, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca(preferably in vegetables, vineyards), Leptinotarsa (preferably in potatos) and Chilo supressalis (preferably in rice).

In a further aspect, the invention may also relate to a method of controlling damage to plant and parts thereof by plant parasitic nematodes (Endoparasitic-, Semiendoparasitic- and Ectoparasitic nematodes), especially plant parasitic nematodes such as root knot nematodes, Meloidogyne hapla, Meloidogyne incognita, Meloidogyne javanica, Meloidogyne arenaria and other Meloidogyne species; cyst-forming nematodes, Globodera rostochiensis and other Globodera species; Heterodera avenae, Heterodera glycines, Heterodera schachtii, Heterodera trifolii, and other Heterodera species; Seed gall nematodes, Anguina species; Stem and foliar nematodes, Aphelenchoides species; Sting nematodes, Belonolaimus longicaudatus and other Belonolaimus species; Pine nematodes, Bursaphelenchus xylophilus and other Bursaphelenchus species; Ring nematodes, Criconema species, Criconemella species, Criconemoides species, Mesocriconema species; Stem and bulb nematodes, Ditylenchus destructor, Ditylenchus dipsaci and other Ditylenchus species; Awl nematodes, Dolichodorus species; Spiral nematodes, Heliocotylenchus multicinctus and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, Longidorus elongatus and other Longidorus species; Pin nematodes, Pratylenchus species; Lesion nematodes, Pratylenchus neglectus, Pratylenchus penetrans, Pratylenchus curvitatus, Pratylenchus goodeyi and other Pratylenchus species; Burrowing nematodes, Radopholus similis and other Radopholus species; Reniform nematodes, Rotylenchus robustus, Rotylenchus reniformis and other Rotylenchus species; Scutellonema species; Stubby root nematodes, Trichodorus primitivus and other Trichodorus species, Paratrichodorus species; Stunt nematodes, Tylenchorhynchus claytoni, Tylenchorhynchus dubius and other Tylenchorhynchus species; Citrus nematodes, Tylenchulus species; Dagger nematodes, Xiphinema species; and other plant parasitic nematode species, such as Subanguina spp., Hypsoperine spp., Macroposthonia spp., Melinius spp., Punctodera spp., and Quinisulcius spp..

The compounds according to embodiment 1 may also have activity against the molluscs. Examples of which include, for example, Ampullariidae; Arion (A. ater, A. circumscriptus, A. hortensis, A. rufus); Bradybaenidae (Bradybaena fruticum); Cepaea (C. hortensis, C. Nemoralis); ochlodina; Deroceras (D. agrestis, D. empiricorum, D. laeve, D. reticulatum); Discus (D. rotundatus); Euomphalia; Galba (G. trunculata); Helicelia (H. itala, H. obvia); Helicidae Helicigona arbustorum); Helicodiscus; Helix (H. aperta); Limax (L. cinereoniger, L. flavus, L. marginatus, L. maximus, L. tenellus); Lymnaea; Milax (M. gagates, M. marginatus, M. sowerbyi); Opeas; Pomacea (P. canaticulata); Vallonia and Zanitoides.

The term "crops" is to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins, for example insecticidal proteins from Bacillus cereus or Bacillus popilliae; or insecticidal proteins from Bacillus thuringiensis, such as δ-endotoxins, e.g. Cry1Ab, Cry1Ac, Cry1F, Cry1Fa2, Cry2Ab, Cry3A, Cry3Bb1 or Cry9C, or vegetative insecticidal proteins (Vip), e.g. Vip1, Vip2, Vip3 or Vip3A; or insecticidal proteins of bacteria colonising nematodes, for example Photorhabdus spp. or Xenorhabdus spp., such as Photorhabdus luminescens, Xenorhabdus nematophilus; toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsin inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.
In the context of the present invention there are to be understood by δ-endotoxins, for example Cry1Ab, Cry1Ac, Cry1F, Cry1Fa2, Cry2Ab, Cry3A, Cry3Bb1 or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO 02/15701). Truncated toxins, for example a truncated Cry1Ab, are known. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO 03/018810). Examples of such toxins or transgenic plants capable of synthesising such toxins are disclosed, for example, in EP-A-0 374 753, WO93/07278, WO95/34656, EP-A-0 427 529, EP-A-451 878 and WO 03/052073.
The processes for the preparation of such transgenic plants are generally known to the person skilled in the art and are described, for example, in the publications mentioned above. Cryl-type deoxyribonucleic acids and their preparation are known, for example, from WO 95/34656, EP-A-0 367 474, EP-A-0 401 979 and WO 90/13651.
The toxin contained in the transgenic plants imparts to the plants tolerance to harmful insects. Such insects can occur in any taxonomic group of insects, but are especially commonly found in the beetles (Coleoptera), two-winged insects (Diptera) and moths (Lepidoptera).
Transgenic plants containing one or more genes that code for an insecticidal resistance and express one or more toxins are known and some of them are commercially available. Examples of such plants are: YieldGard® (maize variety that expresses a Cry1Ab toxin); YieldGard Rootworm® (maize variety that expresses a Cry3Bb1 toxin); YieldGard Plus® (maize variety that expresses a Cry1Ab and a Cry3Bb1 toxin); Starlink® (maize variety that expresses a Cry9C toxin); Herculex I® (maize variety that expresses a Cry1Fa2 toxin and the enzyme phosphinothricine N-acetyltransferase (PAT) to achieve tolerance to the herbicide glufosinate ammonium); NuCOTN 33B® (cotton variety that expresses a Cry1Ac toxin); Bollgard I® (cotton variety that expresses a Cry1Ac toxin); Bollgard II®
(cotton variety that expresses a Cry1Ac and a Cry2Ab toxin); VipCot® (cotton variety that expresses a Vip3A and a Cry1Ab toxin); NewLeaf® (potato variety that expresses a Cry3A toxin); NatureGard®, Agrisure® GT Advantage (GA21 glyphosate-tolerant trait), Agrisure® CB Advantage (Bt11 corn borer (CB) trait) and Protecta®.
Further examples of such transgenic crops are:
1. **Bt11 Maize** from Syngenta Seeds SAS, Chemin de I'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a truncated Cry1Ab toxin. Bt11 maize also transgenically expresses the enzyme PAT to achieve tolerance to the herbicide glufosinate ammonium.
2. **Bt176 Maize** from Syngenta Seeds SAS, Chemin de I'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a Cry1Ab toxin. Bt176 maize also transgenically expresses the enzyme PAT to achieve tolerance to the herbicide glufosinate ammonium.
3. **MIR604 Maize** from Syngenta Seeds SAS, Chemin de I'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO 03/018810.
**4. MON 863 Maize** from Monsanto Europe S.A. 270-272 Avenue de Tervuren, B-1150 Brussels, Belgium, registration number C/DE/02/9. MON 863 expresses a Cry3Bb1 toxin and has resistance to certain Coleoptera insects.
5. **IPC 531 Cotton** from Monsanto Europe S.A. 270-272 Avenue de Tervuren, B-1150 Brussels, Belgium, registration number C/ES/96/02.
6. **1507 Maize** from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. **NK603 × MON 810 Maize** from Monsanto Europe S.A. 270-272 Avenue de Tervuren, B-1150 Brussels, Belgium, registration number C/GB/02/M3/03. Consists of conventionally bred hybrid maize varieties by crossing the genetically modified varieties NK603 and MON 810. NK603 × MON 810 Maize transgenically expresses the protein CP4 EPSPS, obtained from *Agrobacterium sp.* strain CP4, which imparts tolerance to the herbicide Roundup® (contains glyphosate), and also a Cry1Ab toxin obtained from *Bacillus thuringiensis subsp. kurstaki* which brings about tolerance to certain Lepidoptera, include the European corn borer.
Transgenic crops of insect-resistant plants are also described in BATS (Zentrum für Biosicherheit und Nachhaltigkeit, Zentrum BATS, Clarastrasse 13, 4058 Basel, Switzerland) Report 2003, (http://bats.ch).
The term "crops" is to be understood as including also crop plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO95/33818 and EP-A-0 353 191. The methods of producing such transgenic plants are generally known to the person skilled in the art and are described, for example, in the publications mentioned above.

Crops may also be modified for enhanced resistance to fungal (for example Fusarium, Anthracnose, or Phytophthora), bacterial (for example Pseudomonas) or viral (for example potato leafroll virus, tomato spotted wilt virus, cucumber mosaic virus) pathogens.

Crops also include those that have enhanced resistance to nematodes, such as the soybean cyst nematode.

Crops that are tolerant to abiotic stress include those that have enhanced tolerance to drought, high salt, high temperature, chill, frost, or light radiation, for example through expression of NF-YB or other proteins known in the art.

Antipathogenic substances which can be expressed by such transgenic plants include, for example, ion channel blockers, such as blockers for sodium and calcium channels, for example the viral KP1, KP4 or KP6 toxins; stilbene synthases; bibenzyl synthases; chitinases; glucanases; the so-called "pathogenesis-related proteins" (PRPs; see e.g. EP-A-0 392 225); antipathogenic substances produced by microorganisms, for example peptide antibiotics or heterocyclic antibiotics (see e.g. WO95/33818) or protein or polypeptide factors involved in plant pathogen defence (so-called "plant disease resistance genes", as described in WO 03/000906).

Further areas of use of the compositions according to the invention are the protection of stored goods and store rooms and the protection of raw materials, such as wood, textiles, floor coverings or buildings, and also in the hygiene sector, especially the protection of humans, domestic animals and productive livestock against pests of the mentioned type.

The present invention also provides a method for controlling pests (such as mosquitoes and other disease vectors; see also http://www.who.int/malaria/vector_control/irs/en/). In one embodiment, the method for controlling pests comprises applying the compositions of the invention to the target pests, to their locus or to a surface or substrate by brushing, rolling, spraying, spreading or dipping. By way of example, an IRS (indoor residual spraying) application of a surface such as a wall, ceiling or floor surface is contemplated by the method of the invention. In another embodiment, it is contemplated to apply such compositions to a substrate such as non-woven or a fabric material in the form of (or which can be used in the manufacture of) netting, clothing, bedding, curtains and tents.

In another embodiment, the method for controlling such pests comprises applying a pesticidally effective amount of the compositions of the invention to the target pests, to their locus, or to a surface or substrate so as to provide effective residual pesticidal activity on the surface or substrate. Such application may be made by brushing, rolling, spraying, spreading or dipping the pesticidal composition of the invention. By way of example, an IRS application of a surface such as a wall, ceiling or floor surface is contemplated by the method of the invention so as to provide effective residual pesticidal activity on the surface. In another embodiment, it is contemplated to apply such compositions for residual control of pests on a substrate such as a fabric material in the form of (or which can be used in the manufacture of) netting, clothing, bedding, curtains and tents.

Substrates including non-woven, fabrics or netting to be treated may be made of natural fibres such as cotton, raffia, jute, flax, sisal, hessian, or wool, or synthetic fibres such as polyamide, polyester, polypropylene, polyacrylonitrile or the like. The polyesters are particularly suitable. The methods of textile treatment are known, e.g. WO 2008/151984, WO 2003/034823, US 5631072, WO 2005/64072, WO2006/128870, EP 1724392, WO2005113886 or WO 2007/090739.

Further areas of use of the compositions according to the invention are the field of tree injection/trunk treatment for all ornamental trees as well all sort of fruit and nut trees.
In the field of tree injection/trunk treatment, the compounds according to the present invention are especially suitable against wood-boring insects from the order *Lepidoptera* as mentioned above and from the order *Coleoptera,* especially against woodborers listed in the following tables A and B:

**Table A. Examples of exotic woodborers of economic importance.**

| Family | Species | Host or Crop Infested |
|---|---|---|
| Buprestidae | *Agrilus planipennis* | Ash |
| Cerambycidae | *Anoplura glabripennis* | Hardwoods |
| | *Xylosandrus crassiusculus* | Hardwoods |
| Scolytidae | *X. mutilatus* | Hardwoods |
| | *Tomicus piniperda* | Conifers |

**Table B. Examples of native woodborers of economic importance.**

| Family | Species | Host or Crop Infested |
|---|---|---|
| | *Agrilus anxius* | Birch |
| | *Agrilus politus* | Willow, Maple |
| | *Agrilus sayi* | Bayberry, Sweetfern |
| | *Agrilus vittaticolllis* | Apple, Pear, Cranberry, Serviceberry, Hawthorn |
| Buprestidae | *Chrysobothris femorata* | Apple, Apricot, Beech, Boxelder, Cherry, Chestnut, Currant, Elm, Hawthorn, Hackberry, Hickory, Horsechestnut, Linden, Maple, Mountain-ash, Oak, Pecan, Pear, Peach, Persimmon, Plum, Poplar, |
| | | Quince, Redbud, Serviceberry, Sycamore, Walnut, Willow |
| | *Texania campestris* | Basswood, Beech, Maple, Oak, Sycamore, Willow, Yellow-poplar |
| | *Goes pulverulentus* | Beech, Elm, Nuttall, Willow, Black oak, Cherrybark oak, Water oak, Sycamore |
| | *Goes tigrinus* | Oak |
| | *Neoclytus acuminatus* | Ash, Hickory, Oak, Walnut, Birch, Beech, Maple, Eastern hophornbeam, Dogwood, Persimmon, Redbud, Holly, Hackberry, Black locust, Honeylocust, Yellow-poplar, Chestnut, Osage-orange, Sassafras, Lilac, Mountain-mahogany, Pear, Cherry, Plum, Peach, Apple, Elm, Basswood, Sweetgum |
| Cerambycidae | *Neoptychodes trilineatus* | Fig, Alder, Mulberry, Willow, Netleaf hackberry |
| | *Oberea ocellata* | Sumac, Apple, Peach, Plum, Pear, Currant, Blackberry |
| | *Oberea tripunctata* | Dogwood, Viburnum, Elm, Sourwood, Blueberry, Rhododendron, Azalea, Laurel, Poplar, Willow, Mulberry |
| | *Oncideres cingulata* | Hickory, Pecan, Persimmon, Elm, Sourwood, Basswood, Honeylocust, Dogwood, Eucalyptus, Oak, Hackberry, Maple, Fruit trees |
| | *Saperda calcarata* | Poplar |
| | *Strophiona nitens* | Chestnut, Oak, Hickory, Walnut, Beech, Maple |
| Scolytidae | *Corthylus columbianus* | Maple, Oak, Yellow-poplar, Beech, Boxelder, Sycamore, Birch, Basswood, Chestnut, Elm |
| | *Dendroctonus frontalis* | Pine |
| | *Dryocoetes betulae* | Birch, Sweetgum, Wild cherry, Beech, Pear |
| | *Monarthrum fasciatum* | Oak, Maple, Birch, Chestnut, Sweetgum, Blackgum, Poplar, Hickory, Mimosa, Apple, Peach, Pine |
| | *Phloeotribus liminaris* | Peach, Cherry, Plum, Black cherry, Elm, Mulberry, Mountain-ash |
| | *Pseudopityophthorus pruinosus* | Oak, American beech, Black cherry, Chickasaw plum, Chestnut, Maple, Hickory, Hornbeam, Hophornbeam |
| | *Paranthrene simulans* | Oak, American chestnut |
| | *Sannina uroceriformis* | Persimmon |
| | *Synanthedon exitiosa* | Peach, Plum, Nectarine, Cherry, Apricot, Almond, Black cherry |
| | *Synanthedon pictipes* | Peach, Plum, Cherry, Beach, Black |
| | | Cherry |
| Sesiidae | *Synanthedon rubrofascia* | Tupelo |
| | *Synanthedon scitula* | Dogwood, Pecan, Hickory, Oak, Chestnut, Beech, Birch, Black cherry, Elm, Mountain-ash, Viburnum, Willow, Apple, Loquat, Ninebark, Bayberry |
| | *Vitacea polistiformis* | Grape |

In the hygiene sector, the compositions according to the invention are active against ectoparasites such as hard ticks, soft ticks, mange mites, harvest mites, flies (biting and licking), parasitic fly larvae, lice, hair lice, bird lice and fleas.
Examples of such parasites are:
Of the order Anoplurida: Haematopinus spp., Linognathus spp., Pediculus spp. and Phtirus spp., Solenopotes spp..

Of the order Mallophagida: Trimenopon spp., Menopon spp., Trinoton spp., Bovicola spp., Werneckiella spp., Lepikentron spp., Damalina spp., Trichodectes spp. and Felicola spp..

Of the order Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp. and Melophagus spp..

Of the order Siphonapterida, for example Pulex spp., Ctenocephalides spp., Xenopsylla spp., Ceratophyllus spp..

Of the order Heteropterida, for example Cimex spp., Triatoma spp., Rhodnius spp., Panstrongylus spp..

Of the order Blattarida, for example Blatta orientalis, Periplaneta americana, Blattelagermanica and Supella spp..

Of the subclass Acaria (Acarida) and the orders Meta- and Meso-stigmata, for example Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp. and Varroa spp..

Of the orders Actinedida (Prostigmata) and Acaridida (Astigmata), for example Acarapis spp., Cheyletiella spp., Ornithocheyletia spp., Myobia spp., Psorergatesspp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. and Laminosioptes spp..

The compositions according to the invention are also suitable for protecting against insect infestation in the case of materials such as wood, textiles, plastics, adhesives, glues, paints, paper and card, leather, floor coverings and buildings.

The compositions according to the invention can be used, for example, against the following pests: beetles such as Hylotrupes bajulus, Chlorophorus pilosis, Anobium punctatum, Xestobium rufovillosum, Ptilinuspecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthesrugicollis, Xyleborus spec.,Tryptodendron spec., Apate monachus, Bostrychus capucins, Heterobostrychus brunneus, Sinoxylon spec. and Dinoderus minutus, and also hymenopterans such as Sirexjuvencus, Urocerus gigas, Urocerus gigas taignus and Urocerus augur, and termites such as Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis and Coptotermes formosanus, and bristletails such as Lepisma saccharina.

In one aspect, the invention therefore also relates to pesticidal compositions such as emulsifiable concentrates, suspension concentrates, microemulsions, oil dispersibles, directly sprayable or dilutable solutions, spreadable pastes, dilute emulsions, soluble powders, dispersible powders, wettable powders, dusts, granules or encapsulations in polymeric substances, which comprise - at least - one of the active ingredients according to embodiment 1 and which are to be selected to suit the intended aims and the prevailing circumstances. In these compositions, the active ingredient is employed in pure form, a solid active ingredient for example in a specific particle size, or, preferably, together with - at least - one of the auxiliaries conventionally used in the art of formulation, such as extenders, for example solvents or solid carriers, or such as surface-active compounds (surfactants).

Examples of suitable solvents are: unhydrogenated or partially hydrogenated aromatic hydrocarbons, preferably the fractions C₈ to C₁₂ of alkylbenzenes, such as xylene mixtures, alkylated naphthalenes or tetrahydronaphthalene, aliphatic or cycloaliphatic hydrocarbons, such as paraffins or cyclohexane, alcohols such as ethanol, propanol or butanol, glycols and their ethers and esters such as propylene glycol, dipropylene glycol ether, ethylene glycol or ethylene glycol monomethyl ether or ethylene glycol monoethyl ether, ketones, such as cyclohexanone, isophorone or diacetone alcohol, strongly polar solvents, such as N-methylpyrrolid-2-one, dimethyl sulfoxide or N,N-dimethylformamide, water, unepoxidized or epoxidized vegetable oils, such as unexpodized or epoxidized rapeseed, castor, coconut or soya oil, and silicone oils.
Solid carriers which are used for example for dusts and dispersible powders are, as a rule, ground natural minerals such as calcite, talc, kaolin, montmorillonite or attapulgite. To improve the physical properties, it is also possible to add highly disperse silicas or highly disperse absorbtive polymers. Suitable adsorptive carriers for granules are porous types, such as pumice, brick grit, sepiolite or bentonite, and suitable non-sorptive carrier materials are calcite or sand. In addition, a large number of granulated materials of inorganic or organic nature can be used, in particular dolomite or comminuted plant residues.

Suitable surface-active compounds are, depending on the type of the active ingredient to be formulated, non-ionic, cationic and/or anionic surfactants or surfactant mixtures which have good emulsifying, dispersing and wetting properties. The surfactants mentioned below are only to be considered as examples; a large number of further surfactants which are conventionally used in the art of formulation and suitable according to the invention are described in the relevant literature.
Suitable non-ionic surfactants are, especially, polyglycol ether derivatives of aliphatic or cycloaliphatic alcohols, of saturated or unsaturated fatty acids or of alkyl phenols which may contain approximately 3 to approximately 30 glycol ether groups and approximately 8 to approximately 20 carbon atoms in the (cyclo)aliphatic hydrocarbon radical or approximately 6 to approximately 18 carbon atoms in the alkyl moiety of the alkyl phenols. Also suitable are water-soluble polyethylene oxide adducts with polypropylene glycol, ethylenediaminopolypropylene glycol or alkyl polypropylene glycol having 1 to approximately 10 carbon atoms in the alkyl chain and approximately 20 to approximately 250 ethylene glycol ether groups and approximately 10 to approximately 100 propylene glycol ether groups. Normally, the abovementioned compounds contain 1 to approximately 5 ethylene glycol units per propylene glycol unit. Examples which may be mentioned are nonylphenoxypolyethoxyethanol, castor oil polyglycol ether, polypropylene glycol/polyethylene oxide adducts, tributylphenoxypolyethoxyethanol, polyethylene glycol or octylphenoxypolyethoxyethanol. Also suitable are fatty acid esters of polyoxyethylene sorbitan, such as polyoxyethylene sorbitan trioleate.
The cationic surfactants are, especially, quarternary ammonium salts which generally have at least one alkyl radical of approximately 8 to approximately 22 C atoms as substituents and as further substituents (unhalogenated or halogenated) lower alkyl or hydroxyalkyl or benzyl radicals. The salts are preferably in the form of halides, methylsulfates or ethylsulfates. Examples are stearyltrimethylammonium chloride and benzylbis(2-chloroethyl)ethylammonium bromide.
Examples of suitable anionic surfactants are water-soluble soaps or water-soluble synthetic surface-active compounds. Examples of suitable soaps are the alkali, alkaline earth or (unsubstituted or substituted) ammonium salts of fatty acids having approximately 10 to approximately 22 C atoms, such as the sodium or potassium salts of oleic or stearic acid, or of natural fatty acid mixtures which are obtainable for example from coconut or tall oil; mention must also be made of the fatty acid methyl taurates. However, synthetic surfactants are used more frequently, in particular fatty sulfonates, fatty sulfates, sulfonated benzimidazole derivatives or alkylaryl sulfonates. As a rule, the fatty sulfonates and fatty sulfates are present as alkali, alkaline earth or (substituted or unsubstituted) ammonium salts and they generally have an alkyl radical of approximately 8 to approximately 22 C atoms, alkyl also to be understood as including the alkyl moiety of acyl radicals; examples which may be mentioned are the sodium or calcium salts of lignosulfonic acid, of the dodecylsulfuric ester or of a fatty alcohol sulfate mixture prepared from natural fatty acids. This group also includes the salts of the sulfuric esters and sulfonic acids of fatty alcohol/ethylene oxide adducts. The sulfonated benzimidazole derivatives preferably contain 2 sulfonyl groups and a fatty acid radical of approximately 8 to approximately 22 C atoms. Examples of alkylarylsulfonates are the sodium, calcium or triethanolammonium salts of decylbenzenesulfonic acid, of dibutylnaphthalenesulfonic acid or of a naphthalenesulfonic acid/formaldehyde condensate. Also possible are, furthermore, suitable phosphates, such as salts of the phosphoric ester of a p-nonylphenol/(4-14)ethylene oxide adduct, or phospholipids.
As a rule, the compositions comprise 0.1 to 99%, especially 0.1 to 95%, of active ingredient and 1 to 99.9%, especially 5 to 99.9%, of at least one solid or liquid adjuvant, it being possible as a rule for 0 to 25%, especially 0.1 to 20%, of the composition to be surfactants(% in each case meaning percent by weight). Whereas concentrated compositions tend to be preferred for commercial goods, the end consumer as a rule uses dilute compositions which have substantially lower concentrations of active ingredient.

Typically, a pre-mix formulation for foliar application comprises 0.1 to 99.9 %, especially 1 to 95 %, of the desired ingredients, and 99.9 to 0.1 %, especially 99 to 5 %, of a solid or liquid adjuvant (including, for example, a solvent such as water), where the auxiliaries can be a surfactant in an amount of 0 to 50 %, especially 0.5 to 40 %, based on the pre-mix formulation.
Normally, a tank-mix formulation for seed treatment application comprises 0.25 to 80%, especially 1 to 75 %, of the desired ingredients, and 99.75 to 20 %, especially 99 to 25 %, of a solid or liquid auxiliaries (including, for example, a solvent such as water), where the auxiliaries can be a surfactant in an amount of 0 to 40 %, especially 0.5 to 30 %, based on the tank-mix formulation.
Typically, a pre-mix formulation for seed treatment application comprises 0.5 to 99.9 %, especially 1 to 95 %, of the desired ingredients, and 99.5 to 0.1 %, especially 99 to 5 %, of a solid or liquid adjuvant (including, for example, a solvent such as water), where the auxiliaries can be a surfactant in an amount of 0 to 50 %, especially 0.5 to 40 %, based on the pre-mix formulation.
Whereas commercial products will preferably be formulated as concentrates (e.g., pre-mix composition (formulation)), the end user will normally employ dilute formulations (e.g., tank mix composition).

Preferred seed treatment pre-mix formulations are aqueous suspension concentrates. The formulation can be applied to the seeds using conventional treating techniques and machines, such as fluidized bed techniques, the roller mill method, rotostatic seed treaters, and drum coaters. Other methods, such as spouted beds may also be useful. The seeds may be presized before coating. After coating, the seeds are typically dried and then transferred to a sizing machine for sizing. Such procedures are known in the art.
In general, the pre-mix compositions of the invention contain 0.5 to 99.9 especially 1 to 95, advantageously 1 to 50 %, by mass of the desired ingredients, and 99.5 to 0.1, especially 99 to 5 %, by mass of a solid or liquid adjuvant (including, for example, a solvent such as water), where the auxiliaries (or adjuvant) can be a surfactant in an amount of 0 to 50, especially 0.5 to 40 %, by mass based on the mass of the pre-mix formulation.

Examples of foliar formulation types for pre-mix compositions are:
GR: Granules
WP: wettable powders
WG: water dispersable granules (powders)
SG: water soluble granules
SL: soluble concentrates
EC: emulsifiable concentrate
EW: emulsions, oil in water
ME: micro-emulsion
SC: aqueous suspension concentrate
CS: aqueous capsule suspension
OD: oil-based suspension concentrate, and
SE: aqueous suspo-emulsion.
Whereas, examples of seed treatment formulation types for pre-mix compositions are:
WS: wettable powders for seed treatment slurry
LS: solution for seed treatment
ES: emulsions for seed treatment
FS: suspension concentrate for seed treatment
WG: water dispersible granules, and
CS: aqueous capsule suspension.
Examples of formulation types suitable for tank-mix compositions are solutions, dilute emulsions, suspensions, or a mixture thereof, and dusts.

Preferred compositions are composed in particular as follows (% = percent by weight):

### Emulsifiable concentrates:

| | |
|---|---|
| active ingredient: | 1 to 95%, preferably 5 to 20% |
| surfactant: | 1 to 30%, preferably 10 to 20 % |
| solvent: | 5 to 98%, preferably 70 to 85% |

### Dusts:

| | |
|---|---|
| active ingredient: | 0.1 to 10%, preferably 0.1 to 1% |
| solid carrier: | 99.9 to 90%, preferably 99.9 to 99% |

### Suspension concentrates:

| | |
|---|---|
| active ingredient: | 5 to 75%, preferably 10 to 50% |
| water: | 94 to 24%, preferably 88 to 30% |
| surfactant: | 1 to 40%, preferably 2 to 30% |

### Wettable powders:

| | |
|---|---|
| active ingredient: | 0.5 to 90%, preferably 1 to 80% |
| surfactant: | 0.5 to 20%, preferably 1 to 15% |
| solid carrier: | 5 to 99%, preferably 15 to 98% |

### Granulates:

| | |
|---|---|
| active ingredient: | 0.5 to 30%, preferably 3 to 15% |
| solid carrier: | 99.5 to 70%, preferably 97 to 85% |

The activity of the compositions according to the invention can be broadened considerably, and adapted to prevailing circumstances, by adding other insecticidally, acaricidally and/or fungicidally active ingredients. The mixtures of the compounds according to embodiment 1 with other insecticidally, acaricidally and/or fungicidally active ingredients may also have further surprising advantages which can also be described, in a wider sense, as synergistic activity. For example, better tolerance by plants, reduced phytotoxicity, insects can be controlled in their different development stages or better behaviour during their production, for example during grinding or mixing, during their storage or during their use.
Suitable additions to active ingredients here are, for example, representatives of the following classes of active ingredients: organophosphorus compounds, nitrophenol derivatives, thioureas, juvenile hormones, formamidines, benzophenone derivatives, ureas, pyrrole derivatives, carbamates, pyrethroids, chlorinated hydrocarbons, acylureas, pyridylmethyleneamino derivatives, macrolides, neonicotinoids and Bacillus thuringiensis preparations.

The following mixtures of the compounds according to embodiment 1 with active ingredients are preferred (the abbreviation "TX" means "one compound selected from the compounds according to embodiment 1):
an adjuvant selected from the group of substances consisting of petroleum oils (alternative name) (628) + TX,
an acaricide selected from the group of substances consisting of 1,1-bis(4-chlorophenyl)-2-ethoxyethanol (IUPAC name) (910) + TX, 2,4-dichlorophenyl benzenesulfonate (lUPAC/Chemical Abstracts name) (1059) + TX, 2-fluoro-*N*-methyl-*N*-1-naphthylacetamide (IUPAC name) (1295) + TX, 4-chlorophenyl phenyl sulfone (IUPAC name) (981) + TX, abamectin (1) + TX, acequinocyl (3) + TX, acetoprole [CCN] + TX, acrinathrin (9) + TX, aldicarb (16) + TX, aldoxycarb (863) + TX, alpha-cypermethrin (202) + TX, amidithion (870) + TX, amidoflumet [CCN] + TX, amidothioate (872) + TX, amiton (875) + TX, amiton hydrogen oxalate (875) + TX, amitraz (24) + TX, aramite (881) + TX, arsenous oxide (882) + TX, AVI 382 (compound code) + TX, AZ 60541 (compound code) + TX, azinphos-ethyl (44) + TX, azinphos-methyl (45) + TX, azobenzene (IUPAC name) (888) + TX, azocyclotin (46) + TX, azothoate (889) + TX, benomyl (62) + TX, benoxafos (alternative name) [CCN] + TX, benzoximate (71) + TX, benzyl benzoate (IUPAC name) [CCN] + TX, bifenazate (74) + TX, bifenthrin (76) + TX, binapacryl (907) + TX, brofenvalerate (alternative name) + TX, bromo-cyclen (918) + TX, bromophos (920) + TX, bromophos-ethyl (921) + TX, bromopropylate (94) + TX, buprofezin (99) + TX, butocarboxim (103) + TX, butoxycarboxim (104) + TX, butylpyridaben (alternative name) + TX, calcium polysulfide (IUPAC name) (111) + TX, camphechlor (941) + TX, carbanolate (943) + TX, carbaryl (115) + TX, carbofuran (118) + TX, carbophenothion (947) + TX, CGA 50'439 (development code) (125) + TX, chinomethionat (126) + TX, chlorbenside (959) + TX, chlordimeform (964) + TX, chlordimeform hydrochloride (964) + TX, chlorfenapyr (130) + TX, chlorfenethol (968) + TX, chlorfenson (970) + TX, chlorfensulfide (971) + TX, chlorfenvinphos (131) + TX, chlorobenzilate (975) + TX, chloromebuform (977) + TX, chloromethiuron (978) + TX, chloropropylate (983) + TX, chlorpyrifos (145) + TX, chlorpyrifos-methyl (146) + TX, chlorthiophos (994) + TX, cinerin I (696) + TX, cinerin II (696) + TX, cinerins (696) + TX, clofentezine (158) + TX, closantel (alternative name) [CCN] + TX, coumaphos (174) + TX, crotamiton (alternative name) [CCN] + TX, crotoxyphos (1010) + TX, cufraneb (1013) + TX, cyanthoate (1020) + TX, cyflumetofen (CAS Reg. No.: 400882-07-7) + TX, cyhalothrin (196) + TX, cyhexatin (199) + TX, cypermethrin (201) + TX, DCPM (1032) + TX, DDT (219) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulfon (1039) + TX, diafenthiuron (226) + TX, dialifos (1042) + TX, diazinon (227) + TX, dichlofluanid (230) + TX, dichlorvos (236) + TX, dicliphos (alternative name) + TX, dicofol (242) + TX, dicrotophos (243) + TX, dienochlor (1071) + TX, dimefox (1081) + TX, dimethoate (262) + TX, dinactin (alternative name) (653) + TX, dinex (1089) + TX, dinex-diclexine (1089) + TX, dinobuton (269) + TX, dinocap (270) + TX, dinocap-4 [CCN] + TX, dinocap-6 [CCN] + TX, dinocton (1090) + TX, dinopenton (1092) + TX, dinosulfon (1097) + TX, dinoterbon (1098) + TX, dioxathion (1102) + TX, diphenyl sulfone (IUPAC name) (1103) + TX, disulfiram (alternative name) [CCN] + TX, disulfoton (278) + TX, DNOC (282) + TX, dofenapyn (1113) + TX, doramectin (alternative name) [CCN] + TX, endosulfan (294) + TX, endothion (1121) + TX, EPN (297) + TX, eprinomectin (alternative name) [CCN] + TX, ethion (309) + TX, ethoate-methyl (1134) + TX, etoxazole (320) + TX, etrimfos (1142) + TX, fenazaflor (1147) + TX, fenazaquin (328) + TX, fenbutatin oxide (330) + TX, fenothiocarb (337) + TX, fenpropathrin (342) + TX, fenpyrad (alternative name) + TX, fenpyroximate (345) + TX, fenson (1157) + TX, fentrifanil (1161) + TX, fenvalerate (349) + TX, fipronil (354) + TX, fluacrypyrim (360) + TX, fluazuron (1166) + TX, flubenzimine (1167) + TX, flucycloxuron (366) + TX, flucythrinate (367) + TX, fluenetil (1169) + TX, flufenoxuron (370) + TX, flumethrin (372) + TX, fluorbenside (1174) + TX, fluvalinate (1184) + TX, FMC 1137 (development code) (1185) + TX, formetanate (405) + TX, formetanate hydrochloride (405) + TX, formothion (1192) + TX, formparanate (1193) + TX, gamma-HCH (430) + TX, glyodin (1205) + TX, halfenprox (424) + TX, heptenophos (432) + TX, hexadecyl cyclopropanecarboxylate (IUPAC/Chemical Abstracts name) (1216) + TX, hexythiazox (441) + TX, iodomethane (IUPAC name) (542) + TX, isocarbophos (alternative name) (473) + TX, isopropyl *O*-(methoxyaminothiophosphoryl)salicylate (IUPAC name) (473) + TX, ivermectin (alternative name) [CCN] + TX, jasmolin I (696) + TX, jasmolin II (696) + TX, jodfenphos (1248) + TX, lindane (430) + TX, lufenuron (490) + TX, malathion (492) + TX, malonoben (1254) + TX, mecarbam (502) + TX, mephosfolan (1261) + TX, mesulfen (alternative name) [CCN] + TX, methacrifos (1266) + TX, methamidophos (527) + TX, methidathion (529) + TX, methiocarb (530) + TX, methomyl (531) + TX, methyl bromide (537) + TX, metolcarb (550) + TX, mevinphos (556) + TX, mexacarbate (1290) + TX, milbemectin (557) + TX, milbemycin oxime (alternative name) [CCN] + TX, mipafox (1293) + TX, monocrotophos (561) + TX, morphothion (1300) + TX, moxidectin (alternative name) [CCN] + TX, naled (567) + TX, NC-184 (compound code) + TX, NC-512 (compound code) + TX, nifluridide (1309) + TX, nikkomycins (alternative name) [CCN] + TX, nitrilacarb (1313) + TX, nitrilacarb 1:1 zinc chloride complex (1313) + TX, NNI-0101 (compound code) + TX, NNI-0250 (compound code) + TX, omethoate (594) + TX, oxamyl (602) + TX, oxydeprofos (1324) + TX, oxydisulfoton (1325) + TX, pp'-DDT (219) + TX, parathion (615) + TX, permethrin (626) + TX, petroleum oils (alternative name) (628) + TX, phenkapton (1330) + TX, phenthoate (631) + TX, phorate (636) + TX, phosalone (637) + TX, phosfolan (1338) + TX, phosmet (638) + TX, phosphamidon (639) + TX, phoxim (642) + TX, pirimiphos-methyl (652) + TX, polychloroterpenes (traditional name) (1347) + TX, polynactins (alternative name) (653) + TX, proclonol (1350) + TX, profenofos (662) + TX, promacyl (1354) + TX, propargite (671) + TX, propetamphos (673) + TX, propoxur (678) + TX, prothidathion (1360) + TX, prothoate (1362) + TX, pyrethrin I (696) + TX, pyrethrin II (696) + TX, pyrethrins (696) + TX, pyridaben (699) + TX, pyridaphenthion (701) + TX, pyrimidifen (706) + TX, pyrimitate (1370) + TX, quinalphos (711) + TX, quintiofos (1381) + TX, R-1492 (development code) (1382) + TX, RA-17 (development code) (1383) + TX, rotenone (722) + TX, schradan (1389) + TX, sebufos (alternative name) + TX, selamectin (alternative name) [CCN] + TX, SI-0009 (compound code) + TX, sophamide (1402) + TX, spirodiclofen (738) + TX, spiromesifen (739) + TX, SSI-121 (development code) (1404) + TX, sulfiram (alternative name) [CCN] + TX, sulfluramid (750) + TX, sulfotep (753) + TX, sulfur (754) + TX, SZI-121 (development code) (757) + TX, tau-fluvalinate (398) + TX, tebufenpyrad (763) + TX, TEPP (1417) + TX, terbam (alternative name) + TX, tetrachlorvinphos (777) + TX, tetradifon (786) + TX, tetranactin (alternative name) (653) + TX, tetrasul (1425) + TX, thiafenox (alternative name) + TX, thiocarboxime (1431) + TX, thiofanox (800) + TX, thiometon (801) + TX, thioquinox (1436) + TX, thuringiensin (alternative name) [CCN] + TX, triamiphos (1441) + TX, triarathene (1443) + TX, triazophos (820) + TX, triazuron (alternative name) + TX, trichlorfon (824) + TX, trifenofos (1455) + TX, trinactin (alternative name) (653) + TX, vamidothion (847) + TX, vaniliprole [CCN] and YI-5302 (compound code) + TX,
an algicide selected from the group of substances consisting of bethoxazin [CCN] + TX, copper dioctanoate (IUPAC name) (170) + TX, copper sulfate (172) + TX, cybutryne [CCN] + TX, dichlone (1052) + TX, dichlorophen (232) + TX, endothal (295) + TX, fentin (347) + TX, hydrated lime [CCN] + TX, nabam (566) + TX, quinoclamine (714) + TX, quinonamid (1379) + TX, simazine (730) + TX, triphenyltin acetate (IUPAC name) (347) and triphenyltin hydroxide (IUPAC name) (347) + TX,
an anthelmintic selected from the group of substances consisting of abamectin (1) + TX, crufomate (1011) + TX, doramectin (alternative name) [CCN] + TX, emamectin (291) + TX, emamectin benzoate (291) + TX, eprinomectin (alternative name) [CCN] + TX, ivermectin (alternative name) [CCN] + TX, milbemycin oxime (alternative name) [CCN] + TX, moxidectin (alternative name) [CCN] + TX, piperazine [CCN] + TX, selamectin (alternative name) [CCN] + TX, spinosad (737) and thiophanate (1435) + TX,
an avicide selected from the group of substances consisting of chloralose (127) + TX, endrin (1122) + TX, fenthion (346) + TX, pyridin-4-amine (IUPAC name) (23) and strychnine (745) + TX,
a bactericide selected from the group of substances consisting of 1-hydroxy-1H-pyridine-2-thione (IUPAC name) (1222) + TX, 4-(quinoxalin-2-ylamino)benzenesulfonamide (IUPAC name) (748) + TX, 8-hydroxyquinoline sulfate (446) + TX, bronopol (97) + TX, copper dioctanoate (IUPAC name) (170) + TX, copper hydroxide (IUPAC name) (169) + TX, cresol [CCN] + TX, dichlorophen (232) + TX, dipyrithione (1105) + TX, dodicin (1112) + TX, fenaminosulf (1144) + TX, formaldehyde (404) + TX, hydrargaphen (alternative name) [CCN] + TX, kasugamycin (483) + TX, kasugamycin hydrochloride hydrate (483) + TX, nickel bis(dimethyldithiocarbamate) (IUPAC name) (1308) + TX, nitrapyrin (580) + TX, octhilinone (590) + TX, oxolinic acid (606) + TX, oxytetracycline (611) + TX, potassium hydroxyquinoline sulfate (446) + TX, probenazole (658) + TX, streptomycin (744) + TX, streptomycin sesquisulfate (744) + TX, tecloftalam (766) + TX, and thiomersal (alternative name) [CCN] + TX,
a biological agent selected from the group of substances consisting of *Adoxophyes orana* GV (alternative name) (12) + TX, *Agrobacterium radiobacter* (alternative name) (13) + TX, *Amblyseius* spp. (alternative name) (19) + TX, *Anagrapha falcifera* NPV (alternative name) (28) + TX, *Anagrus atomus* (alternative name) (29) + TX, *Aphelinus abdominalis* (alternative name) (33) + TX, *Aphidius colemani* (alternative name) (34) + TX, *Aphidoletes aphidimyza* (alternative name) (35) + TX, *Autographa californica* NPV (alternative name) (38) + TX, *Bacillus firmus* (alternative name) (48) + TX, *Bacillus sphaericus* Neide (scientific name) (49) + TX, *Bacillus thuringiensis* Berliner (scientific name) (51) + TX, *Bacillus thuringiensis* subsp. *aizawai* (scientific name) (51) + TX, *Bacillus thuringiensis* subsp. *israelensis* (scientific name) (51) + TX, *Bacillus thuringiensis* subsp. *japonensis* (scientific name) (51) + TX, *Bacillus thuringiensis* subsp. *kurstaki* (scientific name) (51) + TX, *Bacillus thuringiensis* subsp. *tenebrionis* (scientific name) (51) + TX, *Beauveria bassiana* (alternative name) (53) + TX, *Beauveria brongniartii* (alternative name) (54) + TX, *Chrysoperla carnea* (alternative name) (151) + TX, *Cryptolaemus montrouzieri* (alternative name) (178) + TX, *Cydia pomonella* GV (alternative name) (191) + TX, *Dacnusa sibirica* (alternative name) (212) + TX, *Diglyphus isaea* (alternative name) (254) + TX, *Encarsia formosa* (scientific name) (293) + TX, *Eretmocerus eremicus* (alternative name) (300) + TX, *Helicoverpa zea* NPV (alternative name) (431) + TX, *Heterorhabditis bacteriophora* and *H. megidis* (alternative name) (433) + TX, *Hippodamia convergens* (alternative name) (442) + TX, *Leptomastix dactylopii* (alternative name) (488) + TX, *Macrolophus caliginosus* (alternative name) (491) + TX, *Mamestra brassicae* NPV (alternative name) (494) + TX, *Metaphycus helvolus* (alternative name) (522) + TX, *Metarhizium anisopliae* var. *acridum* (scientific name) (523) + TX, *Metarhizium anisopliae* var. *anisopliae* (scientific name) (523) + TX, *Neodiprion sertifer* NPV and *N. lecontei* NPV (alternative name) (575) + TX, *Orius* spp. (alternative name) (596) + TX, *Paecilomyces fumosoroseus* (alternative name) (613) + TX, *Phytoseiulus persimilis* (alternative name) (644) + TX, *Spodoptera exigua* multicapsid nuclear polyhedrosis virus (scientific name) (741) + TX, *Steinernema bibionis* (alternative name) (742) + TX, *Steinernema carpocapsae* (alternative name) (742) + TX, *Steinernema feltiae* (alternative name) (742) + TX, *Steinernema glaseri* (alternative name) (742) + TX, *Steinernema riobrave* (alternative name) (742) + TX, *Steinernema riobravis* (alternative name) (742) + TX, *Steinernema scapterisci* (alternative name) (742) + TX, *Steinernema* spp. (alternative name) (742) + TX, *Trichogramma* spp. (alternative name) (826) + TX, *Typhlodromus occidentalis* (alternative name) (844) and *Verticillium lecanii* (alternative name) (848) + TX,
a soil sterilant selected from the group of substances consisting of iodomethane (IUPAC name) (542) and methyl bromide (537) + TX,
a chemosterilant selected from the group of substances consisting of apholate [CCN] + TX, bisazir (alternative name) [CCN] + TX, busulfan (alternative name) [CCN] + TX, diflubenzuron (250) + TX, dimatif (alternative name) [CCN] + TX, hemel [CCN] + TX, hempa [CCN] + TX, metepa [CCN] + TX, methiotepa [CCN] + TX, methyl apholate [CCN] + TX, morzid [CCN] + TX, penfluron (alternative name) [CCN] + TX, tepa [CCN] + TX, thiohempa (alternative name) [CCN] + TX, thiotepa (alternative name) [CCN] + TX, tretamine (alternative name) [CCN] and uredepa (alternative name) [CCN] + TX,
an insect pheromone selected from the group of substances consisting of (*E*)-dec-5-en-1-yl acetate with (*E*)-dec-5-en-1-ol (IUPAC name) (222) + TX, (*E*)-tridec-4-en-1-yl acetate (IUPAC name) (829) + TX, (*E*)-6-methylhept-2-en-4-ol (IUPAC name) (541) + TX, (*E,Z*)-tetradeca-4,10-dien-1-yl acetate (IUPAC name) (779) + TX, (Z)-dodec-7-en-1-yl acetate (IUPAC name) (285) + TX, (Z)-hexadec-11-enal (IUPAC name) (436) + TX, (Z)-hexadec-11-en-1-yl acetate (IUPAC name) (437) + TX, (Z)-hexadec-13-en-11-yn-1-yl acetate (IUPAC name) (438) + TX, (Z)-icos-13-en-10-one (IUPAC name) (448) + TX, (Z)-tetradec-7-en-1-al (IUPAC name) (782) + TX, (*Z*)-tetradec-9-en-1-ol (IUPAC name) (783) + TX, (Z)-tetradec-9-en-1-yl acetate (IUPAC name) (784) + TX, (7*E*,9*Z*)-dodeca-7,9-dien-1-yl acetate (IUPAC name) (283) + TX, (9*Z*,11*E*)-tetradeca-9,11-dien-1-yl acetate (IUPAC name) (780) + TX, (9*Z*,12*E*)-tetradeca-9,12-dien-1-yl acetate (IUPAC name) (781) + TX, 14-methyloctadec-1-ene (IUPAC name) (545) + TX, 4-methylnonan-5-ol with 4-methylnonan-5-one (IUPAC name) (544) + TX, alpha-multistriatin (alternative name) [CCN] + TX, brevicomin (alternative name) [CCN] + TX, codlelure (alternative name) [CCN] + TX, codlemone (alternative name) (167) + TX, cuelure (alternative name) (179) + TX, disparlure (277) + TX, dodec-8-en-1-yl acetate (IUPAC name) (286) + TX, dodec-9-en-1-yl acetate (IUPAC name) (287) + TX, dodeca-8 + TX, 10-dien-1-yl acetate (IUPAC name) (284) + TX, dominicalure (alternative name) [CCN] + TX, ethyl 4-methyloctanoate (IUPAC name) (317) + TX, eugenol (alternative name) [CCN] + TX, frontalin (alternative name) [CCN] + TX, gossyplure (alternative name) (420) + TX, grandlure (421) + TX, grandlure I (alternative name) (421) + TX, grandlure II (alternative name) (421) + TX, grandlure III (alternative name) (421) + TX, grandlure IV (alternative name) (421) + TX, hexalure [CCN] + TX, ipsdienol (alternative name) [CCN] + TX, ipsenol (alternative name) [CCN] + TX, japonilure (alternative name) (481) + TX, lineatin (alternative name) [CCN] + TX, litlure (alternative name) [CCN] + TX, looplure (alternative name) [CCN] + TX, medlure [CCN] + TX, megatomoic acid (alternative name) [CCN] + TX, methyl eugenol (alternative name) (540) + TX, muscalure (563) + TX, octadeca-2,13-dien-1-yl acetate (IUPAC name) (588) + TX, octadeca-3,13-dien-1-yl acetate (IUPAC name) (589) + TX, orfralure (alternative name) [CCN] + TX, oryctalure (alternative name) (317) + TX, ostramone (alternative name) [CCN] + TX, siglure [CCN] + TX, sordidin (alternative name) (736) + TX, sulcatol (alternative name) [CCN] + TX, tetradec-11-en-1-yl acetate (IUPAC name) (785) + TX, trimedlure (839) + TX, trimedlure A (alternative name) (839) + TX, trimedlure B₁ (alternative name) (839) + TX, trimedlure B₂ (alternative name) (839) + TX, trimedlure C (alternative name) (839) and trunc-call (alternative name) [CCN] + TX,
an insect repellent selected from the group of substances consisting of 2-(octylthio)ethanol (IUPAC name) (591) + TX, butopyronoxyl (933) + TX, butoxy(polypropylene glycol) (936) + TX, dibutyl adipate (IUPAC name) (1046) + TX, dibutyl phthalate (1047) + TX, dibutyl succinate (IUPAC name) (1048) + TX, diethyltoluamide [CCN] + TX, dimethyl carbate [CCN] + TX, dimethyl phthalate [CCN] + TX, ethyl hexanediol (1137) + TX, hexamide [CCN] + TX, methoquin-butyl (1276) + TX, methylneodecanamide [CCN] + TX, oxamate [CCN] and picaridin [CCN] + TX,
an insecticide selected from the group of substances consisting of 1-dichloro-1-nitroethane (IUPAC/Chemical Abstracts name) (1058) + TX, 1,1-dichloro-2,2-bis(4-ethylphenyl)ethane (IUPAC name) (1056), + TX, 1,2-dichloropropane (IUPAC/Chemical Abstracts name) (1062) + TX, 1,2-dichloropropane with 1,3-dichloropropene (IUPAC name) (1063) + TX, 1-bromo-2-chloroethane (IUPAC/Chemical Abstracts name) (916) + TX, 2,2,2-trichloro-1-(3,4-dichlorophenyl)ethyl acetate (IUPAC name) (1451) + TX, 2,2-dichlorovinyl 2-ethylsulfinylethyl methyl phosphate (IUPAC name) (1066) + TX, 2-(1,3-dithiolan-2-yl)phenyl dimethylcarbamate (IUPAC/ Chemical Abstracts name) (1109) + TX, 2-(2-butoxyethoxy)ethyl thiocyanate (IUPAC/Chemical Abstracts name) (935) + TX, 2-(4,5-dimethyl-1,3-dioxolan-2-yl)phenyl methylcarbamate (IUPAC/ Chemical Abstracts name) (1084) + TX, 2-(4-chloro-3,5-xylyloxy)ethanol (IUPAC name) (986) + TX, 2-chlorovinyl diethyl phosphate (IUPAC name) (984) + TX, 2-imidazolidone (IUPAC name) (1225) + TX, 2-isovalerylindan-1,3-dione (IUPAC name) (1246) + TX, 2-methyl(prop-2-ynyl)aminophenyl methylcarbamate (IUPAC name) (1284) + TX, 2-thiocyanatoethyl laurate (IUPAC name) (1433) + TX, 3-bromo-1-chloroprop-1-ene (IUPAC name) (917) + TX, 3-methyl-1-phenylpyrazol-5-yl dimethylcarbamate (IUPAC name) (1283) + TX, 4-methyl(prop-2-ynyl)amino-3,5-xylyl methylcarbamate (IUPAC name) (1285) + TX, 5,5-dimethyl-3-oxocyclohex-1-enyl dimethylcarbamate (IUPAC name) (1085) + TX, abamectin (1) + TX, acephate (2) + TX, acetamiprid (4) + TX, acethion (alternative name) [CCN] + TX, acetoprole [CCN] + TX, acrinathrin (9) + TX, acrylonitrile (IUPAC name) (861) + TX, alanycarb (15) + TX, aldicarb (16) + TX, aldoxycarb (863) + TX, aldrin (864) + TX, allethrin (17) + TX, allosamidin (alternative name) [CCN] + TX, allyxycarb (866) + TX, alpha-cypermethrin (202) + TX, alpha-ecdysone (alternative name) [CCN] + TX, aluminium phosphide (640) + TX, amidithion (870) + TX, amidothioate (872) + TX, aminocarb (873) + TX, amiton (875) + TX, amiton hydrogen oxalate (875) + TX, amitraz (24) + TX, anabasine (877) + TX, athidathion (883) + TX, AVI 382 (compound code) + TX, AZ 60541 (compound code) + TX, azadirachtin (alternative name) (41) + TX, azamethiphos (42) + TX, azinphos-ethyl (44) + TX, azinphos-methyl (45) + TX, azothoate (889) + TX, *Bacillus thuringiensis* delta endotoxins (alternative name) (52) + TX, barium hexafluorosilicate (alternative name) [CCN] + TX, barium polysulfide (IUPAC/Chemical Abstracts name) (892) + TX, barthrin [CCN] + TX, Bayer 22/190 (development code) (893) + TX, Bayer 22408 (development code) (894) + TX, bendiocarb (58) + TX, benfuracarb (60) + TX, bensultap (66) + TX, beta-cyfluthrin (194) + TX, beta-cypermethrin (203) + TX, bifenthrin (76) + TX, bioallethrin (78) + TX, bioallethrin S-cyclopentenyl isomer (alternative name) (79) + TX, bioethanomethrin [CCN] + TX, biopermethrin (908) + TX, bioresmethrin (80) + TX, bis(2-chloroethyl) ether (IUPAC name) (909) + TX, bistrifluron (83) + TX, borax (86) + TX, brofenvalerate (alternative name) + TX, bromfenvinfos (914) + TX, bromocyclen (918) + TX, bromo-DDT (alternative name) [CCN] + TX, bromophos (920) + TX, bromophos-ethyl (921) + TX, bufencarb (924) + TX, buprofezin (99) + TX, butacarb (926) + TX, butathiofos (927) + TX, butocarboxim (103) + TX, butonate (932) + TX, butoxycarboxim (104) + TX, butylpyridaben (alternative name) + TX, cadusafos (109) + TX, calcium arsenate [CCN] + TX, calcium cyanide (444) + TX, calcium polysulfide (IUPAC name) (111) + TX, camphechlor (941) + TX, carbanolate (943) + TX, carbaryl (115) + TX, carbofuran (118) + TX, carbon disulfide (IUPAC/Chemical Abstracts name) (945) + TX, carbon tetrachloride (IUPAC name) (946) + TX, carbophenothion (947) + TX, carbosulfan (119) + TX, cartap (123) + TX, cartap hydrochloride (123) + TX, cevadine (alternative name) (725) + TX, chlorbicyclen (960) + TX, chlordane (128) + TX, chlordecone (963) + TX, chlordimeform (964) + TX, chlordimeform hydrochloride (964) + TX, chlorethoxyfos (129) + TX, chlorfenapyr (130) + TX, chlorfenvinphos (131) + TX, chlorfluazuron (132) + TX, chlormephos (136) + TX, chloroform [CCN] + TX, chloropicrin (141) + TX, chlorphoxim (989) + TX, chlorprazophos (990) + TX, chlorpyrifos (145) + TX, chlorpyrifos-methyl (146) + TX, chlorthiophos (994) + TX, chromafenozide (150) + TX, cinerin I (696) + TX, cinerin II (696) + TX, cinerins (696) + TX, cis-resmethrin (alternative name) + TX, cismethrin (80) + TX, clocythrin (alternative name) + TX, cloethocarb (999) + TX, closantel (alternative name) [CCN] + TX, clothianidin (165) + TX, copper acetoarsenite [CCN] + TX, copper arsenate [CCN] + TX, copper oleate [CCN] + TX, coumaphos (174) + TX, coumithoate (1006) + TX, crotamiton (alternative name) [CCN] + TX, crotoxyphos (1010) + TX, crufomate (1011) + TX, cryolite (alternative name) (177) + TX, CS 708 (development code) (1012) + TX, cyanofenphos (1019) + TX, cyanophos (184) + TX, cyanthoate (1020) + TX, cyclethrin [CCN] + TX, cycloprothrin (188) + TX, cyfluthrin (193) + TX, cyhalothrin (196) + TX, cypermethrin (201) + TX, cyphenothrin (206) + TX, cyromazine (209) + TX, cythioate (alternative name) [CCN] + TX, d-limonene (alternative name) [CCN] + TX, *d*-tetramethrin (alternative name) (788) + TX, DAEP (1031) + TX, dazomet (216) + TX, DDT (219) + TX, decarbofuran (1034) + TX, deltamethrin (223) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulphon (1039) + TX, diafenthiuron (226) + TX, dialifos (1042) + TX, diamidafos (1044) + TX, diazinon (227) + TX, dicapthon (1050) + TX, dichlofenthion (1051) + TX, dichlorvos (236) + TX, dicliphos (alternative name) + TX, dicresyl (alternative name) [CCN] + TX, dicrotophos (243) + TX, dicyclanil (244) + TX, dieldrin (1070) + TX, diethyl 5-methylpyrazol-3-yl phosphate (IUPAC name) (1076) + TX, diflubenzuron (250) + TX, dilor (alternative name) [CCN] + TX, dimefluthrin [CCN] + TX, dimefox (1081) + TX, dimetan (1085) + TX, dimethoate (262) + TX, dimethrin (1083) + TX, dimethylvinphos (265) + TX, dimetilan (1086) + TX, dinex (1089) + TX, dinex-diclexine (1089) + TX, dinoprop (1093) + TX, dinosam (1094) + TX, dinoseb (1095) + TX, dinotefuran (271) + TX, diofenolan (1099) + TX, dioxabenzofos (1100) + TX, dioxacarb (1101) + TX, dioxathion (1102) + TX, disulfoton (278) + TX, dithicrofos (1108) + TX, DNOC (282) + TX, doramectin (alternative name) [CCN] + TX, DSP (1115) + TX, ecdysterone (alternative name) [CCN] + TX, EI 1642 (development code) (1118) + TX, emamectin (291) + TX, emamectin benzoate (291) + TX, EMPC (1120) + TX, empenthrin (292) + TX, endosulfan (294) + TX, endothion (1121) + TX, endrin (1122) + TX, EPBP (1123) + TX, EPN (297) + TX, epofenonane (1124) + TX, eprinomectin (alternative name) [CCN] + TX, esfenvalerate (302) + TX, etaphos (alternative name) [CCN] + TX, ethiofencarb (308) + TX, ethion (309) + TX, ethiprole (310) + TX, ethoate-methyl (1134) + TX, ethoprophos (312) + TX, ethyl formate (IUPAC name) [CCN] + TX, ethyl-DDD (alternative name) (1056) + TX, ethylene dibromide (316) + TX, ethylene dichloride (chemical name) (1136) + TX, ethylene oxide [CCN] + TX, etofenprox (319) + TX, etrimfos (1142) + TX, EXD (1143) + TX, famphur (323) + TX, fenamiphos (326) + TX, fenazaflor (1147) + TX, fenchlorphos (1148) + TX, fenethacarb (1149) + TX, fenfluthrin (1150) + TX, fenitrothion (335) + TX, fenobucarb (336) + TX, fenoxacrim (1153) + TX, fenoxycarb (340) + TX, fenpirithrin (1155) + TX, fenpropathrin (342) + TX, fenpyrad (alternative name) + TX, fensulfothion (1158) + TX, fenthion (346) + TX, fenthion-ethyl [CCN] + TX, fenvalerate (349) + TX, fipronil (354) + TX, flonicamid (358) + TX, flubendiamide (CAS. Reg. No.: 272451-65-7) + TX, flucofuron (1168) + TX, flucycloxuron (366) + TX, flucythrinate (367) + TX, fluenetil (1169) + TX, flufenerim [CCN] + TX, flufenoxuron (370) + TX, flufenprox (1171) + TX, flumethrin (372) + TX, fluvalinate (1184) + TX, FMC 1137 (development code) (1185) + TX, fonofos (1191) + TX, formetanate (405) + TX, formetanate hydrochloride (405) + TX, formothion (1192) + TX, formparanate (1193) + TX, fosmethilan (1194) + TX, fospirate (1195) + TX, fosthiazate (408) + TX, fosthietan (1196) + TX, furathiocarb (412) + TX, furethrin (1200) + TX, gamma-cyhalothrin (197) + TX, gamma-HCH (430) + TX, guazatine (422) + TX, guazatine acetates (422) + TX, GY-81 (development code) (423) + TX, halfenprox (424) + TX, halofenozide (425) + TX, HCH (430) + TX, HEOD (1070) + TX, heptachlor (1211) + TX, heptenophos (432) + TX, heterophos [CCN] + TX, hexaflumuron (439) + TX, HHDN (864) + TX, hydramethylnon (443) + TX, hydrogen cyanide (444) + TX, hydroprene (445) + TX, hyquincarb (1223) + TX, imidacloprid (458) + TX, imiprothrin (460) + TX, indoxacarb (465) + TX, iodomethane (IUPAC name) (542) + TX, IPSP (1229) + TX, isazofos (1231) + TX, isobenzan (1232) + TX, isocarbophos (alternative name) (473) + TX, isodrin (1235) + TX, isofenphos (1236) + TX, isolane (1237) + TX, isoprocarb (472) + TX, isopropyl O-(methoxy-aminothiophosphoryl)salicylate (IUPAC name) (473) + TX, isoprothiolane (474) + TX, isothioate (1244) + TX, isoxathion (480) + TX, ivermectin (alternative name) [CCN] + TX, jasmolin I (696) + TX, jasmolin II (696) + TX, jodfenphos (1248) + TX, juvenile hormone I (alternative name) [CCN] + TX, juvenile hormone II (alternative name) [CCN] + TX, juvenile hormone III (alternative name) [CCN] + TX, kelevan (1249) + TX, kinoprene (484) + TX, lambda-cyhalothrin (198) + TX, lead arsenate [CCN] + TX, lepimectin (CCN) + TX, leptophos (1250) + TX, lindane (430) + TX, lirimfos (1251) + TX, lufenuron (490) + TX, lythidathion (1253) + TX, m-cumenyl methylcarbamate (IUPAC name) (1014) + TX, magnesium phosphide (IUPAC name) (640) + TX, malathion (492) + TX, malonoben (1254) + TX, mazidox (1255) + TX, mecarbam (502) + TX, mecarphon (1258) + TX, menazon (1260) + TX, mephosfolan (1261) + TX, mercurous chloride (513) + TX, mesulfenfos (1263) + TX, metaflumizone (CCN) + TX, metam (519) + TX, metam-potassium (alternative name) (519) + TX, metam-sodium (519) + TX, methacrifos (1266) + TX, methamidophos (527) + TX, methanesulfonyl fluoride (IUPAC/Chemical Abstracts name) (1268) + TX, methidathion (529) + TX, methiocarb (530) + TX, methocrotophos (1273) + TX, methomyl (531) + TX, methoprene (532) + TX, methoquin-butyl (1276) + TX, methothrin (alternative name) (533) + TX, methoxychlor (534) + TX, methoxyfenozide (535) + TX, methyl bromide (537) + TX, methyl isothiocyanate (543) + TX, methylchloroform (alternative name) [CCN] + TX, methylene chloride [CCN] + TX, metofluthrin [CCN] + TX, metolcarb (550) + TX, metoxadiazone (1288) + TX, mevinphos (556) + TX, mexacarbate (1290) + TX, milbemectin (557) + TX, milbemycin oxime (alternative name) [CCN] + TX, mipafox (1293) + TX, mirex (1294) + TX, monocrotophos (561) + TX, morphothion (1300) + TX, moxidectin (alternative name) [CCN] + TX, naftalofos (alternative name) [CCN] + TX, naled (567) + TX, naphthalene (IUPAC/Chemical Abstracts name) (1303) + TX, NC-170 (development code) (1306) + TX, NC-184 (compound code) + TX, nicotine (578) + TX, nicotine sulfate (578) + TX, nifluridide (1309) + TX, nitenpyram (579) + TX, nithiazine (1311) + TX, nitrilacarb (1313) + TX, nitrilacarb 1:1 zinc chloride complex (1313) + TX, NNI-0101 (compound code) + TX, NNI-0250 (compound code) + TX, nornicotine (traditional name) (1319) + TX, novaluron (585) + TX, noviflumuron (586) + TX, *O*-5-dichloro-4-iodophenyl O-ethyl ethylphosphonothioate (IUPAC name) (1057) + TX, *O,O*-diethyl *O*-4-methyl-2-oxo-2*H*-chromen-7-yl phosphorothioate (IUPAC name) (1074) + TX, *O,O*-diethyl *O*-6-methyl-2-propylpyrimidin-4-yl phosphorothioate (IUPAC name) (1075) + TX, *O,O,O',O'*-tetrapropyl dithiopyrophosphate (IUPAC name) (1424) + TX, oleic acid (IUPAC name) (593) + TX, omethoate (594) + TX, oxamyl (602) + TX, oxydemeton-methyl (609) + TX, oxydeprofos (1324) + TX, oxydisulfoton (1325) + TX, pp'-DDT (219) + TX, para-dichlorobenzene [CCN] + TX, parathion (615) + TX, parathion-methyl (616) + TX, penfluron (alternative name) [CCN] + TX, pentachlorophenol (623) + TX, pentachlorophenyl laurate (IUPAC name) (623) + TX, permethrin (626) + TX, petroleum oils (alternative name) (628) + TX, PH 60-38 (development code) (1328) + TX, phenkapton (1330) + TX, phenothrin (630) + TX, phenthoate (631) + TX, phorate (636) + TX, phosalone (637) + TX, phosfolan (1338) + TX, phosmet (638) + TX, phosnichlor (1339) + TX, phosphamidon (639) + TX, phosphine (IUPAC name) (640) + TX, phoxim (642) + TX, phoxim-methyl (1340) + TX, pirimetaphos (1344) + TX, pirimicarb (651) + TX, pirimiphos-ethyl (1345) + TX, pirimiphos-methyl (652) + TX, polychlorodicyclopentadiene isomers (IUPAC name) (1346) + TX, polychloroterpenes (traditional name) (1347) + TX, potassium arsenite [CCN] + TX, potassium thiocyanate [CCN] + TX, prallethrin (655) + TX, precocene I (alternative name) [CCN] + TX, precocene II (alternative name) [CCN] + TX, precocene III (alternative name) [CCN] + TX, primidophos (1349) + TX, profenofos (662) + TX, profluthrin [CCN] + TX, promacyl (1354) + TX, promecarb (1355) + TX, propaphos (1356) + TX, propetamphos (673) + TX, propoxur (678) + TX, prothidathion (1360) + TX, prothiofos (686) + TX, prothoate (1362) + TX, protrifenbute [CCN] + TX, pymetrozine (688) + TX, pyraclofos (689) + TX, pyrazophos (693) + TX, pyresmethrin (1367) + TX, pyrethrin I (696) + TX, pyrethrin II (696) + TX, pyrethrins (696) + TX, pyridaben (699) + TX, pyridalyl (700) + TX, pyridaphenthion (701) + TX, pyrimidifen (706) + TX, pyrimitate (1370) + TX, pyriproxyfen (708) + TX, quassia (alternative name) [CCN] + TX, quinalphos (711) + TX, quinalphos-methyl (1376) + TX, quinothion (1380) + TX, quintiofos (1381) + TX, R-1492 (development code) (1382) + TX, rafoxanide (alternative name) [CCN] + TX, resmethrin (719) + TX, rotenone (722) + TX, RU 15525 (development code) (723) + TX, RU 25475 (development code) (1386) + TX, ryania (alternative name) (1387) + TX, ryanodine (traditional name) (1387) + TX, sabadilla (alternative name) (725) + TX, schradan (1389) + TX, sebufos (alternative name) + TX, selamectin (alternative name) [CCN] + TX, SI-0009 (compound code) + TX, SI-0205 (compound code) + TX, SI-0404 (compound code) + TX, SI-0405 (compound code) + TX, silafluofen (728) + TX, SN 72129 (development code) (1397) + TX, sodium arsenite [CCN] + TX, sodium cyanide (444) + TX, sodium fluoride (IUPAC/Chemical Abstracts name) (1399) + TX, sodium hexafluorosilicate (1400) + TX, sodium pentachlorophenoxide (623) + TX, sodium selenate (IUPAC name) (1401) + TX, sodium thiocyanate [CCN] + TX, sophamide (1402) + TX, spinosad (737) + TX, spiromesifen (739) + TX, spirotetrmat (CCN) + TX, sulcofuron (746) + TX, sulcofuron-sodium (746) + TX, sulfluramid (750) + TX, sulfotep (753) + TX, sulfuryl fluoride (756) + TX, sulprofos (1408) + TX, tar oils (alternative name) (758) + TX, tau-fluvalinate (398) + TX, tazimcarb (1412) + TX, TDE (1414) + TX, tebufenozide (762) + TX, tebufenpyrad (763) + TX, tebupirimfos (764) + TX, teflubenzuron (768) + TX, tefluthrin (769) + TX, temephos (770) + TX, TEPP (1417) + TX, terallethrin (1418) + TX, terbam (alternative name) + TX, terbufos (773) + TX, tetrachloroethane [CCN] + TX, tetrachlorvinphos (777) + TX, tetramethrin (787) + TX, theta-cypermethrin (204) + TX, thiacloprid (791) + TX, thiafenox (alternative name) + TX, thiamethoxam (792) + TX, thicrofos (1428) + TX, thiocarboxime (1431) + TX, thiocyclam (798) + TX, thiocyclam hydrogen oxalate (798) + TX, thiodicarb (799) + TX, thiofanox (800) + TX, thiometon (801) + TX, thionazin (1434) + TX, thiosultap (803) + TX, thiosultap-sodium (803) + TX, thuringiensin (alternative name) [CCN] + TX, tolfenpyrad (809) + TX, tralomethrin (812) + TX, transfluthrin (813) + TX, transpermethrin (1440) + TX, triamiphos (1441) + TX, triazamate (818) + TX, triazophos (820) + TX, triazuron (alternative name) + TX, trichlorfon (824) + TX, trichlormetaphos-3 (alternative name) [CCN] + TX, trichloronat (1452) + TX, trifenofos (1455) + TX, triflumuron (835) + TX, trimethacarb (840) + TX, triprene (1459) + TX, vamidothion (847) + TX, vaniliprole [CCN] + TX, veratridine (alternative name) (725) + TX, veratrine (alternative name) (725) + TX, XMC (853) + TX, xylylcarb (854) + TX, YI-5302 (compound code) + TX, zeta-cypermethrin (205) + TX, zetamethrin (alternative name) + TX, zinc phosphide (640) + TX, zolaprofos (1469) and ZXI 8901 (development code) (858) + TX, cyantraniliprole [736994-63-19 + TX, chlorantraniliprole [500008-45-7] + TX, cyenopyrafen [560121-52-0] + TX, cyflumetofen [400882-07-7] + TX, pyrifluquinazon [337458-27-2] + TX, spinetoram [187166-40-1 + 187166-15-0] + TX, spirotetramat [203313-25-1] + TX, sulfoxaflor [946578-00-3] + TX, flufiprole [704886-18-0] + TX, meperfluthrin [915288-13-0] + TX, tetramethylfluthrin [84937-88-2] + TX, triflumezopyrim (disclosed in WO 2012/092115) + TX,
a molluscicide selected from the group of substances consisting of bis(tributyltin) oxide (IUPAC name) (913) + TX, bromoacetamide [CCN] + TX, calcium arsenate [CCN] + TX, cloethocarb (999) + TX, copper acetoarsenite [CCN] + TX, copper sulfate (172) + TX, fentin (347) + TX, ferric phosphate (IUPAC name) (352) + TX, metaldehyde (518) + TX, methiocarb (530) + TX, niclosamide (576) + TX, niclosamide-olamine (576) + TX, pentachlorophenol (623) + TX, sodium pentachlorophenoxide (623) + TX, tazimcarb (1412) + TX, thiodicarb (799) + TX, tributyltin oxide (913) + TX, trifenmorph (1454) + TX, trimethacarb (840) + TX, triphenyltin acetate (IUPAC name) (347) and triphenyltin hydroxide (IUPAC name) (347) + TX, pyriprole [394730-71-3] + TX, a nematicide selected from the group of substances consisting of AKD-3088 (compound code) + TX, 1,2-dibromo-3-chloropropane (IUPAC/Chemical Abstracts name) (1045) + TX, 1,2-dichloropropane (IUPAC/ Chemical Abstracts name) (1062) + TX, 1,2-dichloropropane with 1,3-dichloropropene (IUPAC name) (1063) + TX, 1,3-dichloropropene (233) + TX, 3,4-dichlorotetrahydrothiophene 1,1-dioxide (IUPAC/Chemical Abstracts name) (1065) + TX, 3-(4-chlorophenyl)-5-methylrhodanine (IUPAC name) (980) + TX, 5-methyl-6-thioxo-1,3,5-thiadiazinan-3-ylacetic acid (IUPAC name) (1286) + TX, 6-isopentenylaminopurine (alternative name) (210) + TX, abamectin (1) + TX, acetoprole [CCN] + TX, alanycarb (15) + TX, aldicarb (16) + TX, aldoxycarb (863) + TX, AZ 60541 (compound code) + TX, benclothiaz [CCN] + TX, benomyl (62) + TX, butylpyridaben (alternative name) + TX, cadusafos (109) + TX, carbofuran (118) + TX, carbon disulfide (945) + TX, carbosulfan (119) + TX, chloropicrin (141) + TX, chlorpyrifos (145) + TX, cloethocarb (999) + TX, cytokinins (alternative name) (210) + TX, dazomet (216) + TX, DBCP (1045) + TX, DCIP (218) + TX, diamidafos (1044) + TX, dichlofenthion (1051) + TX, dicliphos (alternative name) + TX, dimethoate (262) + TX, doramectin (alternative name) [CCN] + TX, emamectin (291) + TX, emamectin benzoate (291) + TX, eprinomectin (alternative name) [CCN] + TX, ethoprophos (312) + TX, ethylene dibromide (316) + TX, fenamiphos (326) + TX, fenpyrad (alternative name) + TX, fensulfothion (1158) + TX, fosthiazate (408) + TX, fosthietan (1196) + TX, furfural (alternative name) [CCN] + TX, GY-81 (development code) (423) + TX, heterophos [CCN] + TX, iodomethane (IUPAC name) (542) + TX, isamidofos (1230) + TX, isazofos (1231) + TX, ivermectin (alternative name) [CCN] + TX, kinetin (alternative name) (210) + TX, mecarphon (1258) + TX, metam (519) + TX, metam-potassium (alternative name) (519) + TX, metam-sodium (519) + TX, methyl bromide (537) + TX, methyl isothiocyanate (543) + TX, milbemycin oxime (alternative name) [CCN] + TX, moxidectin (alternative name) [CCN] + TX, *Myrothecium verrucaria* composition (alternative name) (565) + TX, NC-184 (compound code) + TX, oxamyl (602) + TX, phorate (636) + TX, phosphamidon (639) + TX, phosphocarb [CCN] + TX, sebufos (alternative name) + TX, selamectin (alternative name) [CCN] + TX, spinosad (737) + TX, terbam (alternative name) + TX, terbufos (773) + TX, tetrachlorothiophene (IUPAC/ Chemical Abstracts name) (1422) + TX, thiafenox (alternative name) + TX, thionazin (1434) + TX, triazophos (820) + TX, triazuron (alternative name) + TX, xylenols [CCN] + TX, YI-5302 (compound code) and zeatin (alternative name) (210) + TX, fluensulfone [318290-98-1] + TX,
a nitrification inhibitor selected from the group of substances consisting of potassium ethylxanthate [CCN] and nitrapyrin (580) + TX,
a plant activator selected from the group of substances consisting of acibenzolar (6) + TX, acibenzolar-S-methyl (6) + TX, probenazole (658) and *Reynoutria sachalinensis* extract (alternative name) (720) + TX,
a rodenticide selected from the group of substances consisting of 2-isovalerylindan-1,3-dione (IUPAC name) (1246) + TX, 4-(quinoxalin-2-ylamino)benzenesulfonamide (IUPAC name) (748) + TX, alpha-chlorohydrin [CCN] + TX, aluminium phosphide (640) + TX, antu (880) + TX, arsenous oxide (882) + TX, barium carbonate (891) + TX, bisthiosemi (912) + TX, brodifacoum (89) + TX, bromadiolone (91) + TX, bromethalin (92) + TX, calcium cyanide (444) + TX, chloralose (127) + TX, chlorophacinone (140) + TX, cholecalciferol (alternative name) (850) + TX, coumachlor (1004) + TX, coumafuryl (1005) + TX, coumatetralyl (175) + TX, crimidine (1009) + TX, difenacoum (246) + TX, difethialone (249) + TX, diphacinone (273) + TX, ergocalciferol (301) + TX, flocoumafen (357) + TX, fluoroacetamide (379) + TX, flupropadine (1183) + TX, flupropadine hydrochloride (1183) + TX, gamma-HCH (430) + TX, HCH (430) + TX, hydrogen cyanide (444) + TX, iodomethane (IUPAC name) (542) + TX, lindane (430) + TX, magnesium phosphide (IUPAC name) (640) + TX, methyl bromide (537) + TX, norbormide (1318) + TX, phosacetim (1336) + TX, phosphine (IUPAC name) (640) + TX, phosphorus [CCN] + TX, pindone (1341) + TX, potassium arsenite [CCN] + TX, pyrinuron (1371) + TX, scilliroside (1390) + TX, sodium arsenite [CCN] + TX, sodium cyanide (444) + TX, sodium fluoroacetate (735) + TX, strychnine (745) + TX, thallium sulfate [CCN] + TX, warfarin (851) and zinc phosphide (640) + TX,
a synergist selected from the group of substances consisting of 2-(2-butoxyethoxy)ethyl piperonylate (IUPAC name) (934) + TX, 5-(1,3-benzodioxol-5-yl)-3-hexylcyclohex-2-enone (IUPAC name) (903) + TX, farnesol with nerolidol (alternative name) (324) + TX, MB-599 (development code) (498) + TX, MGK 264 (development code) (296) + TX, piperonyl butoxide (649) + TX, piprotal (1343) + TX, propyl isomer (1358) + TX, S421 (development code) (724) + TX, sesamex (1393) + TX, sesasmolin (1394) and sulfoxide (1406) + TX,
an animal repellent selected from the group of substances consisting of anthraquinone (32) + TX, chloralose (127) + TX, copper naphthenate [CCN] + TX, copper oxychloride (171) + TX, diazinon (227) + TX, dicyclopentadiene (chemical name) (1069) + TX, guazatine (422) + TX, guazatine acetates (422) + TX, methiocarb (530) + TX, pyridin-4-amine (IUPAC name) (23) + TX, thiram (804) + TX, trimethacarb (840) + TX, zinc naphthenate [CCN] and ziram (856) + TX,
a virucide selected from the group of substances consisting of imanin (alternative name) [CCN] and ribavirin (alternative name) [CCN] + TX,
a wound protectant selected from the group of substances consisting of mercuric oxide (512) + TX, octhilinone (590) and thiophanate-methyl (802) + TX,
and biologically active compounds selected from the group consisting of azaconazole (60207-31-0] + TX, bitertanol [70585-36-3] + TX, bromuconazole [116255-48-2] + TX, cyproconazole [94361-06-5] + TX, difenoconazole [119446-68-3] + TX, diniconazole [83657-24-3] + TX, epoxiconazole [106325-08-0] + TX, fenbuconazole [114369-43-6] + TX, fluquinconazole [136426-54-5] + TX, flusilazole [85509-19-9] + TX, flutriafol [76674-21-0] + TX, hexaconazole [79983-71-4] + TX, imazalil [35554-44-0] + TX, imibenconazole [86598-92-7] + TX, ipconazole [125225-28-7] + TX, metconazole [125116-23-6] + TX, myclobutanil [88671-89-0] + TX, pefurazoate [101903-30-4] + TX, penconazole [66246-88-6] + TX, prothioconazole [178928-70-6] + TX, pyrifenox [88283-41-4] + TX, prochloraz [67747-09-5] + TX, propiconazole [60207-90-1] + TX, simeconazole [149508-90-7] + TX, tebuconazole [107534-96-3] + TX, tetraconazole [112281-77-3] + TX, triadimefon [43121-43-3] + TX, triadimenol [55219-65-3] + TX, triflumizole [99387-89-0] + TX, triticonazole [131983-72-7] + TX, ancymidol [12771-68-5] + TX, fenarimol [60168-88-9] + TX, nuarimol [63284-71-9] + TX, bupirimate [41483-43-6] + TX, dimethirimol [5221-53-4] + TX, ethirimol [23947-60-6] + TX, dodemorph [1593-77-7] + TX, fenpropidine [67306-00-7] + TX, fenpropimorph [67564-91-4] + TX, spiroxamine [118134-30-8] + TX, tridemorph [81412-43-3] + TX, cyprodinil [121552-61-2] + TX, mepanipyrim [110235-47-7] + TX, pyrimethanil [53112-28-0] + TX, fenpiclonil [74738-17-3] + TX, fludioxonil [131341-86-1] + TX, benalaxyl [71626-11-4] + TX, furalaxyl [57646-30-7] + TX, metalaxyl [57837-19-1] + TX, R-metalaxyl [70630-17-0] + TX, ofurace [58810-48-3] + TX, oxadixyl [77732-09-3] + TX, benomyl [17804-35-2] + TX, carbendazim [10605-21-7] + TX, debacarb [62732-91-6] + TX, fuberidazole [3878-19-1] + TX, thiabendazole [148-79-8] + TX, chlozolinate [84332-86-5] + TX, dichlozoline [24201-58-9] + TX, iprodione [36734-19-7] + TX, myclozoline [54864-61-8] + TX, procymidone [32809-16-8] + TX, vinclozoline [50471-44-8] + TX, boscalid [188425-85-6] + TX, carboxin [5234-68-4] + TX, fenfuram [24691-80-3] + TX, flutolanil [66332-96-5] + TX, mepronil [55814-41-0] + TX, oxycarboxin [5259-88-1] + TX, penthiopyrad [183675-82-3] + TX, thifluzamide [130000-40-7] + TX, guazatine [108173-90-6] + TX, dodine [2439-10-3] [112-65-2] (free base) + TX, iminoctadine [13516-27-3] + TX, azoxystrobin [131860-33-8] + TX, dimoxystrobin [149961-52-4] + TX, enestroburin {Proc. BCPC, Int. Congr., Glasgow, 2003, 1, 93} + TX, fluoxastrobin [361377-29-9] + TX, kresoxim-methyl [143390-89-0] + TX, metominostrobin [133408-50-1] + TX, trifloxystrobin [141517-21-7] + TX, orysastrobin [248593-16-0] + TX, picoxystrobin [117428-22-5] + TX, pyraclostrobin [175013-18-0] + TX, ferbam [14484-64-1] + TX, mancozeb [8018-01-7] + TX, maneb [12427-38-2] + TX, metiram [9006-42-2] + TX, propineb [12071-83-9] + TX, thiram [137-26-8] + TX, zineb [12122-67-7] + TX, ziram [137-30-4] + TX, captafol [2425-06-1] + TX, captan [133-06-2] + TX, dichlofluanid [1085-98-9] + TX, fluoroimide [41205-21-4] + TX, folpet [133-07-3] + TX, tolylfluanid [731-27-1] + TX, bordeaux mixture [8011-63-0] + TX, copperhydroxid [20427-59-2] + TX, copperoxychlorid [1332-40-7] + TX, coppersulfat [7758-98-7] + TX, copperoxid [1317-39-1] + TX, mancopper [53988-93-5] + TX, oxine-copper [10380-28-6] + TX, dinocap [131-72-6] + TX, nitrothal-isopropyl [10552-74-6] + TX, edifenphos [17109-49-8] + TX, iprobenphos [26087-47-8] + TX, isoprothiolane [50512-35-1] + TX, phosdiphen [36519-00-3] + TX, pyrazophos [13457-18-6] + TX, tolclofos-methyl [57018-04-9] + TX, acibenzo-lar-S-methyl [135158-54-2] + TX, anilazine [101-05-3] + TX, benthiavalicarb [413615-35-7] + TX, blasticidin-S [2079-00-7] + TX, chinomethionat [2439-01-2] + TX, chloroneb [2675-77-6] + TX, chlorothalonil *[1897-45-6]* + TX, cyflufenamid *[180409-60-3]* + TX, cymoxanil *[57966-95-7]* + TX, dichlone *[117-80-6]* + TX, diclocymet [139920-32-4] + TX, diclomezine *[62865-36-5]* + TX, dicloran *[99-30-9]* + TX, diethofencarb *[87130-20-9]* + TX, dimethomorph *[110488-70-5]* + TX, SYP-LI90 (Flumorph) *[211867-47-9]* + TX, dithianon *[3347-22-6]* + TX, ethaboxam *[162650-77-3]* + TX, etridiazole *[2593-15-9]* + TX, famoxadone *[131807-57-3]* + *TX,* fenamidone *[161326-34-7]* + TX, fenoxanil *[115852-48-7]* + TX, fentin *[668-34-8]* + TX, ferimzone *[89269-64-7]* + TX, fluazinam *[79622-59-6]* + TX, fluopicolide [239110-15-7] + TX, flusulfamide [106917-52-6] + TX, fenhexamid [126833-17-8] + TX, fosetyl-aluminium [39148-24-8] + TX, hymexazol [10004-44-1] + TX, iprovalicarb [140923-17-7] + TX, IKF-916 (Cyazofamid) *[Y20YY6-88-3]* + TX, kasugamycin [6980-18-3] + TX, methasulfocarb *[66952-49-6]* + TX, metrafenone *[220899-03-6]* + TX, pencycuron *[66063-05-6]* + TX, phthalide [27355-22-2] + TX, polyoxins [11113-80-7] + TX, probenazole [27605-76-1] + TX, propamocarb [25606-41-1] + TX, proquinazid [189278-12-4] + TX, pyroquilon [57369-32-1] + TX, quinoxyfen [124495-18-7] + TX, quintozene [82-68-8] + TX, sulfur [7704-34-9] + TX, tiadinil [223580-51-6] + TX, triazoxide [72459-58-6] + TX, tricyclazole [41814-78-2] + TX, triforine [26644-46-2] + TX, validamycin [37248-47-8] + TX, zoxamide (RH7281) [156052-68-5] + TX, mandipropamid [374726-62-2] + TX, isopyrazam [881685-58-1] + TX, sedaxane [874967-67-6] + TX, 3-difluoromethyl-1-methyl-1H-pyrazole-4-carboxylic acid (9-dichloromethylene-1,2,3,4-tetrahydro-1,4-methano-naphthalen-5-yl)-amide (dislosed in WO 2007/048556) + TX, 3-difluoromethyl-1-methyl-1H-pyrazole-4-carboxylic acid (3',4',5'-trifluoro-biphenyl-2-yl)-amide (disclosed in WO 2006/087343) + TX, [(3*S*,4*R*,4a*R*,6*S*,6a*S*,12*R*,12a*S*,12b*S*)-3-[(cyclopropylcarbonyl)oxy]- 1,3,4,4a,5,6,6a,12,12a,12b-decahydro-6,12-dihydroxy-4,6a,12b-trimethyl-11-oxo-9-(3-pyridinyl)-2*H*,11*H*naphtho[2,1-*b*]pyrano[3,4-*e*]pyran-4-yl]methyl-cyclopropanecarboxylate [915972-17-7] + TX and 1,3,5-trimethyl-N-(2-methyl-1-oxopropyl)-N-[3-(2-methylpropyl)-4-[2,2,2-trifluoro-1-methoxy-1-(trifluoromethyl)ethyl]phenyl]-1H-pyrazole-4-carboxamide [926914-55-8] + TX.

The references in brackets behind the active ingredients, e.g. *[3878-19-1]* refer to the Chemical Abstracts Registry number. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet [A. Wood; Compendium of Pesticide Common Names, Copyright © 1995-2004]; for example, the compound "acetoprole" is described under the internet address http://www.alanwood.net/pesticides/acetoprole.html.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound; in that case, the IUPAC name, the IUPAC/Chemical Abstracts name, a "chemical name", a "traditional name", a "compound name" or a "develoment code" is used or, if neither one of those designations nor a "common name" is used, an "alternative name" is employed. "CAS Reg. No" means the Chemical Abstracts Registry Number.

The active ingredient mixture of the compounds according to any one of embodiments 1 to 24 with active ingredients described above comprises a compound according to any one of embodiments 1 to 24 and an active ingredient as described above preferably in a mixing ratio of from 100:1 to 1:6000, especially from 50:1 to 1:50, more especially in a ratio of from 20:1 to 1:20, even more especially from 10:1 to 1:10, very especially from 5:1 and 1:5, special preference being given to a ratio of from 2:1 to 1:2, and a ratio of from 4:1 to 2:1 being likewise preferred, above all in a ratio of 1:1, or 5:1, or 5:2, or 5:3, or 5:4, or 4:1, or 4:2, or 4:3, or 3:1, or 3:2, or 2:1, or 1:5, or 2:5, or 3:5, or 4:5, or 1:4, or 2:4, or 3:4, or 1:3, or 2:3, or 1:2, or 1:600, or 1:300, or 1:150, or 1:35, or 2:35, or 4:35, or 1:75, or 2:75, or 4:75, or 1:6000, or 1:3000, or 1:1500, or 1:350, or 2:350, or 4:350, or 1:750, or 2:750, or 4:750. Those mixing ratios are by weight.

The mixtures as described above can be used in a method for controlling pests, which comprises applying a composition comprising a mixture as described above to the pests or their environment, with the exception of a method for treatment of the human or animal body by surgery or therapy and diagnostic methods practised on the human or animal body.

The mixtures comprising a compound of according to embodiment 1 and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compounds according to embodiment 1 and the active ingredients as described above is not essential for working the present invention.

The compositions according to the invention can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers or other active ingredients for achieving specific effects, for example bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds I for the preparation of these compositions are also a subject of the invention.

The application methods for the compositions, that is the methods of controlling pests of the abovementioned type, such as spraying, atomizing, dusting, brushing on, dressing, scattering or pouring - which are to be selected to suit the intended aims of the prevailing circumstances - and the use of the compositions for controlling pests of the abovementioned type are other subjects of the invention. Typical rates of concentration are between 0.1 and 1000 ppm, preferably between 0.1 and 500 ppm, of active ingredient. The rate of application per hectare is generally 1 to 2000 g of active ingredient per hectare, in particular 10 to 1000 g/ha, preferably 10 to 600 g/ha.

A preferred method of application in the field of crop protection is application to the foliage of the plants (foliar application), it being possible to select frequency and rate of application to match the danger of infestation with the pest in question. Alternatively, the active ingredient can reach the plants via the root system (systemic action), by drenching the locus of the plants with a liquid composition or by incorporating the active ingredient in solid form into the locus of the plants, for example into the soil, for example in the form of granules (soil application). In the case of paddy rice crops, such granules can be metered into the flooded paddy-field.

The compounds of the invention and compositions thereof are also be suitable for the protection of plant propagation material, for example seeds, such as fruit, tubers or kernels, or nursery plants, against pests of the abovementioned type. The propagation material can be treated with the compound prior to planting, for example seed can be treated prior to sowing. Alternatively, the compound can be applied to seed kernels (coating), either by soaking the kernels in a liquid composition or by applying a layer of a solid composition. It is also possible to apply the compositions when the propagation material is planted to the site of application, for example into the seed furrow during drilling. These treatment methods for plant propagation material and the plant propagation material thus treated are further subjects of the invention. Typical treatment rates would depend on the plant and pest/fungi to be controlled and are generally between 1 to 200 grams per 100 kg of seeds, preferably between 5 to 150 grams per 100 kg of seeds, such as between 10 to 100 grams per 100 kg of seeds.

The term seed embraces seeds and plant propagules of all kinds including but not limited to true seeds, seed pieces, suckers, corns, bulbs, fruit, tubers, grains, rhizomes, cuttings, cut shoots and the like and means in a preferred embodiment true seeds.

The present invention also comprises seeds coated or treated with or containing a compound according to embodiment 1. The term "coated or treated with and/or containing" generally signifies that the active ingredient is for the most part on the surface of the seed at the time of application, although a greater or lesser part of the ingredient may penetrate into the seed material, depending on the method of application. When the said seed product is (re)planted, it may absorb the active ingredient. In an embodiment, the present invention makes available a plant propagation material adhered thereto with according to embodiment 1 . Further, it is hereby made available, a composition comprising a plant propagation material treated with a compound according to embodiment 1.

Seed treatment comprises all suitable seed treatment techniques known in the art, such as seed dressing, seed coating, seed dusting, seed soaking and seed pelleting. The seed treatment application of the compound according to embodiment 1 can be carried out by any known methods, such as spraying or by dusting the seeds before sowing or during the sowing/planting of the seeds.

### Experimental

The following Examples illustrate, but do not limit, the invention.

### Examples:

The following compounds may be prepared according to the methods described herein or according to known methods. "Mp" means melting point in °C. ¹H NMR measurements were recorded on a Brucker 400MHz spectrometer, chemical shifts are given in ppm relevant to a TMS standard. Spectra measured in deuterated solvents as indicated.

### Method LCMS:

Spectra were recorded on a Mass Spectrometer from Waters (SQD or ZQ Single quadrupole mass spectrometer) equipped with an electrospray source (Polarity: positive or negative ions, Capillary: 3.00 kV, Cone range: 30-60 V, Extractor: 2.00 V, Source Temperature: 150°C, Desolvation Temperature: 350°C, Cone Gas Flow: 0 L/Hr, Desolvation Gas Flow: 650 L/Hr, Mass range: 100 to 900 Da) and an Acquity UPLC from Waters: Binary pump, heated column compartment and diode-array detector. Solvent degasser, binary pump, heated column compartment and diode-array detector. Column: Waters UPLC HSS T3, 1.8 µm, 30 x 2.1 mm, Temp: 60 °C, DAD Wavelength range (nm): 210 to 500, Solvent Gradient: A = water + 5% MeOH + 0.05 % HCOOH, B= Acetonitrile + 0.05 % HCOOH: gradient: gradient: 0 min 0% B, 100%A; 1.2-1.5min 100% B; Flow (ml/min) 0.85.

### Example 1.1: Preparation of 2-[1-[[5-(4-chlorophenyl)-2-(2-fluorophenyl)-1,2,4-triazol-3-yl]methyl]-4-piperidyl]pyrimidine

### Step 1:

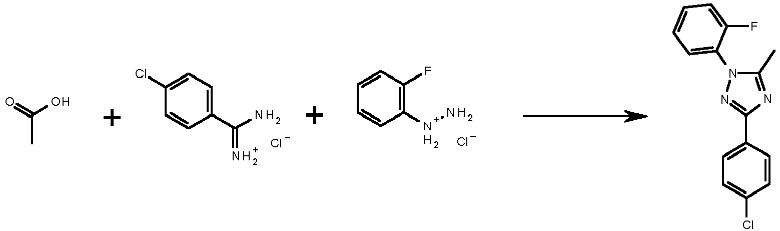

In a round bottom flask equipped with a reflux condenser, 1-[Bis(dimethylamino)methylene]-1H-1,2,3-triazolo[4,5-b]pyridinium 3-oxid hexafluorophosphate (4.88 g) and 4-chlorobenzamidine hydrochloride (2.90 g) were dissolved into a solution of acetic acid (0.66 mL) and DMF (20 mL) after which Hunig's base (4.0 mL) was added and the resulting brown suspension was stirred at room temperature for 3 hours. Subsequently acetic acid (6.66 mL) was added to this mixture followed by (2-fluorophenyl)hydrazine hydrochloride (1.3 g) at room temperature and the reaction mixture was heated to 80°C for 4 hours. The mixture was cooled to ambient temperature then poured into a solution of saturated NaHCO₃. The resulting mixture was extracted twice with ethyl acetate; the combined organic layers were washed with brine, dried over Na₂SO₄ and evaporated under vacuum. The crude material was purified by flash chromatography (SiO₂, gradient: ethyl acetate/cyclohexane) to give 3-(4-chlorophenyl)-1-(2-fluorophenyl)-5-methyl-1,2,4-triazole (0.48 g) as light yellow solid.
¹H-NMR (CDCl₃, δ in ppm): 8.1 (d, 2H), 7.55 (m, 2H), 7.45 (d, 2H), 7.35 (m, 2H), 2.5 (s, 3H).

### Step 2:

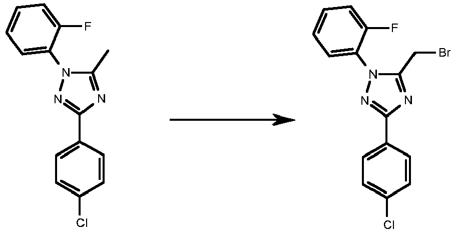

In a round bottom flask equipped with a reflux condenser, 3-(4-chlorophenyl)-1-(2-fluorophenyl)-5-methyl-1,2,4-triazole (0.48 g) was dissolved in carbon tetrachloride (20 mL) then NBS (0.37 g) and 2-[(E)-(1-cyano-1-methyl-ethyl)azo]-2-methyl-propanenitrile (0.04 g) were added and the mixture was heated to 65 °C under light irradiation with a halogen lamp (LED) for 14 hours. Another batch of NBS (0.07 g) was subsequently added and the whole further heated at 65 °C under light irradiation for an additional 14 hours. The mixture was cooled to ambient temperature and poured into water and a saturated solution of sodium thiosulfate was added. The resulting mixture was extracted twice with ethyl acetate; the combined organic layers were washed with brine, dried over Na₂SO₄ and evaporated under vacuum. The crude material was purified by flash chromatography (SiO₂, gradient: ethyl acetate/cyclohexane) to give 5-(bromomethyl)-3-(4-chlorophenyl)-1-(2-fluorophenyl)-1,2,4-triazole (0.20 g) as light yellow solid.
¹H-NMR (CDCl₃, δ in ppm): 8.1 (d, 2H), 7.6 (m, 2H), 7.45 (d, 2H), 7.4 (m, 2H), 4.5 (s, 2H).

### Step 3:

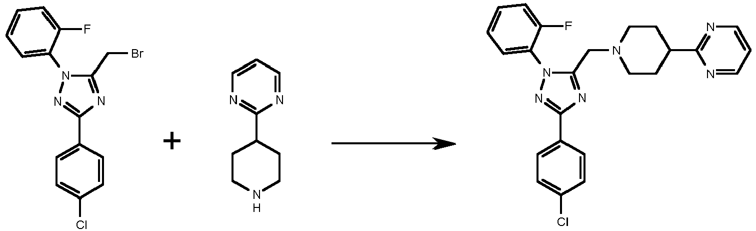

In a round bottom flask 2-(4-piperidyl)pyrimidine hydrochloride (0.089 g) was dissolved in DMF (5 mL) and NaH (0.037 g, 60% in oil) was added in one portion at room temperature upon which a gas evolution was observed. The mixture was stirred for 0.5 hour at room temperature after which 5-(bromomethyl)-3-(4-chlorophenyl)-1-(2-fluorophenyl)-1,2,4-triazole (0.15 g) was added in one portion and the whole was further stirred for 2 hours at ambient temperature. The mixture was poured into water, the resulting solution was extracted twice with ethyl acetate; the combined organic layers were washed with brine, dried over Na₂SO₄ and evaporated under vacuum. The crude material was purified by flash chromatography (SiO₂, gradient: ethyl acetate/cyclohexane) to give 2-[1-[[5-(4-chlorophenyl)-2-(2-fluorophenyl)-1,2,4-triazol-3-yl]methyl]-4-piperidyl]pyrimidine (0.177 g) as light yellow oil.
¹H-NMR (CDCl3, δ in ppm): 8.7 (d, 2H), 8.1 (d, 2H), 7.6 (t, 1H), 7.5 (m, 1H), 7.4 (m, 2H), 7.3 (m, 2H), 7.1 (t, 1H), 3.8 (brs, 2H), 2.7-2.9 (br, 3H), 2.15-2.35 (br, 2H), 1.9-2.0 (br, 2H), 1.6-1.8 (br, 2H).

The following compounds in Table 1 were prepared in a similar manner as illustrated above in Example 1.1 or according to the Schemes provided. The measured [M+1]+ value can deviate from the calculated value by one or two atom units.

**Table 1: The compounds disclosed below in Table 1have formula (la) wherein the substituents Z¹, Z², A and R^{3g} are as defined in the table.**

| | | | | | |
|---|---|---|---|---|---|
| 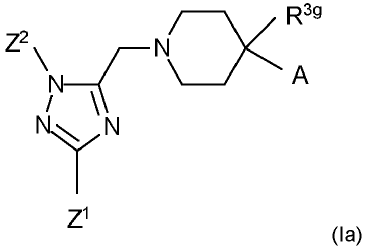 | | | | | |

| Example No. | Z¹ | Z² | A | R^{3g} | Melting point (°C) or [M+1]+ /Rt [min] |
|---|---|---|---|---|---|
| 1.1 | 4-chlorophenyl | 2-fluorophenyl | pyrimidin-2-yl | H | 450.4/ 0.82 |
| 1.2 | 5-chloropyridin-2-yl | 2-fluorophenyl | CF₃ | H | 132-133 °C |
| 1.3 | 5-chloropyridin-2-yl | 2-fluorophenyl | 1,3,4-oxadiazol-2-yl | H | 170-172 °C |
| 1.4 | 5-chloropyridin-2-yl | 2-fluorophenyl | pyrimidin-2-yl | H | 450.4/ 0.70 |
| 1.5 | 4-chlorophenyl | 2-fluorophenyl | CF₃ | H | 92-94 °C |
| 1.6 | 4-chlorophenyl | 2-fluorophenyl | 1,3,4-oxadiazol-2-yl | H | 440.4/ 0.97 |
| 1.7 | 5-chloropyridin-2-yl | 2-fluorophenyl | CN | H | 398.3/ 0.82 |
| 1.8 | 4-chlorophenyl | 2-fluorophenyl | CN | H | 396.3/ 1.03 |
| 1.9 | 4-chlorophenyl | 2-fluorophenyl | CO₂CH₃ | H | 482.2/ 1.14 |
| 1.10 | 4-chlorophenyl | 2-fluorophenyl | CH=N-OCH₃ | H | 429.4/ |
| | | | | | 0.93 |
| 1.11 | 4-chlorophenyl | 2-fluorophenyl | pyrazin-2-yl | H | 450.4/ 0.84 |
| 1.12 | 4-chlorophenyl | 2-fluorophenyl | piperazin-1-yl | H | 450.4/ 0.84 |
| 1.13 | 4-chlorophenyl | 2-fluorophenyl | 5-chloro-pyrimidin-2-yl | H | 136-137 °C |
| 1.14 | 4-chlorophenyl | 2-fluorophenyl | triazol-2-yl | H | 438.5/ 1.02 |
| 1.15 | 4-chlorophenyl | 2-fluorophenyl | triazol-1-yl | H | 166-168 °C |
| 1.16 | 4-chlorophenyl | 2,6-difluorophenyl | pyrimidin-2-yl | H | 117-119 °C |
| 1.17 | 4-chlorophenyl | 2-fluorophenyl | 3-(4-fluorophenyl)-1,2,4-oxadiazol-5-yl | H | 533.7/ 1.24 |
| 1.18 | 4-chlorophenyl | 4-fluorophenyl | pyrimidin-2-yl | H | 152-154 °C |
| 1.19 | 4-chlorophenyl | 2-fluorophenyl | 4-bromothiazol-2-yl | H | 534.2/ 1.11 |
| 1.20 | 3,4-dichlorophenyl | 2-fluorophenyl | pyrimidin-2-yl | H | 485.2/ 0.91 |
| 1.21 | 4-(trifluoromethyl)-phenyl | 2,6-difluorophenyl | pyrimidin-2-yl | H | 501.7/ 0.93 |
| 1.22 | 4-chlorophenyl | 2-fluorophenyl | tetrazol-1-yl | H | 439.6/ 0.98 |
| 1.23 | 4-chlorophenyl | 2,6-dichlorophenyl | pyrimidin-2-yl | H | 181-186 °C |
| 1.24 | 4-chlorophenyl | 2-fluorophenyl | 5-chloro-1 H-benzimidazol-2-yl | H | 106-114 °C |
| 1.25 | 4-chlorophenyl | 2-fluorophenyl | tetrazol-2-yl | H | 440.4/ 1.05 |
| 1.26 | 4-(trifluoromethyl)-phenyl | 2-fluorophenyl | pyrimidin-2-yl | H | 483.6/ 0.88 |
| 1.27 | 4-chlorophenyl | 2-fluorophenyl | 5-(trifluoromethyl)-pyrimidin-2-yl | H | 159-161 °C |
| 1.28 | 4-chlorophenyl | 3,5-(bis-trifluoromethyl)phenyl | pyrimidin-2-yl | H | 184-186 °C |
| 1.29 | 4-fluorophenyl | 4-fluorophenyl | pyrimidin-2-yl | H | 433.7/ 0.77 |
| 1.30 | 4-chlorophenyl | 2,4-difluorophenyl | pyrimidin-2-yl | H | 467.6/ 0.86 |
| 1.31 | 4-chlorophenyl | 2-fluorophenyl | CH₃ | H | 386.4/ 0.85 |
| 1.32 | 4-chlorophenyl | 2-fluorophenyl | 4-(trifluoromethyl)-pyrimidin-2-yl | H | 518.4/ 1.02 |
| 1.33 | 3-chloro-4-fluorophenyl | 2-fluorophenyl | pyrimidin-2-yl | H | 468.3/ |
| | | | | | 0.85 |
| 1.34 | 4-(trifluoromethyl)-phenyl | 4-(trifluoromethyl)-phenyl | pyrimidin-2-yl | H | 164-166 °C |
| 1.35 | 4-chlorophenyl | 2-fluorophenyl | CH₃ | CH₃ | 399.6/ 0.91 |
| 1.36 | 2-methylphenyl | 2-fluorophenyl | pyrimidin-2-yl | H | 429.8/ 0.79 |
| 1.37 | 3-methylphenyl | 2-fluorophenyl | pyrimidin-2-yl | H | 429.7/ 0.82 |
| 1.38 | 3-(trifluoromethyl)-phenyl | 2-fluorophenyl | pyrimidin-2-yl | H | 483.6/ 0.89 |
| 1.39 | 4-chlorophenyl | 2-fluorophenyl I | 4-(trifluoromethyl)-pyrazol-1-yl | H | 505.21/ 1.85 |
| 1.40 | 4-chlorophenyl | 2-fluorophenyl | 5-(2-thienyl)-1 H-pyrazol-3-yl | H | 519.22/ 1.39 |
| 1.41 | 4-chlorophenyl | 2-fluorophenyl | 5-(4-chlorophenyl)-1H-pyrazol-3-yl | H | 547.24/ 1.53 |
| 1.42 | 4-chlorophenyl | 2-fluorophenyl | (4-chlorophenyl)methyl | H | 495.22/ 1.63 |
| 1.43 | 4-chlorophenyl | 2-fluorophenyl | phenyl | H | 447.24/ 1.52 |
| 1.44 | 4-chlorophenyl | 2-fluorophenyl | CO₂-tert-C₄H₉ | H | 486.27/ 1.41 |
| 1.45 | 4-chlorophenyl | 2-fluorophenyl | (phenyl)methyl | H | 461.25/ 1.51 |
| 1.46 | 4-chlorophenyl | 2-fluorophenyl | CH₃ | H | 385.27/ 1.24 |
| 1.47 | 4-chlorophenyl | 2-fluorophenyl | CONH₂ | H | 414.19/ 0.99 |
| 1.48 | 4-chlorophenyl | 2-fluorophenyl | 4-(fluorophenyl)-methanone | H | 493.21/ 1.53 |
| 1.49 | 4-chlorophenyl | 2,3,5,6-tetrafluoro-phenyl | pyrimidin-2-yl | H | 503.2/ 1.47 |
| 1.50 | 4-chlorophenyl | 3,4-dichlorophenyl | pyrimidin-2-yl | H | 499.2/ 1.49 |
| 1.51 | 4-chlorophenyl | 3-methylphenyl | pyrimidin-2-yl | H | 445.3/ 1.21 |
| 1.52 | 4-chlorophenyl | 4-methylphenyl | pyrimidin-2-yl | H | 445.2/ 1.20 |
| 1.53 | 4-chlorophenyl | 3,5-dichlorophenyl | pyrimidin-2-yl | H | 499.2/ 1.61 |
| 1.54 | 4-chlorophenyl | 2,4-difluorophenyl | pyrimidin-2-yl | H | 467.2/ 1.20 |
| 1.55 | 4-chlorophenyl | 2,3-dichlorophenyl | pyrimidin-2-yl | H | 4.99/ 1.38 |
| 1.56 | 4-chlorophenyl | (phenyl)methyl | pyrimidin-2-yl | H | 445.3/ 1.20 |
| 1.57 | 4-chlorophenyl | 3-bromophenyl | pyrimidin-2-yl | H | 509.2/ 1.36 |
| 1.58 | 4-chlorophenyl | cyclobutyl | pyrimidin-2-yl | H | 409.2/ 1.13 |
| 1.59 | 4-chlorophenyl | 6-chloropyridine-6-yl | pyrimidin-2-yl | H | 466.2/ 1.20 |
| 1.60 | 4-chlorophenyl | isopropyl | pyrimidin-2-yl | H | 397.2/ 1.08 |
| 1.61 | 4-chlorophenyl | phenyl | pyrimidin-2-yl | H | 431.2/ 1.12 |
| 1.62 | 4-chlorophenyl | 2-methylphenyl | pyrimidin-2-yl | H | 445.2/ 1.17 |
| 1.63 | 4-chlorophenyl | 4-(trifluoromethoxy)-phenyl | pyrimidin-2-yl | H | 515.2 |
| 1.64 | 4-chlorophenyl | 3-chloro-4-methylphenyl | pyrimidin-2-yl | H | 479.2/ 1.42 |
| 1.65 | 4-chlorophenyl | 2,4-dichlorophenyl | pyrimidin-2-yl | H | 499.2/ 1.38 |
| 1.66 | 4-chlorophenyl | 4-chlorophenyl | pyrimidin-2-yl | H | 465.2/ 1.30 |
| 1.67 | 4-chlorophenyl | 2-chloro-4-(trifluoromethyl)-phenyl | pyrimidin-2-yl | H | 533.2/ 1.51 |
| 1.68 | 4-chlorophenyl | 3-chloro-5-(trifluoromethyl)-pyridin-2-yl | pyrimidin-2-yl | H | 534.2/ 1.44 |
| 1.69 | 4-chlorophenyl | 3-chlorophenyl | pyrimidin-2-yl | H | 465.2/ 1.31 |
| 1.70 | 4-chlorophenyl | 2-methoxyphenyl | pyrimidin-2-yl | H | 461.3/ 1.14 |
| 1.71 | 4-chlorophenyl | 4-sulfonamido phenyl | pyrimidin-2-yl | H | 510.2/ 0.98 |
| 1.72 | 4-chlorophenyl | 6-chloropyridine-2-yl | pyrimidin-2-yl | H | 466.2/ 1.12 |
| 1.73 | 4-chlorophenyl | 3,4-dimethylphenyl | pyrimidin-2-yl | H | 459.3/ 1.29 |
| 1.74 | 4-chlorophenyl | 2,5-dimethylphenyl | pyrimidin-2-yl | H | 459.3/ 1.28 |
| 1.75 | 4-chlorophenyl | 3-methoxyphenyl | pyrimidin-2-yl | H | 461.3/ 1.17 |
| 1.76 | 4-chlorophenyl | 4-bromophenyl | pyrimidin-2-yl | H | 509.2/ 1.30 |
| 1.77 | 4-chlorophenyl | 2,4,6-trichlorophenyl | pyrimidin-2-yl | H | 533.1/ 1.59 |
| 1.78 | 4-chlorophenyl | pyridin-2-yl | pyrimidin-2-yl | H | 432.2/ 1.06 |
| 1.79 | 4-chlorophenyl | 4-methoxyphenyl | pyrimidin-2-yl | H | 461.3/ 1.14 |
| 1.80 | 4-chlorophenyl | 3-fluorophenyl | pyrimidin-2-yl | H | 449.2/ 1.20 |
| 1.81 | 4-chlorophenyl | 3-(trifluoromethyl)-phenyl | pyrimidin-2-yl | H | 499.2/ 1.43 |
| 1.82 | 4-chlorophenyl | 2,3-dimethylphenyl | pyrimidin-2-yl | H | 459.3/ 1.25 |
| 1.83 | 4-chlorophenyl | 2-chlorophenyl | pyrimidin-2-yl | H | 465.2/ 1.20 |
| 1.84 | 4-chlorophenyl | 3-chloro-2-methylphenyl | pyrimidin-2-yl | H | 479.2/ 1.35 |
| 1.85 | 4-chlorophenyl | 4-chloro-2-methylphenyl | pyrimidin-2-yl | H | 479.2/ 1.33 |
| 1.86 | 4-chlorophenyl | 2,5-dichlorophenyl | pyrimidin-2-yl | H | 499.2/ 1.38 |
| 1.87 | 4-chlorophenyl | 2,4-dimethylphenyl | pyrimidin-2-yl | H | 459.3/ 1.26 |
| 1.88 | 4-chlorophenyl | 4-(trifluoromethyl)-phenyl | pyrimidin-2-yl | H | 499.2/ 1.39 |
| 1.89 | 4-chlorophenyl | 3-chloro-pyridin-2-yl | pyrimidin-2-yl | H | 466.2/ 1.14 |
| 1.90 | 4-chlorophenyl | 3,5-dichloro-pyridin-2-yl | pyrimidin-2-yl | H | 500.2/ 1.32 |
| 1.91 | (4-chloro-phenyl)methyl | 2-fluorophenyl | pyrimidin-2-yl | H | 463.3/ 1.11 |
| 1.92 | 3-fluoro-4-methylphenyl | 2-fluorophenyl | pyrimidin-2-yl | H | 447.3/ 1.13 |
| 1.93 | cyclopropyl | 2-fluorophenyl | pyrimidin-2-yl | H | 379.2/ 0.77 |
| 1.94 | phenyl | 2-fluorophenyl | pyrimidin-2-yl | H | 415.3/ |
| | | | | | 0.98 |
| 1.95 | 4-methylphenyl | 2-fluorophenyl | pyrimidin-2-yl | H | 429.3/ 1.07 |
| 1.96 | thiophen-2-yl | 2-fluorophenyl | pyrimidin-2-yl | H | 421.2/ 0.94 |
| 1.97 | 5-(trifluoromethyl)-pyridin-2-yl | 2-fluorophenyl | pyrimidin-2-yl | H | 484.3/ 1.04 |
| 1.98 | pyridin-3-yl | 2-fluorophenyl | pyrimidin-2-yl | H | 416.3/ 0.73 |
| 1.99 | ethyl | 2-fluorophenyl | pyrimidin-2-yl | H | 367.2/ 0.76 |
| 1.100 | isopropyl | 2-fluorophenyl | pyrimidin-2-yl | H | 381.3/ 0.87 |
| 1.101 | 4-methoxyphenyl | 2-fluorophenyl | pyrimidin-2-yl | H | 445.3/ 0.99 |
| 1.102 | pyridin-2-yl | 2-fluorophenyl | pyrimidin-2-yl | H | 416.2/ 0.77 |
| 1.103 | 2-methyl-propyl | 2-fluorophenyl | pyrimidin-2-yl | H | 395.3/ 0.94 |
| 1.104 | cyclohexyl | 2-fluorophenyl | pyrimidin-2-yl | H | 421.3/ 1.09 |
| 1.105 | 2-fluorophenyl | 2-fluorophenyl | pyrimidin-2-yl | H | 395.3/ 0.94 |
| 1.106 | 2-ethyl-propyl | 2-fluorophenyl | pyrimidin-2-yl | H | 409.3/ 1.05 |
| 1.107 | 6-methyl-pyridin-2-yl | 2-fluorophenyl | pyrimidin-2-yl | H | 430.3/ 0.82 |
| 1.108 | 4-chlorophenyl | 2-fluorophenyl | 4-carbonyl-piperidin-4-one | H | 496.38/ 1.11 |
| 1.109 | 4-chlorophenyl | 2-fluorophenyl | 4-(trifluoromethyl)-1H-imdazol-2-yl | H | 505.41/ 1.27 |
| 1.110 | 4-chlorophenyl | 2-fluorophenyl | 2-methyl-pyrimidin-4-yl | H | 463.47/ 1.22 |
| 1.111 | 4-chlorophenyl | 2-fluorophenyl | N-methyl-N-methoxy-carboxamide | H | 458.22/ 1.17 |
| 1.112 | 4-chlorophenyl | 2-fluorophenyl | 1H-imidazol-2-yl | H | 437.21/ 1.01 |
| 1.113 | 4-chlorophenyl | 2-fluorophenyl | 1,2,4-oxadiazol-3-yl | H | 439.45/ 1.27 |
| 1.114 | 4-chlorophenyl | 2-fluorophenyl | N-cyclopropyl-carboxamide | H | 454.47/ 1.14 |
| 1.115 | 4-chlorophenyl | 2-fluorophenyl | thiazol-2-yl | H | 454.33/ 1.37 |
| 1.116 | 4-chlorophenyl | 2-fluorophenyl | pyrimidin-4-yl | H | 449.2/ 1.17 |
| 1.117 | 4-chlorophenyl | 2-fluorophenyl | 1-methylimidazol-2-yl | H | 451.24/ 0.99 |
| 1.118 | 4-chlorophenyl | 2-fluorophenyl | 2-methyl-1,3,4-oxadiazol-5-yl | H | 453.19/ 1.26 |
| 1.119 | 4-chlorophenyl | 2-fluorophenyl | thiazol-4-yl | H | 454.35/ 1.22 |
| 1.120 | 4-chlorophenyl | 2-fluorophenyl | isoxazol-3-yl | H | 438.19/ 1.28 |
| 1.121 | 4-chlorophenyl | 2-fluorophenyl | 1,2,4-oxadiazol-5-yl | H | 439.30/ 1.41 |
| 1.122 | 4-chlorophenyl | 2-fluorophenyl | 5-fluoro-pyridin-2-yl | H | 466.21/ 1.32 |
| 1.123 | 4-chlorophenyl | 2-fluorophenyl | (4-pyridyl)-1,3,4-oxadiazol-2-yl | H | 516.24/ 1.39 |
| 1.124 | 4-chlorophenyl | 2-fluorophenyl | isoxazol-2-yl | H | 438.27/ 1.28 |
| 1.125 | 4-chlorophenyl | 2-fluorophenyl | 4-methylpyrimidin-2-yl | H | 463.57/ 1.20 |
| 1.126 | 4-chlorophenyl | 2-fluorophenyl | 3-chloro-pyridin-2-yl | H | 482.18/ 1.45 |
| 1.127 | 4-chloro-3-fluorophenyl | 2-fluorophenyl | pyrimidin-2-yl | H | 467.6/ 0.88 |

### Biological Examples (% = percent by weight, unless otherwise specified)

### Spodoptera littoralis (Egyptian cotton leaf worm)

Cotton leaf discs were placed on agar in 24-well microtiter plates and sprayed with aqueous test solutions prepared from 10,000 ppm DMSO stock solutions. After drying, the leaf discs were infested with five L1 larvae. The samples were assessed for mortality, anti-feedant effect, and growth inhibition in comparison to untreated samples 3 days after infestation. Control of Spodoptera littoralis by a test sample is when at least one of mortality, anti-feedant effect, and growth inhibition is higher than the untreated sample.

The following compounds resulted in at least 80% control at an application rate of 200 ppm: 1.1, 1.4, 1.6, 1.9, 1.10, 1.11, 1.13, 1.17, 1.18, 1.20, 1.21, 1.26, 1.29, 1.30, 1.31, 1.32, 1.33, 1.35, 1.37, 1.38, 1.43, 5.10, 1.46, 1.47, 1.52, 1.54, 1.55, 1.56, 1.61, 1.62, 1.65, 1.66, 1.67, 1.68, 1.70, 1.72, 1.76, 1.77, 1.78, 1.79, 1.80, 1.81, 1.82, 1.83, 1.84, 1.85, 1.87, 1.89, 1.90, 1.91, 1.92, 1.94, 1.95, 1.97, 1.101, 1.109, 1.110, 1.113, 1.115, 1.116, 1.117, 1.119, 1.120, 1.122, 1.124, 1.125, 1.126

### Example B2: Plutella xylostella (Diamond back moth)

24-well microtiter plates with artificial diet were treated with aqueous test solutions prepared from 10,000 ppm DMSO stock solutions by pipetting. After drying, the plates were infested with L2 larvae (10 to 15 per well). The samples were assessed for mortality and growth inhibition in comparison to untreated samples 5 days after infestation.

The following compounds gave an effect of at least 80% in at least one of the two categories (mortality or growth inhibition) at an application rate of 200 ppm: Example 1.

1.1, 1.4, 1.6, 1.9, 1.10, 1.11, 1.18, 1.20, 1.21, 1.24, 1.26, 1.27, 1.29, 1.30, 1.31, 1.32, 1.33, 1.35, 1.38, 1.43, 1.46, 1.54, 1.55, 1.56, 1.61, 1.62, 1.66, 1.67, 1.68, 1.70, 1.76, 1.77, 1.78, 1.80, 1.83, 1.84, 1.85, 1.89, 1.90, 1.97, 1.109, 1.110, 1.120, 1.122, 1.126

### Example B3: Diabrotica balteata (Corn root worm)

Maize sprouts, placed on an agar layer in 24-well microtiter plates were treated with aqueous test solutions prepared from 10,000 ppm DMSO stock solutions by spraying. After drying, the plates were infested with L2 larvae (6 to 10 per well). The samples were assessed for mortality and growth inhibition in comparison to untreated samples 4 days after infestation.

The following compounds gave an effect of at least 80% in at least one of the two categories (mortality or growth inhibition) at an application rate of 200 ppm:
1.1, 1.4, 1.5, 1.6, 1.9, 1.10, 1.11, 1.13, 1.14, 1.18, 1.20, 1.21, 1.23, 1.25, 1.26, 1.27, 1.29, 1.30, 1.31, 1.32, 1.33, 1.35, 1.36, 1.37, 1.38, 1.39, 1.41, 1.43, 1.44, 1.46, 1.47, 1.48, 1.49, 1.50, 1.51, 1.52, 1.54, 1.55, 1.56, 1.57, 1.58, 1.59, 1.60, 1.61, 1.62, 1.63, 1.65, 1.66, 1.67, 1.68, 1.69, 1.70, 1.72, 1.73, 1.74, 1.75, 1.76, 1.77, 1.78, 1.79, 1.80, 1.81, 1.82, 1.83, 1.84, 1.85, 1.86, 1.87, 1.88, 1.89, 1.90, 1.91, 1.92, 1.93, 1.94, 1.95, 1.96, 1.97, 1.99, 1.100, 1.101, 1.102, 1.105, 1.109, 1.110, 1.111, 1.112, 1.113, 1.114, 1.115, 1.116, 1.117, 1.118, 1.119, 1.120, 1.121, 1.122, 1.124, 1.125, 1.126

### Biological comparison data:

Compound B is disclosed in WO2013/063282 as an example on page 114 (Table 4, M is CH(Me), Rx= 2-F, Ry = 4-CI) and compound C is disclosed in WO 2013/173218 as example on page 123 (Table 14, M is CH(Me), R4a = 2-F, R4b = 4-CI). The activities of compounds B and C to treat different pests are compared with the activities of compound A according to the present invention. The tests are carried out at different concentrations (ppm). It can be seen that compound A of the present invention has surprisingly improved activity in comparison with compound B and C.

### a) Insecticidal activity against Spodoptera littoralis (Egyptian cotton leaf worm)

Cotton leaf discs were placed onto agar in 24-well microtiter plates and sprayed with aqueous test solutions prepared from 10'000 ppm DMSO stock solutions. After drying the leaf discs were infested with five L1 larvae. The samples were assessed for mortality 3 days after infestation.

| Compound | Compound structure | Concentration (ppm) | Mortality (%) |
|---|---|---|---|
| A | 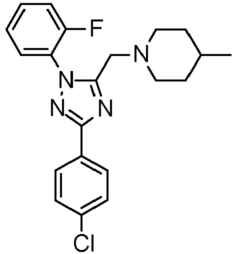 | 200 | 100 |
| | | 50 | 100 |
| | | 12.5 | 100 |
| | | | |
| | | | I |
| | | | |
| B | 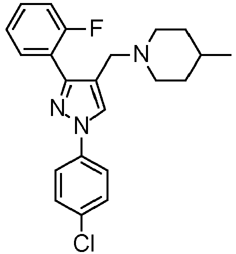 | 200 | 100 |
| | | 50 | 0 |
| | | 12.5 | 0 |
| | | | |
| | | | |
| | | | |
| C | 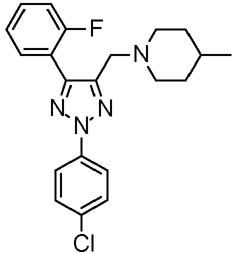 | 200 | 50 |
| | | 50 | 0 |
| | | 12.5 | 0 |
| | | | |
| | | | |
| | | | |

### b) Insecticidal activity against Plutella xylostella (Diamond back moth):

24-well microtiter plates with artificial diet were treated with aqueous test solutions prepared from 10'000 ppm DMSO stock solutions by pipetting. After drying, the plates were infested with L2 larvae (10 to 15 per well). The samples were assessed for mortality and growth inhibition in comparison to untreated samples 5 days after infestation.

| Compound | Compound structure | Concentration (ppm) | Mortality (%) |
|---|---|---|---|
| A | 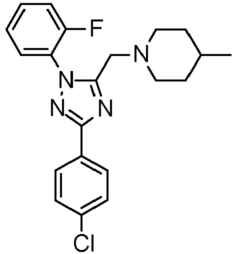 | 200 | 100 |
| | | 50 | 100 |
| | | 12.5 | 50 |
| | | | |
| | | | |
| | | | |
| B | 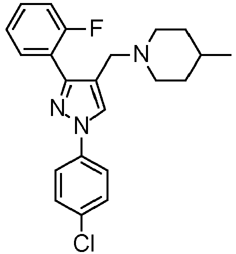 | 200 | 0 |
| | | 50 | 0 |
| | | 12.5 | 0 |
| | | | |
| | | | |
| | | | |
| C | 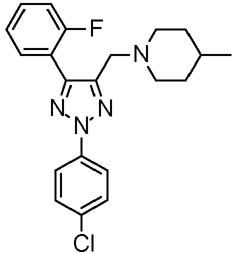 | 200 | 0 |
| | | 50 | 0 |
| | | 12.5 | 0 |
| | | | |
| | | | |
| | | | |

### c) Insecticidal activity against Diabrotica balteata (Corn root worm):

Maize sprouts, placed on an agar layer in 24-well microtiter plates were treated with aqueous test solutions prepared from 10'000 ppm DMSO stock solutions by spraying. After drying, the plates were infested with L2 larvae (6 to 10 per well). The samples were assessed for mortality and growth inhibition in comparison to untreated samples 4 days after infestation.

| Compound | Compound structure | Concentration (ppm) | Mortality (%) |
|---|---|---|---|
| A | 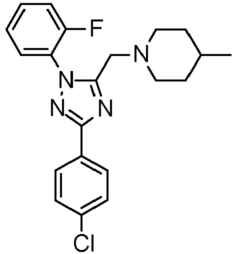 | 200 | 100 |
| | | 50 | 100 |
| | | 12.5 | 100 |
| | | | |
| | | | 0 |
| | | | |
| B | 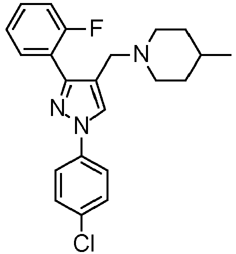 | 200 | 100 |
| | | 50 | 100 |
| | | 12.5 | 0 |
| | | | |
| | | | |
| | | | |
| C | 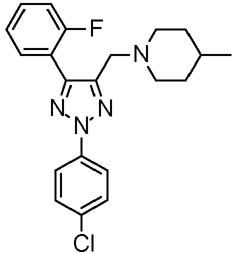 | 200 | 100 |
| | | 50 | 100 |
| | | 12.5 | 0 |
| | | | |
| | | | |
| | | | |

## Claims

1. A compound of formula (la) 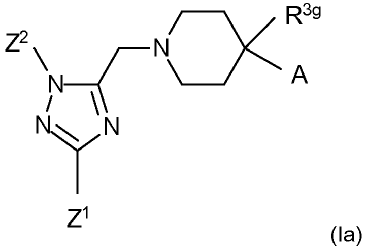 wherein Z¹, Z², A and R^{3g} are as defined in the following table:
| Example No. | Z¹ | Z² | A | R^{3g} |
|---|---|---|---|---|
| 1.1 | 4-chlorophenyl | 2-fluorophenyl | pyrimidin-2-yl | H |
| 1.2 | 5-chloropyridin-2-yl | 2-fluorophenyl | CF₃ | H |
| 1.3 | 5-chloropyridin-2-yl | 2-fluorophenyl | 1,3,4-oxadiazol-2-yl | H |
| 1.4 | 5-chloropyridin-2-yl | 2-fluorophenyl | pyrimidin-2-yl | H |
| 1.5 | 4-chlorophenyl | 2-fluorophenyl | CF₃ | H |
| 1.6 | 4-chlorophenyl | 2-fluorophenyl | 1,3,4-oxadiazol-2-yl | H |
| 1.7 | 5-chloropyridin-2-yl | 2-fluorophenyl | CN | H |
| 1.8 | 4-chlorophenyl | 2-fluorophenyl | CN | H |
| 1.9 | 4-chlorophenyl | 2-fluorophenyl | CO₂CH₃ | H |
| 1.10 | 4-chlorophenyl | 2-fluorophenyl | CH=N-OCH₃ | H |
| 1.11 | 4-chlorophenyl | 2-fluorophenyl | 1yridine-2-yl | H |
| 1.12 | 4-chlorophenyl | 2-fluorophenyl | piperazin-1-yl | H |
| 1.13 | 4-chlorophenyl | 2-fluorophenyl | 5-chloro-pyrimidin-2-yl | H |
| 1.14 | 4-chlorophenyl | 2-fluorophenyl | triazol-2-yl | H |
| 1.15 | 4-chlorophenyl | 2-fluorophenyl | triazol-1-yl | H |
| 1.16 | 4-chlorophenyl | 2,6-difluorophenyl | pyrimidin-2-yl | H |
| 1.17 | 4-chlorophenyl | 2-fluorophenyl | 3-(4-fluorophenyl)-1,2,4-oxadiazol-5-yl | H |
| 1.18 | 4-chlorophenyl | 4-fluorophenyl | pyrimidin-2-yl | H |
| 1.19 | 4-chlorophenyl | 2-fluorophenyl | 4-bromothiazol-2-yl | H |
| 1.20 | 3,4-dichlorophenyl | 2-fluorophenyl | pyrimidin-2-yl | H |
| 1.21 | 4-(trifluoromethyl)-phenyl | 2,6-difluorophenyl | pyrimidin-2-yl | H |
| 1.22 | 4-chlorophenyl | 2-fluorophenyl | tetrazol-1-yl | H |
| 1.23 | 4-chlorophenyl | 2,6-dichlorophenyl | pyrimidin-2-yl | H |
| 1.24 | 4-chlorophenyl | 2-fluorophenyl | 5-chloro-1 H-benzimidazol-2-yl | H |
| 1.25 | 4-chlorophenyl | 2-fluorophenyl | tetrazol-2-yl | H |
| 1.26 | 4-(trifluoromethyl)-phenyl | 2-fluorophenyl | pyrimidin-2-yl | H |
| 1.27 | 4-chlorophenyl | 2-fluorophenyl | 5-(trifluoromethyl)-pyrimidin-2-yl | H |
| 1.28 | 4-chlorophenyl | 3,5-(bis-trifluoromethyl)phenyl | pyrimidin-2-yl | H |
| 1.29 | 4-fluorophenyl | 4-fluorophenyl | pyrimidin-2-yl | H |
| 1.30 | 4-chlorophenyl | 2,4-difluorophenyl | pyrimidin-2-yl | H |
| 1.31 | 4-chlorophenyl | 2-fluorophenyl | CH₃ | H |
| 1.32 | 4-chlorophenyl | 2-fluorophenyl | 4-(trifluoromethyl)-pyrimidin-2-yl | H |
| 1.33 | 3-chloro-4-fluorophenyl | 2-fluorophenyl | pyrimidin-2-yl | H |
| 1.34 | 4-(trifluoromethyl)-phenyl | 4-(trifluoromethyl)-phenyl | pyrimidin-2-yl | H |
| 1.35 | 4-chlorophenyl | 2-fluorophenyl | CH₃ | CH₃ |
| 1.36 | 2-methylphenyl | 2-fluorophenyl | pyrimidin-2-yl | H |
| 1.37 | 3-methylphenyl | 2-fluorophenyl | pyrimidin-2-yl | H |
| 1.38 | 3-(trifluoromethyl)-phenyl | 2-fluorophenyl | pyrimidin-2-yl | H |
| 1.39 | 4-chlorophenyl | 2-fluorophenyl I | 4-(trifluoromethyl)-pyrazol-1-yl | H |
| 1.40 | 4-chlorophenyl | 2-fluorophenyl | 5-(2-thienyl)-1 H-pyrazol-3-yl | H |
| 1.41 | 4-chlorophenyl | 2-fluorophenyl | 5-(4-chlorophenyl)-1 H-pyrazol-3-yl | H |
| 1.42 | 4-chlorophenyl | 2-fluorophenyl | (4-chlorophenyl)methyl | H |
| 1.43 | 4-chlorophenyl | 2-fluorophenyl | phenyl | H |
| 1.44 | 4-chlorophenyl | 2-fluorophenyl | CO₂-tert-C₄H₉ | H |
| 1.45 | 4-chlorophenyl | 2-fluorophenyl | (phenyl)methyl | H |
| 1.46 | 4-chlorophenyl | 2-fluorophenyl | CH₃ | H |
| 1.47 | 4-chlorophenyl | 2-fluorophenyl | CONH₂ | H |
| 1.48 | 4-chlorophenyl | 2-fluorophenyl | 4-(fluorophenyl)-methanone | H |
| 1.49 | 4-chlorophenyl | 2,3,5,6-tetrafluoro-phenyl | pyrimidin-2-yl | H |
| 1.50 | 4-chlorophenyl | 3,4-dichlorophenyl | pyrimidin-2-yl | H |
| 1.51 | 4-chlorophenyl | 3-methylphenyl | pyrimidin-2-yl | H |
| 1.52 | 4-chlorophenyl | 4-methylphenyl | pyrimidin-2-yl | H |
| 1.53 | 4-chlorophenyl | 3,5-dichlorophenyl | pyrimidin-2-yl | H |
| 1.54 | 4-chlorophenyl | 2,4-difluorophenyl | pyrimidin-2-yl | H |
| 1.55 | 4-chlorophenyl | 2,3-dichlorophenyl | pyrimidin-2-yl | H |
| 1.56 | 4-chlorophenyl | (phenyl)methyl | pyrimidin-2-yl | H |
| 1.57 | 4-chlorophenyl | 3-bromophenyl | pyrimidin-2-yl | H |
| 1.58 | 4-chlorophenyl | cyclobutyl | pyrimidin-2-yl | H |
| 1.59 | 4-chlorophenyl | 6-chloropyridine-6-yl | pyrimidin-2-yl | H |
| 1.60 | 4-chlorophenyl | isopropyl | pyrimidin-2-yl | H |
| 1.61 | 4-chlorophenyl | phenyl | pyrimidin-2-yl | H |
| 1.62 | 4-chlorophenyl | 2-methylphenyl | pyrimidin-2-yl | H |
| 1.63 | 4-chlorophenyl | 4-(trifluoromethoxy)-phenyl | pyrimidin-2-yl | H |
| 1.64 | 4-chlorophenyl | 3-chloro-4-methylphenyl | pyrimidin-2-yl | H |
| 1.65 | 4-chlorophenyl | 2,4-dichlorophenyl | pyrimidin-2-yl | H |
| 1.66 | 4-chlorophenyl | 4-chlorophenyl | pyrimidin-2-yl | H |
| 1.67 | 4-chlorophenyl | 2-chloro-4-(trifluoromethyl)-phenyl | pyrimidin-2-yl | H |
| 1.68 | 4-chlorophenyl | 3-chloro-5-(trifluoromethyl)-pyridin-2-yl | pyrimidin-2-yl | H |
| 1.69 | 4-chlorophenyl | 3-chlorophenyl | pyrimidin-2-yl | H |
| 1.70 | 4-chlorophenyl | 2-methoxyphenyl | pyrimidin-2-yl | H |
| 1.71 | 4-chlorophenyl | 4-sulfonamido phenyl | pyrimidin-2-yl | H |
| 1.72 | 4-chlorophenyl | 6-chloropyridine-2-yl | pyrimidin-2-yl | H |
| 1.73 | 4-chlorophenyl | 3,4-dimethylphenyl | pyrimidin-2-yl | H |
| 1.74 | 4-chlorophenyl | 2,5-dimethylphenyl | pyrimidin-2-yl | H |
| 1.75 | 4-chlorophenyl | 3-methoxyphenyl | pyrimidin-2-yl | H |
| 1.76 | 4-chlorophenyl | 4-bromophenyl | pyrimidin-2-yl | H |
| 1.77 | 4-chlorophenyl | 2,4,6-trichlorophenyl | pyrimidin-2-yl | H |
| 1.78 | 4-chlorophenyl | 3yridine-2-yl | pyrimidin-2-yl | H |
| 1.79 | 4-chlorophenyl | 4-methoxyphenyl | pyrimidin-2-yl | H |
| 1.80 | 4-chlorophenyl | 3-fluorophenyl | pyrimidin-2-yl | H |
| 1.81 | 4-chlorophenyl | 3-(trifluoromethyl)-phenyl | pyrimidin-2-yl | H |
| 1.82 | 4-chlorophenyl | 2,3-dimethylphenyl | pyrimidin-2-yl | H |
| 1.83 | 4-chlorophenyl | 2-chlorophenyl | pyrimidin-2-yl | H |
| 1.84 | 4-chlorophenyl | 3-chloro-2-methylphenyl | pyrimidin-2-yl | H |
| 1.85 | 4-chlorophenyl | 4-chloro-2-methylphenyl | pyrimidin-2-yl | H |
| 1.86 | 4-chlorophenyl | 2,5-dichlorophenyl | pyrimidin-2-yl | H |
| 1.87 | 4-chlorophenyl | 2,4-dimethylphenyl | pyrimidin-2-yl | H |
| 1.88 | 4-chlorophenyl | 4-(trifluoromethyl)-phenyl | pyrimidin-2-yl | H |
| 1.89 | 4-chlorophenyl | 3-chloro-3yridine-2-yl | pyrimidin-2-yl | H |
| 1.90 | 4-chlorophenyl | 3,5-dichloro-3yridine-2-yl | pyrimidin-2-yl | H |
| 1.91 | (4-chloro-phenyl)methyl | 2-fluorophenyl | pyrimidin-2-yl | H |
| 1.92 | 3-fluoro-4-methylphenyl | 2-fluorophenyl | pyrimidin-2-yl | H |
| 1.93 | cyclopropyl | 2-fluorophenyl | pyrimidin-2-yl | H |
| 1.94 | phenyl | 2-fluorophenyl | pyrimidin-2-yl | H |
| 1.95 | 4-methylphenyl | 2-fluorophenyl | pyrimidin-2-yl | H |
| 1.96 | thiophen-2-yl | 2-fluorophenyl | pyrimidin-2-yl | H |
| 1.97 | 5-(trifluoromethyl)-pyridin-2-yl | 2-fluorophenyl | pyrimidin-2-yl | H |
| 1.98 | 3yridine-3-yl | 2-fluorophenyl | pyrimidin-2-yl | H |
| 1.99 | ethyl | 2-fluorophenyl | pyrimidin-2-yl | H |
| 1.100 | isopropyl | 2-fluorophenyl | pyrimidin-2-yl | H |
| 1.101 | 4-methoxyphenyl | 2-fluorophenyl | pyrimidin-2-yl | H |
| 1.102 | 3yridine-2-yl | 2-fluorophenyl | pyrimidin-2-yl | H |
| 1.103 | 2-methyl-propyl | 2-fluorophenyl | pyrimidin-2-yl | H |
| 1.104 | cyclohexyl | 2-fluorophenyl | pyrimidin-2-yl | H |
| 1.105 | 2-fluorophenyl | 2-fluorophenyl | pyrimidin-2-yl | H |
| 1.106 | 2-ethyl-propyl | 2-fluorophenyl | pyrimidin-2-yl | H |
| 1.107 | 6-methyl-pyridin-2-yl | 2-fluorophenyl | pyrimidin-2-yl | H |
| 1.108 | 4-chlorophenyl | 2-fluorophenyl | 4-carbonyl-piperidin-4-one | H |
| 1.109 | 4-chlorophenyl | 2-fluorophenyl | 4-(trifluoromethyl)-1 H-imdazol-2-yl | H |
| 1.110 | 4-chlorophenyl | 2-fluorophenyl | 2-methyl-pyrimidin-4-yl | H |
| 1.111 | 4-chlorophenyl | 2-fluorophenyl | N-methyl-N-methoxy-carboxamide | H |
| 1.112 | 4-chlorophenyl | 2-fluorophenyl | 1 H-imidazol-2-yl | H |
| 1.113 | 4-chlorophenyl | 2-fluorophenyl | 1,2,4-oxadiazol-3-yl | H |
| 1.114 | 4-chlorophenyl | 2-fluorophenyl | N-cyclopropyl-carboxamide | H |
| 1.115 | 4-chlorophenyl | 2-fluorophenyl | thiazol-2-yl | H |
| 1.116 | 4-chlorophenyl | 2-fluorophenyl | pyrimidin-4-yl | H |
| 1.117 | 4-chlorophenyl | 2-fluorophenyl | 1-methylimidazol-2-yl | H |
| 1.118 | 4-chlorophenyl | 2-fluorophenyl | 2-methyl-1,3,4-oxadiazol-5-yl | H |
| 1.119 | 4-chlorophenyl | 2-fluorophenyl | thiazol-4-yl | H |
| 1.120 | 4-chlorophenyl | 2-fluorophenyl | isoxazol-3-yl | H |
| 1.121 | 4-chlorophenyl | 2-fluorophenyl | 1,2,4-oxadiazol-5-yl | H |
| 1.122 | 4-chlorophenyl | 2-fluorophenyl | 5-fluoro-pyridin-2-yl | H |
| 1.123 | 4-chlorophenyl | 2-fluorophenyl | (4-pyridyl)-1,3,4-oxadiazol-2-yl | H |
| 1.124 | 4-chlorophenyl | 2-fluorophenyl | isoxazol-2-yl | H |
| 1.125 | 4-chlorophenyl | 2-fluorophenyl | 4-methylpyrimidin-2-yl | H |
| 1.126 | 4-chlorophenyl | 2-fluorophenyl | 3-chloro-pyridin-2-yl | H |
| 1.127 | 4-chloro-3-fluorophenyl | 2-fluorophenyl | pyrimidin-2-yl | H |
or an agrochemically acceptable salt or N-oxide thereof.

2. A pesticidal composition, which comprises at least one compound according to claim 1, or an agrochemically acceptable salt or N-oxide thereof, as active ingredient and at least one auxiliary.

3. The composition according to claim 2, which further comprises one or more other insecticidally, acaricidally, nematicidally and/or fungicidally active agents.

4. A method for controlling pests, which comprises applying a composition according to claim 2 or 3 to the pests or their environment with the exception of a method for treatment of the human or animal body by surgery or therapy and diagnostic methods practised on the human or animal body.

5. A method for the protection of plant propagation material from the attack by pests, which comprises treating the propagation material or the site, where the propagation material is planted, with a composition according to claim 2 or 3.

6. A coated plant propagation material, wherein the coating of the plant propagation material comprises a compound as defined in claim 1.

## Patentansprüche

1. Verbindung der Formel (Ia) 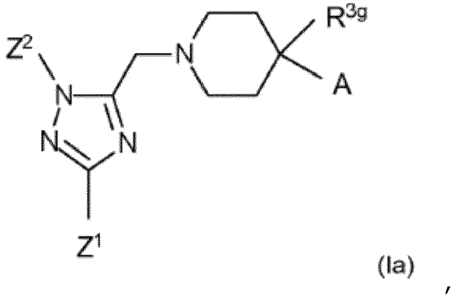 wobei Z¹, Z², A und R^{3g} wie in der folgenden Tabelle definiert sind:
| Beispiel Nr. | Z¹ | Z² | A | R^{3g} |
|---|---|---|---|---|
| 1.1 | 4-Chlorphenyl | 2-Fluorphenyl | Pyrimidin-2-yl | H |
| 1.2 | 5-Chlorpyridin-2-yl | 2-Fluorphenyl | CF₃ | H |
| 1.3 | 5-Chlorpyridin-2-yl | 2-Fluorphenyl | 1,3,4-Oxadiazol-2-yl | H |
| 1.4 | 5-Chlorpyridin-2-yl | 2-Fluorphenyl | Pyrimidin-2-yl | H |
| 1.5 | 4-Chlorphenyl | 2-Fluorphenyl | CF₃ | H |
| 1.6 | 4-Chlorphenyl | 2-Fluorphenyl | 1,3,4-Oxadiazol-2-yl | H |
| 1.7 | 5-Chlorpyridin-2-yl | 2-Fluorphenyl | CN | H |
| 1.8 | 4-Chlorphenyl | 2-Fluorphenyl | CN | H |
| 1.9 | 4-Chlorphenyl | 2-Fluorphenyl | CO₂CH₃ | H |
| 1.10 | 4-Chlorphenyl | 2-Fluorphenyl | CH=N-OCH₃ | H |
| 1.11 | 4-Chlorphenyl | 2-Fluorphenyl | 1-Pyridin-2-yl | H |
| 1.12 | 4-Chlorphenyl | 2-Fluorphenyl | Piperazin-1-yl | H |
| 1.13 | 4-Chlorphenyl | 2-Fluorphenyl | 5-Chlorpyrimidin-2-yl | H |
| 1.14 | 4-Chlorphenyl | 2-Fluorphenyl | Triazol-2-yl | H |
| 1.15 | 4-Chlorphenyl | 2-Fluorphenyl | Triazol-1-yl | H |
| 1.16 | 4-Chlorphenyl | 2,6-Difluorphenyl | Pyrimidin-2-yl | H |
| 1.17 | 4-Chlorphenyl | 2-Fluorphenyl | 3-(4-Fluorphenyl)-1,2,4-oxadiazol-5-yl | H |
| 1.18 | 4-Chlorphenyl | 4-Fluorphenyl | Pyrimidin-2-yl | H |
| 1.19 | 4-Chlorphenyl | 2-Fluorphenyl | 4-Bromthiazol-2-yl | H |
| 1.20 | 3,4-Dichlorphenyl | 2-Fluorphenyl | Pyrimidin-2-yl | H |
| 1.21 | 4-(Trifluormethyl)phenyl | 2,6-Difluorphenyl | Pyrimidin-2-yl | H |
| 1.22 | 4-Chlorphenyl | 2-Fluorphenyl | Tetrazol-1-yl | H |
| 1.23 | 4-Chlorphenyl | 2,6-Dichlorphenyl | Pyrimidin-2-yl | H |
| 1.24 | 4-Chlorphenyl | 2-Fluorphenyl | 5-Chlor-1H-benzimidazol-2-yl | H |
| 1.25 | 4-Chlorphenyl | 2-Fluorphenyl | Tetrazol-2-yl | H |
| 1.26 | 4-(Trifluormethyl)phenyl | 2-Fluorphenyl | Pyrimidin-2-yl | H |
| 1.27 | 4-Chlorphenyl | 2-Fluorphenyl | 5-(Trifluormethyl)pyrimidin-2-yl | H |
| 1.28 | 4-Chlorphenyl | 3,5-(Bistrifluormethyl)phenyl | Pyrimidin-2-yl | H |
| 1.29 | 4-Fluorphenyl | 4-Fluorphenyl | Pyrimidin-2-yl | H |
| 1.30 | 4-Chlorphenyl | 2,4-Difluorphenyl | Pyrimidin-2-yl | H |
| 1.31 | 4-Chlorphenyl | 2-Fluorphenyl | CH₃ | H |
| 1.32 | 4-Chlorphenyl | 2-Fluorphenyl | 4-(Trifluormethyl)pyrimidin-2-yl | H |
| 1.33 | 3-Chlor-4-fluorphenyl | 2-Fluorphenyl | Pyrimidin-2-yl | H |
| 1.34 | 4-(Trifluormethyl)phenyl | 4-(Trifluormethyl)phenyl | Pyrimidin-2-yl | H |
| 1.35 | 4-Chlorphenyl | 2-Fluorphenyl | CH₃ | CH₃ |
| 1.36 | 2-Methylphenyl | 2-Fluorphenyl | Pyrimidin-2-yl | H |
| 1.37 | 3-Methylphenyl | 2-Fluorphenyl | Pyrimidin-2-yl | H |
| 1.38 | 3-(Trifluormethyl) phenyl | 2-Fluorphenyl | Pyrimidin-2-yl | H |
| 1.39 | 4-Chlorphenyl | 2-Fluorphenyl | 4-(Trifluormethyl)pyrazol-1-yl | H |
| 1.40 | 4-Chlorphenyl | 2-Fluorphenyl | 5-(2-Thienyl)-1H-pyrazol-3-yl | H |
| 1.41 | 4-Chlorphenyl | 2-Fluorphenyl | 5-(4-Chlorphenyl)-1H-pyrazol-3-yl | H |
| 1.42 | 4-Chlorphenyl | 2-Fluorphenyl | (4-Chlorphenyl) methyl | H |
| 1.43 | 4-Chlorphenyl | 2-Fluorphenyl | Phenyl | H |
| 1.44 | 4-Chlorphenyl | 2-Fluorphenyl | CO₂-tert.-C₄H₉ | H |
| 1.45 | 4-Chlorphenyl | 2-Fluorphenyl | (Phenyl)methyl | H |
| 1.46 | 4-Chlorphenyl | 2-Fluorphenyl | CH₃ | H |
| 1.47 | 4-Chlorphenyl | 2-Fluorphenyl | CONH₂ | H |
| 1.48 | 4-Chlorphenyl | 2-Fluorphenyl | 4-(Fluorphenyl)methanon | H |
| 1.49 | 4-Chlorphenyl | 2,3,5,6-Tetrafluorphenyl | Pyrimidin-2-yl | H |
| 1.50 | 4-Chlorphenyl | 3,4-Dichlorphenyl | Pyrimidin-2-yl | H |
| 1.51 | 4-Chlorphenyl | 3-Methylphenyl | Pyrimidin-2-yl | H |
| 1.52 | 4-Chlorphenyl | 4-Methylphenyl | Pyrimidin-2-yl | H |
| 1.53 | 4-Chlorphenyl | 3,5-Dichlorphenyl | Pyrimidin-2-yl | H |
| 1.54 | 4-Chlorphenyl | 2,4-Difluorphenyl | Pyrimidin-2-yl | H |
| 1.55 | 4-Chlorphenyl | 2,3-Dichlorphenyl | Pyrimidin-2-yl | H |
| 1.56 | 4-Chlorphenyl | (Phenyl)methyl | Pyrimidin-2-yl | H |
| 1.57 | 4-Chlorphenyl | 3-Bromphenyl | Pyrimidin-2-yl | H |
| 1.58 | 4-Chlorphenyl | Cyclobutyl | Pyrimidin-2-yl | H |
| 1.59 | 4-Chlorphenyl | 6-Chlorpyridin-6-yl | Pyrimidin-2-yl | H |
| 1.60 | 4-Chlorphenyl | Isopropyl | Pyrimidin-2-yl | H |
| 1.61 | 4-Chlorphenyl | Phenyl | Pyrimidin-2-yl | H |
| 1.62 | 4-Chlorphenyl | 2-Methylphenyl | Pyrimidin-2-yl | H |
| 1.63 | 4-Chlorphenyl | 4-(Trifluor-methoxy)phenyl | Pyrimidin-2-yl | H |
| 1.64 | 4-Chlorphenyl | 3-Chlor-4-methylphenyl | Pyrimidin-2-yl | H |
| 1.65 | 4-Chlorphenyl | 2,4-Dichlorphenyl | Pyrimidin-2-yl | H |
| 1.66 | 4-Chlorphenyl | 4-Chlorphenyl | Pyrimidin-2-yl | H |
| 1.67 | 4-Chlorphenyl | 2-Chlor-4-(trifluormethyl) phenyl | Pyrimidin-2-yl | H |
| 1.68 | 4-Chlorphenyl | 3-Chlor-5-(trifluormethyl)pyridin-2-yl | Pyrimidin-2-yl | H |
| 1.69 | 4-Chlorphenyl | 3-Chlorphenyl | Pyrimidin-2-yl | H |
| 1.70 | 4-Chlorphenyl | 2-Methoxyphenyl | Pyrimidin-2-yl | H |
| 1.71 | 4-Chlorphenyl | 4-Sulfonamidophenyl | Pyrimidin-2-yl | H |
| 1.72 | 4-Chlorphenyl | 6-Chlorpyridin-2-yl | Pyrimidin-2-yl | H |
| 1.73 | 4-Chlorphenyl | 3,4-Dimethylphenyl | Pyrimidin-2-yl | H |
| 1.74 | 4-Chlorphenyl | 2,5-Dimethylphenyl | Pyrimidin-2-yl | H |
| 1.75 | 4-Chlorphenyl | 3-Methoxyphenyl | Pyrimidin-2-yl | H |
| 1.76 | 4-Chlorphenyl | 4-Bromphenyl | Pyrimidin-2-yl | H |
| 1.77 | 4-Chlorphenyl | 2,4,6-Trichlorphenyl | Pyrimidin-2-yl | H |
| 1.78 | 4-Chlorphenyl | 3-Pyridin-2-yl | Pyrimidin-2-yl | H |
| 1.79 | 4-Chlorphenyl | 4-Methoxyphenyl | Pyrimidin-2-yl | H |
| 1.80 | 4-Chlorphenyl | 3-Fluorphenyl | Pyrimidin-2-yl | H |
| 1.81 | 4-Chlorphenyl | 3-(Trifluormethyl) phenyl | Pyrimidin-2-yl | H |
| 1.82 | 4-Chlorphenyl | 2,3-Dimethylphenyl | Pyrimidin-2-yl | H |
| 1.83 | 4-Chlorphenyl | 2-Chlorphenyl | Pyrimidin-2-yl | H |
| 1.84 | 4-Chlorphenyl | 3-Chlor-2-methylphenyl | Pyrimidin-2-yl | H |
| 1.85 | 4-Chlorphenyl | 4-Chlor-2-methylphenyl | Pyrimidin-2-yl | H |
| 1.86 | 4-Chlorphenyl | 2,5-Dichlorphenyl | Pyrimidin-2-yl | H |
| 1.87 | 4-Chlorphenyl | 2,4-Dimethylphenyl | Pyrimidin-2-yl | H |
| 1.88 | 4-Chlorphenyl | 4-(Trifluormethyl) phenyl | Pyrimidin-2-yl | H |
| 1.89 | 4-Chlorphenyl | 3-Chlor-3-pyridin-2-yl | Pyrimidin-2-yl | H |
| 1.90 | 4-Chlorphenyl | 3,5-Dichlor-3-pyridin-2-yl | Pyrimidin-2-yl | H |
| 1.91 | (4-Chlorphenyl) methyl | 2-Fluorphenyl | Pyrimidin-2-yl | H |
| 1.92 | 3-Fluor-4-methylphenyl | 2-Fluorphenyl | Pyrimidin-2-yl | H |
| 1.93 | Cyclopropyl | 2-Fluorphenyl | Pyrimidin-2-yl | H |
| 1.94 | Phenyl | 2-Fluorphenyl | Pyrimidin-2-yl | H |
| 1.95 | 4-Methylphenyl | 2-Fluorphenyl | Pyrimidin-2-yl | H |
| 1.96 | Thiophen-2-yl | 2-Fluorphenyl | Pyrimidin-2-yl | H |
| 1.97 | 5-(Trifluormethyl)pyridin-2-yl | 2-Fluorphenyl | Pyrimidin-2-yl | H |
| 1. 98 | 3-Pyridin-3-yl | 2-Fluorphenyl | Pyrimidin-2-yl | H |
| 1.99 | Ethyl | 2-Fluorphenyl | Pyrimidin-2-yl | H |
| 1.100 | Isopropyl | 2-Fluorphenyl | Pyrimidin-2-yl | H |
| 1.101 | 4-Methoxyphenyl | 2-Fluorphenyl | Pyrimidin-2-yl | H |
| 1.102 | 3-Pyridin-2-yl | 2-Fluorphenyl | Pyrimidin-2-yl | H |
| 1.103 | 3-Methylpropyl | 2-Fluorphenyl | Pyrimidin-2-yl | H |
| 1.104 | Cyclohexyl | 2-Fluorphenyl | Pyrimidin-2-yl | H |
| 1.105 | 2-Fluorphenyl | 2-Fluorphenyl | Pyrimidin-2-yl | H |
| 1.106 | 2-Ethylpropyl | 2-Fluorphenyl | Pyrimidin-2-yl | H |
| 1.107 | 6-Methylpyridin-2-yl | 2-Fluorphenyl | Pyrimidin-2-yl | H |
| 1.108 | 4-Chlorphenyl | 2-Fluorphenyl | 4-Carbonylpiperidin-4-on | H |
| 1.109 | 4-Chlorphenyl | 2-Fluorphenyl | 4-(Trifluormethyl)-1H-imidazol-2-yl | H |
| 1.110 | 4-Chlorphenyl | 2-Fluorphenyl | 2-Methylpyrimidin-4-yl | H |
| 1.111 | 4-Chlorphenyl | 2-Fluorphenyl | N-Methyl-N-methoxycarboxamid | H |
| 1.112 | 4-Chlorphenyl | 2-Fluorphenyl | 1H-Imidazol-2-yl | H |
| 1.113 | 4-Chlorphenyl | 2-Fluorphenyl | 1,2,4-Oxadiazol-3-yl | H |
| 1.114 | 4-Chlorphenyl | 2-Fluorphenyl | N-Cyclopropylcarboxyamid | H |
| 1.115 | 4-Chlorphenyl | 2-Fluorphenyl | Thiazol-2-yl | H |
| 1.116 | 4-Chlorphenyl | 2-Fluorphenyl | Pyrimidin-4-yl | H |
| 1.117 | 4-Chlorphenyl | 2-Fluorphenyl | 1-Methylimidazol-2-yl | H |
| 1. 118 | 4-Chlorphenyl | 2-Fluorphenyl | 2-Methyl-1,3,4-oxadiazol-5-yl | H |
| 1.119 | 4-Chlorphenyl | 2-Fluorphenyl | Thiazol-4-yl | H |
| 1.120 | 4-Chlorphenyl | 2-Fluorphenyl | Isoxazol-3-yl | H |
| 1.121 | 4-Chlorphenyl | 2-Fluorphenyl | 1,2,4-Oxadiazol-5-yl | H |
| 1.122 | 4-Chlorphenyl | 2-Fluorphenyl | 5-Fluorpyridin-2-yl | H |
| 1.123 | 4-Chlorphenyl | 2-Fluorphenyl | (4-Pyridyl)-1,3,4-oxadiazol-2-yl | H |
| 1.124 | 4-Chlorphenyl | 2-Fluorphenyl | Isoxazol-2-yl | H |
| 1.125 | 4-Chlorphenyl | 2-Fluorphenyl | 4-Methylpyrimidin-2-yl | H |
| 1.126 | 4-Chlorphenyl | 2-Fluorphenyl | 3-Chlorpyridin-2-yl | H |
| 1.127 | 4-Chlor-3-fluorphenyl | 2-Fluorphenyl | Pyrimidin-2-yl | H |
oder ein landwirtschaftlich unbedenkliches Salz oder N-Oxid davon.

2. Pestizide Zusammensetzung, umfassend mindestens eine Verbindung nach Anspruch 1 oder ein agrochemisch unbedenkliches Salz oder N-Oxid davon als Wirkstoff und mindestens einen Hilfsstoff.

3. Zusammensetzung nach Anspruch 2, welche weiterhin ein oder mehrere insektizid, akarizid, nematizid und/oder fungizid wirksame Mittel umfasst.

4. Verfahren zur Bekämpfung von Schädlingen, bei dem man eine Zusammensetzung nach Anspruch 2 oder 3 auf die Schädlinge oder ihre Umgebung ausbringt, mit Ausnahme eines Verfahrens zur Behandlung des menschlichen oder tierischen Körpers durch einen chirurgischen Eingriff oder Therapie und am menschlichen oder tierischen Körper durchgeführten diagnostischen Verfahren.

5. Verfahren zum Schützen von Pflanzenfortpflanzungsmaterial gegen Befall durch Schädlinge, bei dem man das Fortpflanzungsmaterial oder die Stelle, an der das Fortpflanzungsmaterial gepflanzt ist, mit einer Zusammensetzung nach Anspruch 2 oder 3 behandelt.

6. Beschichtetes Pflanzenfortpflanzungsmaterial, wobei die Beschichtung des Pflanzenfortpflanzungsmaterials eine wie in Anspruch 1 definierte Verbindung umfasst.

## Revendications

1. Composé de formule (Ia) 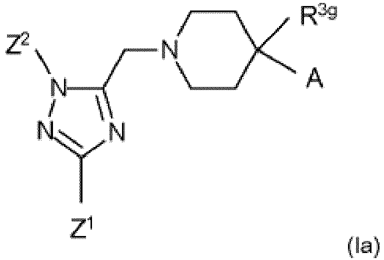 Z¹, Z², A et R^{3g} étant tels que définis dans le tableau suivant :
| Exemple n° | z¹ | z² | A | R^{3g} |
|---|---|---|---|---|
| 1.1 | 4-chlorophényle | 2-fluorophényle | pyrimidin-2-yle | H |
| 1.2 | 5-chloropyridin-2-yle | 2-fluorophényle | CF₃ | H |
| 1.3 | 5-chloropyridin-2-yle | 2-fluorophényle | 1,3,4-oxadiazol-2-yle | H |
| 1.4 | 5-chloropyridin-2-yle | 2-fluorophényle | pyrimidin-2-yle | H |
| 1.5 | 4-chlorophényle | 2-fluorophényle | CF₃ | H |
| 1.6 | 4-chlorophényle | 2-fluorophényle | 1,3,4-oxadiazol-2-yle | H |
| 1.7 | 5-chloropyridin-2-yle | 2-fluorophényle | CN | H |
| 1.8 | 4-chlorophényle | 2-fluorophényle | CN | H |
| 1.9 | 4-chlorophényle | 2-fluorophényle | CO₂CH₃ | H |
| 1.10 | 4-chlorophényle | 2-fluorophényle | CH=N-OCH₃ | H |
| 1.11 | 4-chlorophényle | 2-fluorophényle | pyridine-2-yle | H |
| 1.12 | 4-chlorophényle | 2-fluorophényle | pipérazin-1-yle | H |
| 1.13 | 4-chlorophényle | 2-fluorophényle | 5-chloro-pyrimidin-2-yle | H |
| 1.14 | 4-chlorophényle | 2-fluorophényle | triazol-2-yle | H |
| 1.15 | 4-chlorophényle | 2-fluorophényle | triazol-1-yle | H |
| 1.16 | 4-chlorophényle | 2,6-difluorophényle | pyrimidin-2-yle | H |
| 1.17 | 4-chlorophényle | 2-fluorophényle | 3-(4-fluorophényl)-1,2,4-oxadiazol-5-yle | H |
| 1.18 | 4-chlorophényle | 4-fluorophényle | pyrimidin-2-yle | H |
| 1.19 | 4-chlorophényle | 2-fluorophényle | 4-bromothiazol-2-yle | H |
| 1.20 | 3,4-dichlorophényle | 2-fluorophényle | pyrimidin-2-yle | H |
| 1.21 | 4-(trifluorométhyl) -phényle | 2,6-difluorophényle | pyrimidin-2-yle | H |
| 1.22 | 4-chlorophényle | 2-fluorophényle | tétrazol-1-yle | H |
| 1.23 | 4-chlorophényle | 2,6-dichlorophényle | pyrimidin-2-yle | H |
| 1.24 | 4-chlorophényle | 2-fluorophényle | 5-chloro-1H-benzimidazol-2-yle | H |
| 1.25 | 4-chlorophényle | 2-fluorophényle | tétrazol-2-yle | H |
| 1.26 | 4-(trifluorométhyl) -phényle | 2-fluorophényle | pyrimidin-2-yle | H |
| 1.27 | 4-chlorophényle | 2-fluorophényle | 5-(trifluorométhyl)-pyrimidin-2-yle | H |
| 1.28 | 4-chlorophényle | 3,5-(bis-trifluorométhyl) phényle | pyrimidin-2-yle | H |
| 1.29 | 4-fluorophényle | 4-fluorophényle | pyrimidin-2-yle | H |
| 1.30 | 4-chlorophényle | 2,4-difluorophényle | pyrimidin-2-yle | H |
| 1.31 | 4-chlorophényle | 2-fluorophényle | CH₃ | H |
| 1.32 | 4-chlorophényle | 2-fluorophényle | 4-(trifluorométhyl)-pyrimidin-2-yle | H |
| 1.33 | 3-chloro-4-fluorophényle | 2-fluorophényle | pyrimidin-2-yle | H |
| 1.34 | 4-(trifluorométhyl) -phényle | 4-(trifluorométhyl )-phényle | pyrimidin-2-yle | H |
| 1.35 | 4-chlorophényle | 2-fluorophényle | CH₃ | CH3 |
| 1.36 | 2-méthylphényle | 2-fluorophényle | pyrimidin-2-yle | H |
| 1.37 | 3-méthylphényle | 2-fluorophényle | pyrimidin-2-yle | H |
| 1.38 | 3-(trifluorométhyl) -phényle | 2-fluorophényle | pyrimidin-2-yle | H |
| 1.39 | 4-chlorophényle | 2-fluorophényle I | 4-(trifluorométhyl)-pyrazol-1-yle | H |
| 1.40 | 4-chlorophényle | 2-fluorophényle | 5-(2-thienyl)-1H-pyrazol-3-yle | H |
| 1.41 | 4-chlorophényle | 2-fluorophényle | 5-(4-chlorophényl)-1H-pyrazol-3-yle | H |
| 1.42 | 4-chlorophényle | 2-fluorophényle | (4-chlorophényl)méthyl e | H |
| 1.43 | 4-chlorophényle | 2-fluorophényle | phényle | H |
| 1.44 | 4-chlorophényle | 2-fluorophényle | CO₂-tert-C₄H₉ | H |
| 1.45 | 4-chlorophényle | 2-fluorophényle | (phényl)méthyle | H |
| 1.46 | 4-chlorophényle | 2-fluorophényle | CH₃ | H |
| 1.47 | 4-chlorophényle | 2-fluorophényle | CONH₂ | H |
| 1.48 | 4-chlorophényle | 2-fluorophényle | 4-(fluorophényl)-méthanone | H |
| 1.49 | 4-chlorophényle | 2,3,5,6-tétrafluorophényle | pyrimidin-2-yle | H |
| 1.50 | 4-chlorophényle | 3,4-dichlorophényle | pyrimidin-2-yle | H |
| 1.51 | 4-chlorophényle | 3-méthylphényle | pyrimidin-2-yle | H |
| 1.52 | 4-chlorophényle | 4-méthylphényle | pyrimidin-2-yle | H |
| 1.53 | 4-chlorophényle | 3,5-dichlorophényle | pyrimidin-2-yle | H |
| 1.54 | 4-chlorophényle | 2,4-difluorophényle | pyrimidin-2-yle | H |
| 1.55 | 4-chlorophényle | 2,3-dichlorophényle | pyrimidin-2-yle | H |
| 1.56 | 4-chlorophényle | (phényl)méthyle | pyrimidin-2-yle | H |
| 1.57 | 4-chlorophényle | 3-bromophényle | pyrimidin-2-yle | H |
| 1.58 | 4-chlorophényle | cyclobutyle | pyrimidin-2-yle | H |
| 1.59 | 4-chlorophényle | 6-chloropyridine-6-yle | pyrimidin-2-yle | H |
| 1.60 | 4-chlorophényle | isopropyle | pyrimidin-2-yle | H |
| 1.61 | 4-chlorophényle | phényle | pyrimidin-2-yle | H |
| 1.62 | 4-chlorophényle | 2-méthylphényle | pyrimidin-2-yle | H |
| 1.63 | 4-chlorophényle | 4-(trifluorométhox y)-phényle | pyrimidin-2-yle | H |
| 1.64 | 4-chlorophényle | 3-chloro-4-méthylphényle | pyrimidin-2-yle | H |
| 1.65 | 4-chlorophényle | 2,4-dichlorophényle | pyrimidin-2-yle | H |
| 1.66 | 4-chlorophényle | 4-chlorophényle | pyrimidin-2-yle | H |
| 1.67 | 4-chlorophényle | 2-chloro-4-(trifluorométhyl )-phényle | pyrimidin-2-yle | H |
| 1.68 | 4-chlorophényle | 3-chloro-5-(trifluorométhyl )-pyridin-2-yle | pyrimidin-2-yle | H |
| 1.69 | 4-chlorophényle | 3-chlorophényle | pyrimidin-2-yle | H |
| 1.70 | 4-chlorophényle | 2-méthoxyphényle | pyrimidin-2-yle | H |
| 1.71 | 4-chlorophényle | 4-sulfonamido phényle | pyrimidin-2-yle | H |
| 1.72 | 4-chlorophényle | 6-chloropyridine-2-yle | pyrimidin-2-yle | H |
| 1.73 | 4-chlorophényle | 3,4-diméthylphényle | pyrimidin-2-yle | H |
| 1.74 | 4-chlorophényle | 2,5-diméthylphényle | pyrimidin-2-yle | H |
| 1.75 | 4-chlorophényle | 3-méthoxyphényle | pyrimidin-2-yle | H |
| 1.76 | 4-chlorophényle | 4-bromophényle | pyrimidin-2-yle | H |
| 1.77 | 4-chlorophényle | 2,4,6-trichlorophényle | pyrimidin-2-yle | H |
| 1.78 | 4-chlorophényle | 3yridine-2-yle | pyrimidin-2-yle | H |
| 1.79 | 4-chlorophényle | 4-méthoxyphényle | pyrimidin-2-yle | H |
| 1.80 | 4-chlorophényle | 3-fluorophényle | pyrimidin-2-yle | H |
| 1.81 | 4-chlorophényle | 3-(trifluorométhyl )-phényle | pyrimidin-2-yle | H |
| 1.82 | 4-chlorophényle | 2,3-diméthylphényle | pyrimidin-2-yle | H |
| 1.83 | 4-chlorophényle | 2-chlorophényle | pyrimidin-2-yle | H |
| 1.84 | 4-chlorophényle | 3-chloro-2-méthylphényle | pyrimidin-2-yle | H |
| 1.85 | 4-chlorophényle | 4-chloro-2-méthylphényle | pyrimidin-2-yle | H |
| 1.86 | 4-chlorophényle | 2,5-dichlorophényle | pyrimidin-2-yle | H |
| 1.87 | 4-chlorophényle | 2,4-diméthylphényle | pyrimidin-2-yle | H |
| 1.88 | 4-chlorophényle | 4-(trifluorométhyl )-phényle | pyrimidin-2-yle | H |
| 1.89 | 4-chlorophényle | 3-chloro-3yridine-2-yle | pyrimidin-2-yle | H |
| 1.90 | 4-chlorophényle | 3,5-dichloro3yridine-2-yle | pyrimidin-2-yle | H |
| 1.91 | (4-chlorophényl)méthyle | 2-fluorophényle | pyrimidin-2-yle | H |
| 1.92 | 3-fluoro-4-méthylphényle | 2-fluorophényle | pyrimidin-2-yle | H |
| 1.93 | cyclopropyle | 2-fluorophényle | pyrimidin-2-yle | H |
| 1.94 | phényle | 2-fluorophényle | pyrimidin-2-yle | H |
| 1.95 | 4-méthylphényle | 2-fluorophényle | pyrimidin-2-yle | H |
| 1.96 | thiophén-2-yle | 2-fluorophényle | pyrimidin-2-yle | H |
| 1.97 | 5-(trifluorométhyl) -pyridin-2-yle | 2-fluorophényle | pyrimidin-2-yle | H |
| 1. 98 | 3yridine-3-yle | 2-fluorophényle | pyrimidin-2-yle | H |
| 1.99 | éthyle | 2-fluorophényle | pyrimidin-2-yle | H |
| 1.100 | isopropyle | 2-fluorophényle | pyrimidin-2-yle | H |
| 1.101 | 4-méthoxyphényle | 2-fluorophényle | pyrimidin-2-yle | H |
| 1.102 | 3yridine-2-yle | 2-fluorophényle | pyrimidin-2-yle | H |
| 1.103 | 2-méthyl-propyle | 2-fluorophényle | pyrimidin-2-yle | H |
| 1.104 | cyclohexyle | 2-fluorophényle | pyrimidin-2-yle | H |
| 1.105 | 2-fluorophényle | 2-fluorophényle | pyrimidin-2-yle | H |
| 1.106 | 2-éthyl-propyle | 2-fluorophényle | pyrimidin-2-yle | H |
| 1.107 | 6-méthyl-pyridin-2-yle | 2-fluorophényle | pyrimidin-2-yle | H |
| 1.108 | 4-chlorophényle | 2-fluorophényle | 4-carbonyl-pipéridin-4-one | H |
| 1.109 | 4-chlorophényle | 2-fluorophényle | 4-(trifluorométhyl)-1H-imdazol-2-yle | H |
| 1.110 | 4-chlorophényle | 2-fluorophényle | 2-méthyl-pyrimidin-4-yle | H |
| 1.111 | 4-chlorophényle | 2-fluorophényle | N-méthyl-N-méthoxy-carboxamide | H |
| 1.112 | 4-chlorophényle | 2-fluorophényle | 1H-imidazol-2-yle | H |
| 1.113 | 4-chlorophényle | 2-fluorophényle | 1,2,4-oxadiazol-3-yle | H |
| 1.114 | 4-chlorophényle | 2-fluorophényle | N-cyclopropyl-carboxamide | H |
| 1.115 | 4-chlorophényle | 2-fluorophényle | thiazol-2-yle | H |
| 1.116 | 4-chlorophényle | 2-fluorophényle | pyrimidin-4-yle | H |
| 1.117 | 4-chlorophényle | 2-fluorophényle | 1-méthyleimidazol-2-yle | H |
| 1.118 | 4-chlorophényle | 2-fluorophényle | 2-méthyl-1,3,4-oxadiazol-5-yle | H |
| 1.119 | 4-chlorophényle | 2-fluorophényle | thiazol-4-yle | H |
| 1.120 | 4-chlorophényle | 2-fluorophényle | isoxazol-3-yle | H |
| 1.121 | 4-chlorophényle | 2-fluorophényle | 1,2,4-oxadiazol-5-yle | H |
| 1.122 | 4-chlorophényle | 2-fluorophényle | 5-fluoro-pyridin-2-yle | H |
| 1.123 | 4-chlorophényle | 2-fluorophényle | (4-pyridyl)-1,3,4-oxadiazol-2-yle | H |
| 1.124 | 4-chlorophényle | 2-fluorophényle | isoxazol-2-yle | H |
| 1.125 | 4-chlorophényle | 2-fluorophényle | 4-méthylpyrimidin-2-yle | H |
| 1.126 | 4-chlorophényle | 2-fluorophényle | 3-chloro-pyridin-2-yle | H |
| 1.127 | 4-chloro-3-fluorophényle | 2-fluorophényle | pyrimidin-2-yle | H |
ou sel ou N-oxyde acceptable sur le plan agrochimique correspondant.

2. Composition pesticide, qui comprend au moins un composé selon la revendication 1, ou un sel ou un N-oxyde acceptable sur le plan agrochimique correspondant, en tant qu'ingrédient actif et au moins un auxiliaire.

3. Composition selon la revendication 2, qui comprend en outre un ou plusieurs autres agents actifs insecticides, acaricides, nématicides et/ou fongicides.

4. Procédé pour la lutte contre des nuisibles, qui comprend l'application d'une composition selon la revendication 2 ou 3 aux nuisibles ou à leur environnement, à l'exception d'un procédé pour le traitement du corps humain ou animal par chirurgie ou thérapie et de procédés de diagnostic pratiqués sur le corps humain ou animal.

5. Procédé pour la protection de matériel de propagation végétale contre l'attaque par des nuisibles, qui comprend le traitement du matériel de propagation ou du site, où le matériel de propagation est planté, avec une composition selon la revendication 2 ou 3.

6. Matériau de propagation végétale revêtu, le revêtement du matériel de propagation végétale comprenant un composé tel que défini dans la revendication 1.
